# EUROPEAN PATENT APPLICATION

(11) **EP 4 722 195 A1**
(43) Date of publication of application: **08.04.2026**
(21) Application number: 24815626.7
(22) Date of filing: 31.05.2024
(51) Int. Cl.: C07D 211/62, A61K 9/127, A61K 47/22, A61K 47/28, A61K 47/34, C07D 295/15

(54) **PH-SENSITIVE CATIONIC LIPID AND LIPID NANOPARTICLES**

(30) Priority: 31.05.2023 JP 2023090454
(71) Applicant: National University Corporation Hokkaido University, Hokkaido 060-0808 (JP)
(72) Inventor: SATO, Yusuke, Sapporo-shi, Hokkaido 060-0808 (JP); HARASHIMA, Hideyoshi, Sapporo-shi, Hokkaido 060-0808 (JP); ITO, Rina, Sapporo-shi, Hokkaido 060-0808 (JP); SUZUKI, Yuichi, Sapporo-shi, Hokkaido 060-0808 (JP)
(74) Representative: Müller-Boré & Partner Patentanwälte PartG mbB
(86) International application number: PCT/JP2024/020084
(87) International publication number: WO 2024/248146

(57) **Abstract**

Provided are: a pH-sensitive cationic lipid which can be more easily synthesized through a small number of steps without requiring any organometal reaction or use of any relatively expensive starting materials; and lipid nanoparticles including the lipid. Disclosed is a pH-sensitive cationic lipid comprising a compound represented by general formula (I) or (I'), wherein R¹, Z², s, Z¹, and X are as defined in the description.

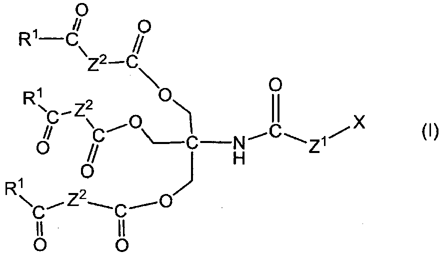

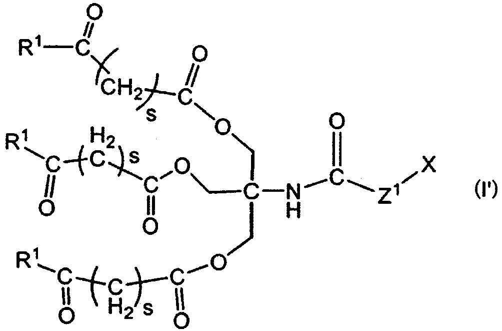

## Description

### Technical Field

The present invention relates to a pH-sensitive cationic lipid having three hydrophobic chains and a lipid nanoparticle containing the pH-sensitive cationic lipid.

### Background Art

Lipid nanoparticles (LNPs) are used as carriers to encapsulate fat-soluble drugs and nucleic acids such as siRNA (short interfering RNA) and mRNA and deliver them to target cells. For example, as lipid nanoparticles that serve as carriers for efficiently delivering nucleic acids such as siRNA into target cells, lipid nanoparticles have been reported that contain pH-sensitive cationic lipids, which are electrically neutral at physiological pH and change to cationic in a weakly acidic pH environment such as an endosome, as constituent lipids (Patent Literature 1). Lipid nanoparticles carrying a positive charge in the blood interact non-specifically with plasma proteins and are rapidly cleared from the blood by the reticuloendothelial system. In contrast, lipid nanoparticles containing pH-sensitive cationic lipids as constituent lipids are not charged under neutral conditions in the blood, but are positively charged under weakly acidic conditions in endosomes and can efficiently escape from endosomes.

As a pH-sensitive cationic lipid, for example, Jayaraman et al. developed DLin-MC3-DMA and achieved an ED₅₀ of 0.005 mg siRNA/kg in factor VII (F7) knockdown in mouse liver (Non-patent Literature 1). The present inventors have also previously developed their own pH-sensitive cationic lipids YSK05 and YSK13-C3, achieving ED₅₀s of 0.06 and 0.015 mg siRNA/kg, respectively, in F7 knockdown (Non-patent Literatures 2-4). The present inventors have further developed compounds having two hydrocarbon chains and one hydrophilic group, such as 7-(4-(diisopropylamino)butyl)-7-hydroxytridecane-1,13-diyl dioleate (CL4H6), as pH-sensitive cationic lipids (Patent Literature 1).

On the other hand, as a method for producing lipid nanoparticles encapsulating nucleic acids and the like, there is a method based on the principle of alcohol dilution using a flow path. For example, it has been reported that lipid nanoparticles having a diameter of about 30 nm can be reproducibly produced by using a microchannel with a built-in three-dimensional micromixer capable of achieving instantaneous mixing of two liquids (Non-patent Literature 5). It has also been reported that by using a simple two-dimensional flow path structure in which baffles of a certain width are arranged alternately from both sides in a micro-sized flow path through which a raw material solution flows, it is possible to form a nano-sized lipid particle formation system with higher particle size controllability than the conventional flow path structure using a three-dimensional mixer (Patent Literature 2). Recently, these methods for producing nanoparticle formulations have been mainly employed for the production of lipid nanoparticles (LNPs) loaded with fat-soluble drugs and nucleic acids such as siRNA (short interfering RNA) or mRNA.

### Citation List

### Patent Literatures

Patent Literature 1: International Publication No. WO 2018/230710
Patent Literature 2: International Publication No. WO 2018/190423

### Non Patent Literatures

Non Patent Literature 1: Jayaraman et al., Angewandte Chemie International Edition, 2012, vol. 51, pp. 8529-8533.
Non Patent Literature 2: Watanabe et al., Scientific Reports, 2014, 4:4750, DOI: 10.1038/srep04750.
Non Patent Literature 3: Yamamoto et al., Journal of Hepatology, 2016, vol. 64, pp. 547-555.
Non Patent Literature 4: Sato et al., Molecular Therapy, 2016, vol. 24, pp. 788-795.
Non Patent Literature 5: Leung et al., Journal of Physical Chemistry C Nanomater Interfaces, 2012, vol. 116(34), pp. 18440-18450.

### Summary of Invention

When a compound is mass-produced in a factory, it is preferable that it can be synthesized using inexpensive raw materials and in a small number of reaction steps from the viewpoints of production cost, quality stability, and the like. However, the synthesis reaction of pH-sensitive cationic lipids described in Patent Literature 1, such as CL4H6, is a multi-step reaction of as many as 6 to 7 steps and further requires a Grignard reaction using an organometallic compound. The following shows the outline of the synthesis reaction of CL4H6.

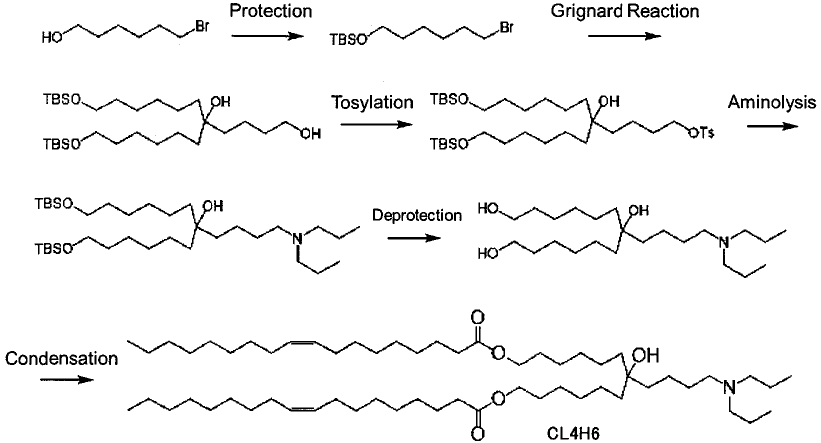

An object of the present invention is to provide a pH-sensitive cationic lipid that can be synthesized more easily in fewer steps without using an organometallic reaction or a relatively expensive raw material, and a lipid nanoparticle containing the pH-sensitive cationic lipid.

The present inventors have found that a pH-sensitive cationic lipid can be synthesized in a smaller number of steps without using a relatively expensive raw material by using tris(hydroxymethyl)aminomethane (CAS number: 77-86-1) (hereinafter abbreviated as "Tris") as a linker, bonding to each of the three hydroxyl groups of Tris a hydrocarbon chain via a linking group serving as a spacer, and bonding one hydrophilic group to the amino group of Tris, thereby completing the present invention.

That is, the present invention provides, for example, the following pH-sensitive cationic lipids and the like.
[1] A pH-sensitive cationic lipid comprising a compound represented by the following general formula (I) or (I'): 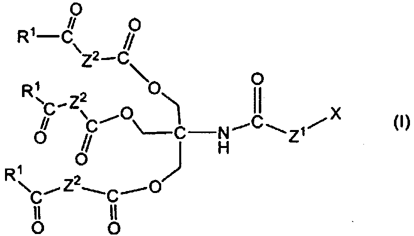 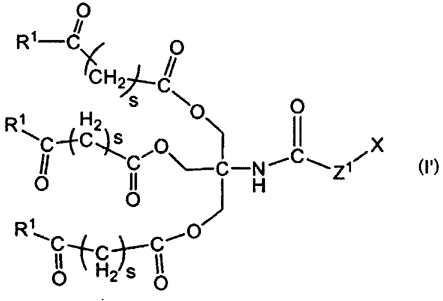
   wherein, in the general formulae (I) and (I'), R¹ is a group represented by any of the general formulae (T-1) to (T-3): 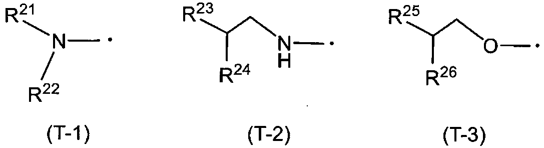
   wherein, in the general formulae, R²¹ is a hydrocarbon group having 1 to 22 carbon atoms; R²² is a hydrocarbon group having 1 to 22 carbon atoms; R²³ is a hydrocarbon group having 1 to 22 carbon atoms; R²⁴ is a hydrogen atom or a hydrocarbon group having 1 to 22 carbon atoms; R²⁵ is a hydrocarbon group having 1 to 22 carbon atoms; and R²⁶ is a hydrogen atom or a hydrocarbon group having 1 to 22 carbon atoms, provided that the total number of carbon atoms in R²¹ and R²² is equal to or greater than 8, the total number of carbon atoms in R²³ and R²⁴ is equal to or greater than 6, and the total number of carbon atoms in R²⁵ and R²⁶ is equal to or greater than 6; and a black circle represents a bonding arm;
      three R¹ groups in one molecule may be the same as or different from each other; Z¹ is an alkylene group having 1 to 6 carbon atoms which may be substituted with one hydroxyalkyl group having 1 to 3 carbon atoms, or a single bond; X is -N(R²)(R³) or a 5-to 7-membered nonaromatic heterocyclic group, wherein the group is bonded to Z¹ via a carbon atom, and the ring may be substituted to have one or two hydrocarbon groups having 1 to 4 carbon atoms or amide groups thereon; and R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group;
      in the formulae (I), Z² is a linear alkylene group having 4 to 6 carbon atoms or a cycloalkylene group; three Z² in one molecule may be the same as or different from each other, and
      in formula (I'), "s" is 2, 3, or 4; three "s" in one molecule may be the same as or different from each other.
[2] The pH-sensitive cationic lipid described in [1] above, wherein -Z¹-X in the following general formula (I) or (I') is selected from the following formulae (H-1) to (H-8) and (H-16) to (H-26): 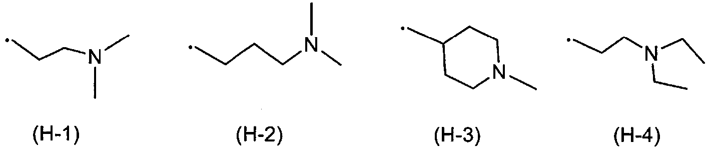 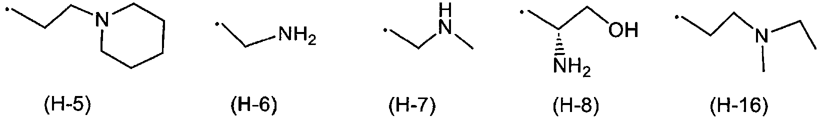 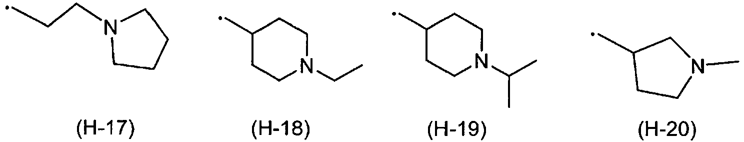 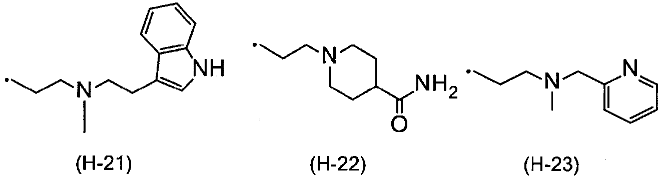 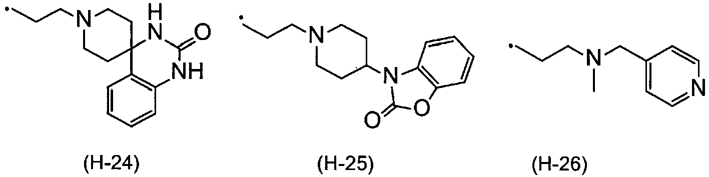 wherein a black circle represents a bonding arm.
[3] The pH-sensitive cationic lipid described in [1] or [2] above, wherein the three R¹ groups in one molecule are all the same.
[4] The pH-sensitive cationic lipid described in any of [1] to [3] above, which is represented by the general formula (I') wherein "s" is 2 or 3.
[5] The pH-sensitive cationic lipid described in any of [1] to [4] above, wherein R¹ is any group of the formula (A8), (A8β), (A10), (A12), (A12β), (A14), (A16), (A16β), (A16b), (A20), (A24), (E12), (E14), (A14β), (A24β), (A10L), (A12L), or (A14L): 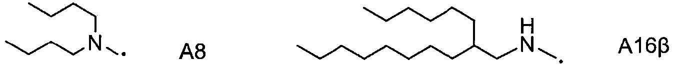 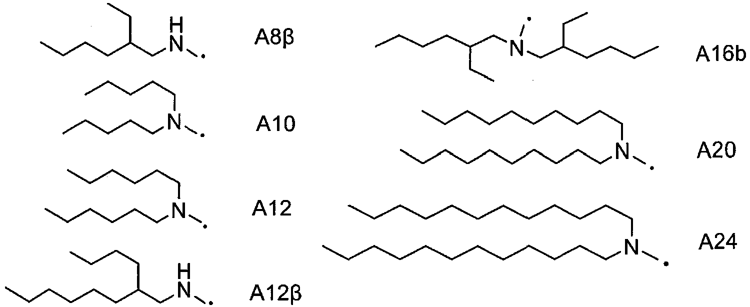 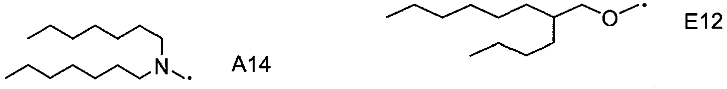 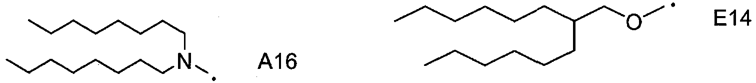 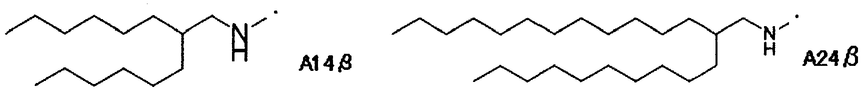 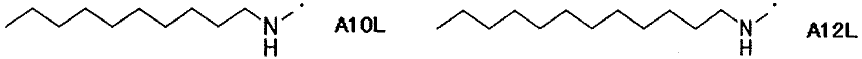 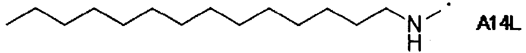 wherein a black circle represents a bonding arm.
[6] The pH-sensitive cationic lipid described in any of [1] to [5] above, wherein pKa is between 4.0 and 9.0.
[7] The pH-sensitive cationic lipid described in any of [1] to [6] above which is selected from the following compounds: 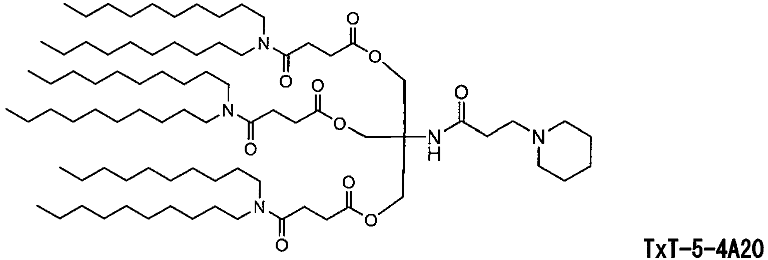 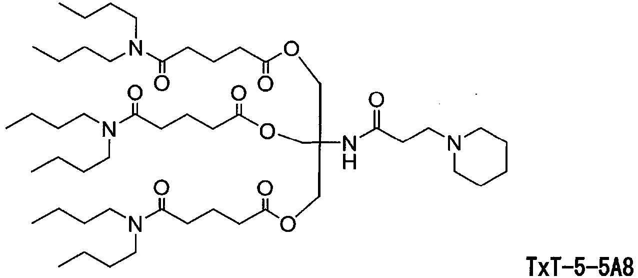 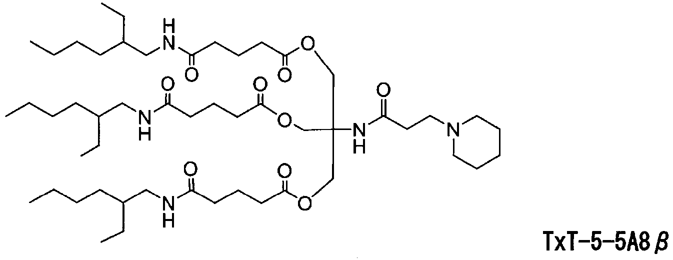 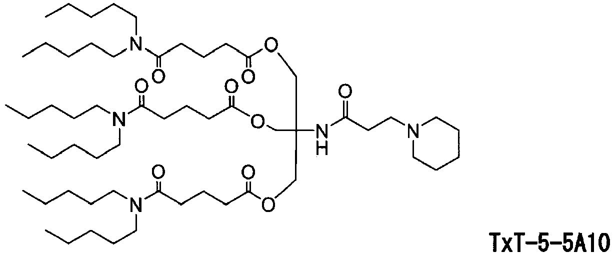 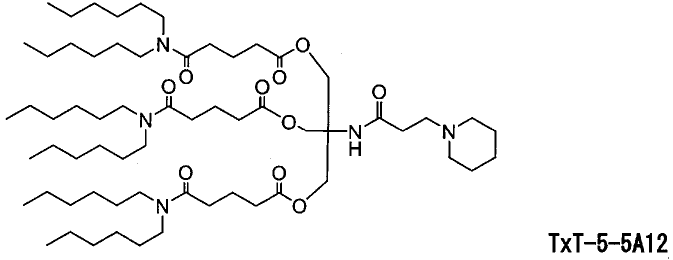 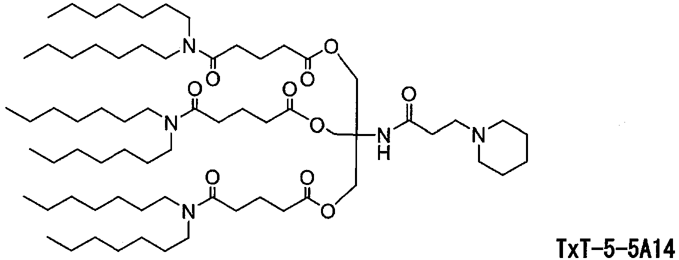 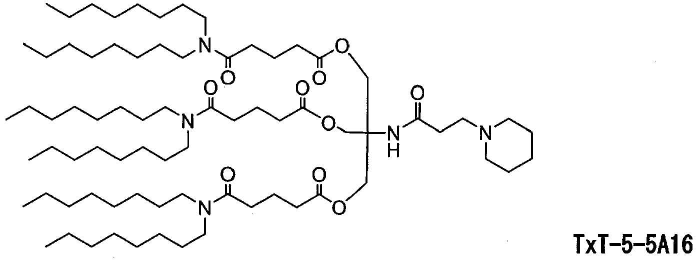 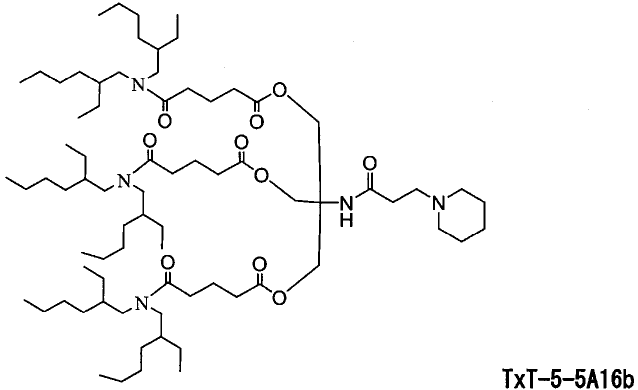 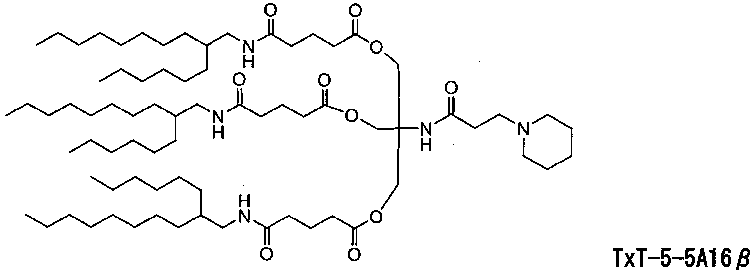 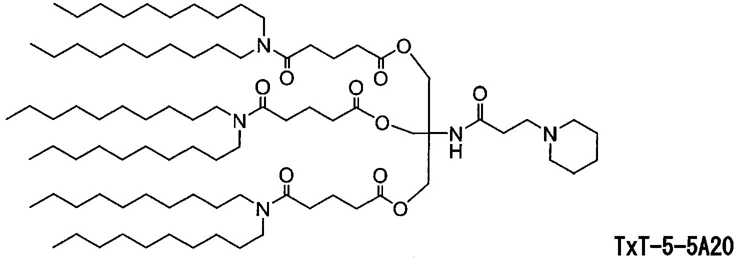 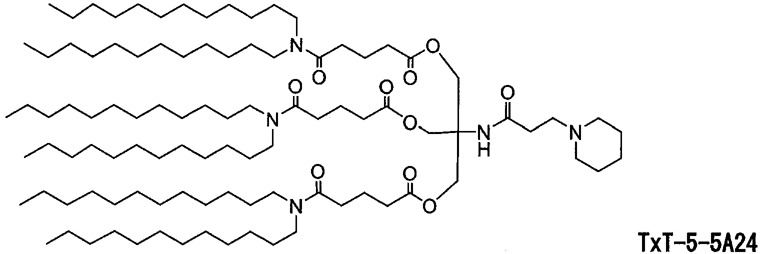 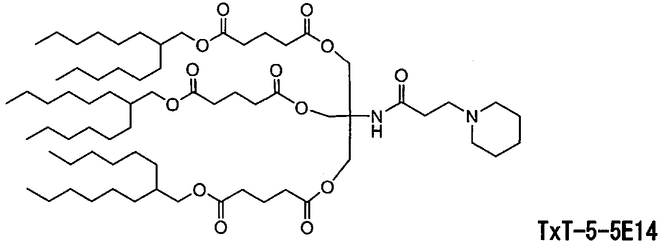 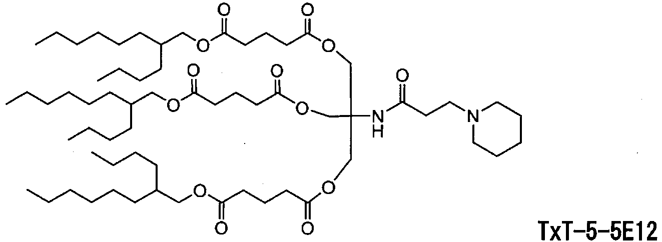 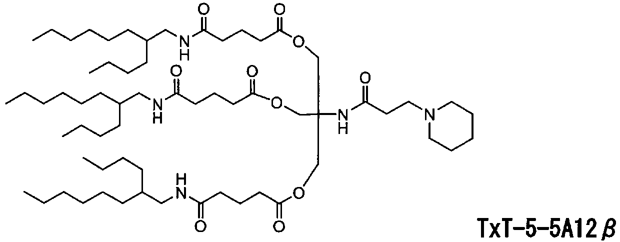 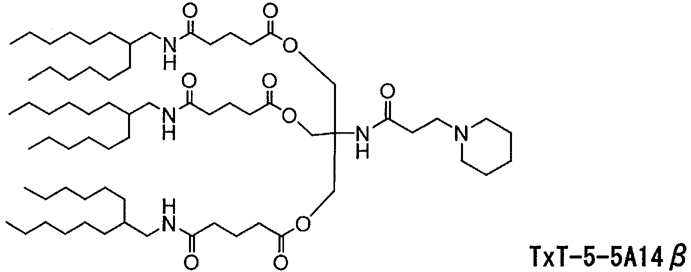 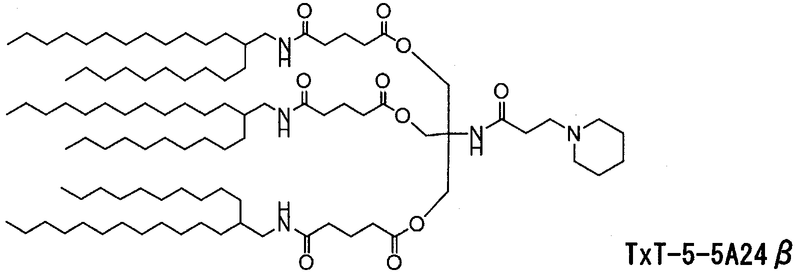 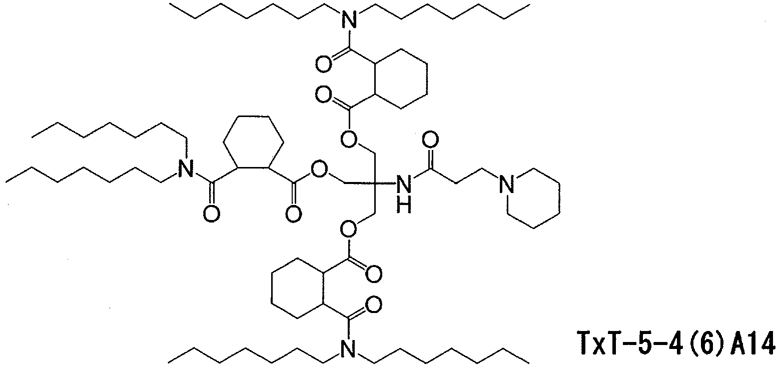 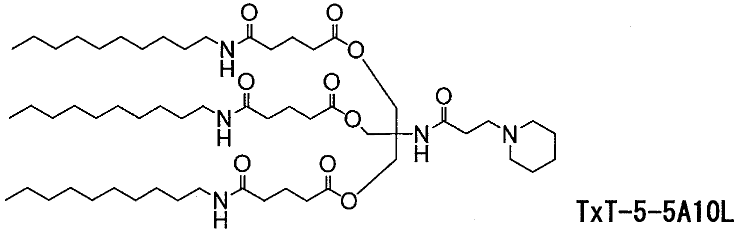 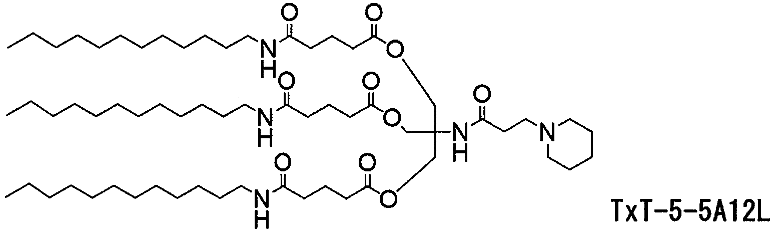 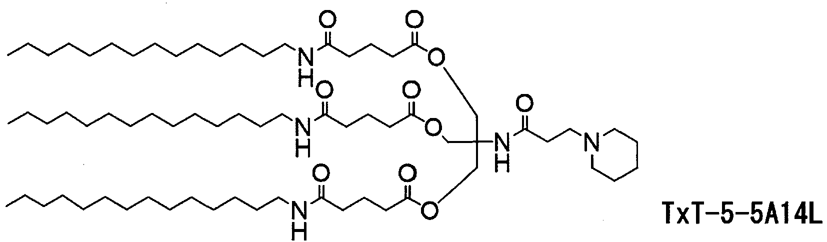 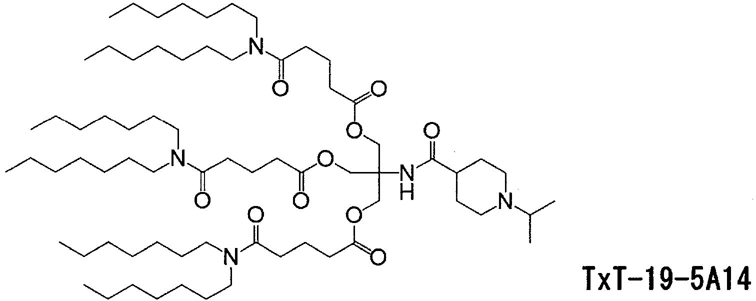
[8] A method for producing a pH-sensitive cationic lipid, the method comprising condensing a compound represented by the following general formula (A), a compound represented by the following general formula (B), and tris(hydroxymethyl)aminomethane to produce a pH-sensitive cationic lipid comprising a compound represented by the following general formula (I): 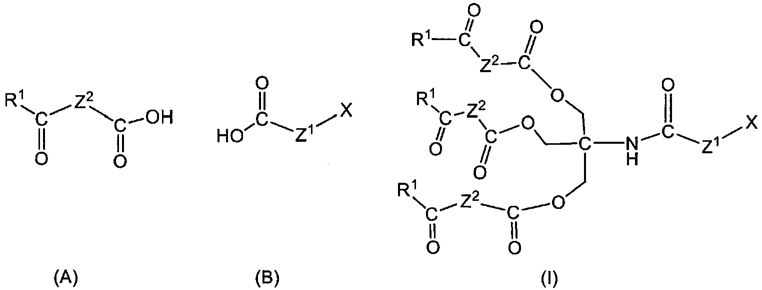
   wherein R¹ is a group represented by any of the following general formulae (T-1) to (T-3): 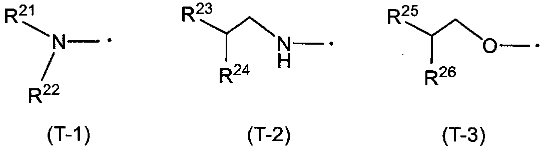
   wherein R²¹ is a hydrocarbon group having 1 to 22 carbon atoms; R²² is a hydrocarbon group having 1 to 22 carbon atoms; R²³ is a hydrocarbon group having 1 to 22 carbon atoms; R²⁴ is a hydrogen atom or a hydrocarbon group having 1 to 22 carbon atoms; R²⁵ is a hydrocarbon group having 1 to 22 carbon atoms; and R²⁶ is a hydrogen atom or a hydrocarbon group having 1 to 22 carbon atoms, provided that the total number of carbon atoms in R²¹ and R²² is equal to or greater than 8, the total number of carbon atoms in R²³ and R²⁴ is equal to or greater than 6, and the total number of carbon atoms in R²⁵ and R²⁶ is equal to or greater than 6; and a black circle represents a bonding arm; and
      Z² is a linear alkylene group having 4 to 6 carbon atoms or a cycloalkylene group; Z¹ is an alkylene group having 1 to 6 carbon atoms which may be substituted with one hydroxyalkyl group having 1 to 3 carbon atoms, or a single bond; X is -N(R²)(R³) or a 5-to 7-membered nonaromatic heterocyclic group, wherein the group is bonded to Z¹ via a carbon atom, and the ring may be substituted to have one or two hydrocarbon groups having 1 to 4 carbon atoms or amide groups thereon; and R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group; in the formula (I), three R¹ groups in one molecule may be the same as or different from each other; and in the formula (I), three Z² in one molecule may be the same as or different from each other
[9] A method for producing a pH-sensitive cationic lipid, the method comprising:
   condensing a compound represented by the following general formula (A-0) and a compound represented by the following general formula (D) to produce a compound represented by the following general formula (A'); and
   condensing the compound represented by the general formula (A'), a compound represented by the following general formula (B), and tris(hydroxymethyl)aminomethane to produce a pH-sensitive cationic lipid comprising a compound represented by the following general formula (I'). 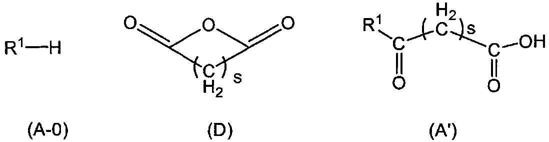 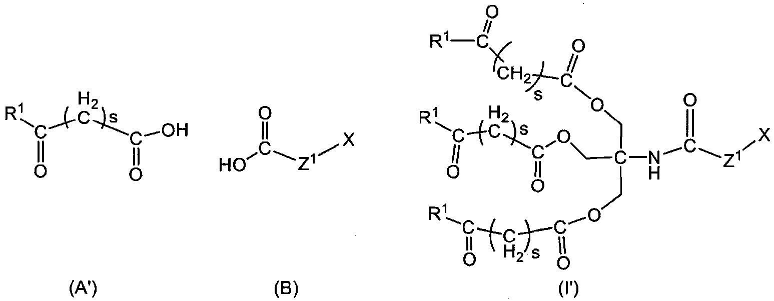
   wherein R¹ is a group represented by any of the general formulae (T-1) to (T-3): 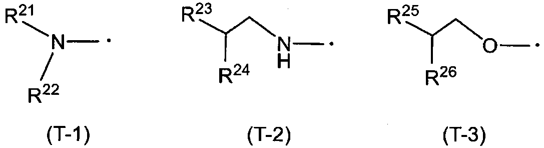
   wherein R²¹ is a hydrocarbon group having 1 to 22 carbon atoms; R²² is a hydrocarbon group having 1 to 22 carbon atoms; R²³ is a hydrocarbon group having 1 to 22 carbon atoms; R²⁴ is a hydrogen atom or a hydrocarbon group having 1 to 22 carbon atoms; R²⁵ is a hydrocarbon group having 1 to 22 carbon atoms; and R²⁶ is a hydrogen atom or a hydrocarbon group having 1 to 22 carbon atoms, provided that the total number of carbon atoms in R²¹ and R²² is equal to or greater than 8, the total number of carbon atoms in R²³ and R²⁴ is equal to or greater than 6, and the total number of carbon atoms in R²⁵ and R²⁶ is equal to or greater than 6; and a black circle represents a bonding arm; and
      "s" is 2, 3, or 4; Z¹ is an alkylene group having 1 to 6 carbon atoms which may be substituted with one hydroxyalkyl group having 1 to 3 carbon atoms, or a single bond; X is -N(R²)(R³) or a 5- to 7-membered nonaromatic heterocyclic group, wherein the group is bonded to Z¹ via a carbon atom, and the ring may be substituted to have one or two hydrocarbon groups having 1 to 4 carbon atoms or amide groups thereon; and R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group; in the general formula (I'), three R¹ groups in one molecule may be the same as or different from each other; and in the general formula (I'), three "s" in one molecule may be the same as or different from each other.
[10] A method for producing a pH-sensitive cationic lipid, the method comprising:
   adding N-[tris(hydroxymethyl)methyl]acrylamide to a compound represented by the following general formula (C): 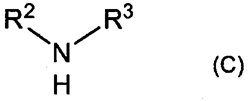
   wherein R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group; and
   then condensing the resulting compound with a compound represented by the following general formula (A) or (A'): 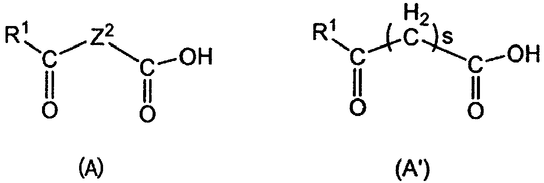
   wherein R¹ is a group represented by any of the general formulae (T-1) to (T-3): 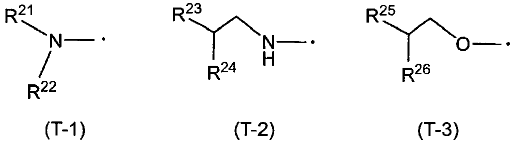
   wherein R²¹ is a hydrocarbon group having 1 to 22 carbon atoms; R²² is a hydrocarbon group having 1 to 22 carbon atoms; R²³ is a hydrocarbon group having 1 to 22 carbon atoms; R²⁴ is a hydrogen atom or a hydrocarbon group having 1 to 22 carbon atoms; R²⁵ is a hydrocarbon group having 1 to 22 carbon atoms; and R²⁶ is a hydrogen atom or a hydrocarbon group having 1 to 22 carbon atoms, provided that the total number of carbon atoms in R²¹ and R²² is equal to or greater than 8, the total number of carbon atoms in R²³ and R²⁴ is equal to or greater than 6, and the total number of carbon atoms in R²⁵ and R²⁶ is equal to or greater than 6; and a black circle represents a bonding arm; in formula (A), Z² is a linear alkylene group having 4 to 6 carbon atoms or a cycloalkylene group; and
      in formula (A'), "s" is 2, 3, or 4,
      thereby producing a pH-sensitive cationic lipid comprising a compound represented by the following general formula (I-a) or (I'-a): 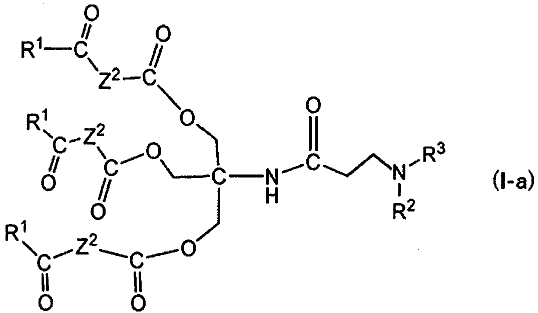 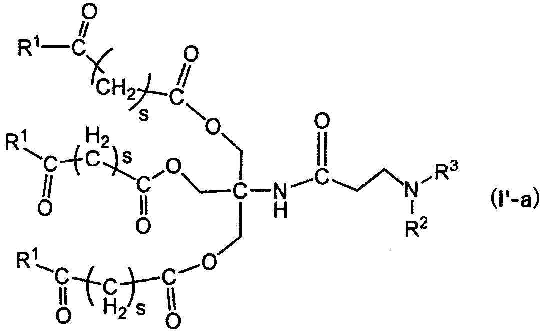
   wherein R¹, R², and R³ are defined in the same manner as described above; three R¹ groups in one molecule may be the same as or different from each other;
      in formula (I-a), Z² is defined in the same manner as described above; and
      in formula (I'-a), "s" is defined in the same manner as described above.
[11] A lipid nanoparticle comprising the pH-sensitive cationic lipid described in any of [1] to [7] above.
[12] The lipid nanoparticle described in [11] above, further comprising a sterol and a polyalkylene glycol-modified lipid.
[13] The lipid nanoparticle described in [11] or [12] above, further comprising phosphatidylcholine.
[14] The lipid nanoparticle described in any of [11] to [13] above, containing a nucleic acid.
[15] The lipid nanoparticle described in [14] above, wherein the nucleic acid is siRNA, mRNA, gRNA, or plasmid DNA.
[16] A pharmaceutical composition comprising the lipid nanoparticle containing the pH-sensitive cationic lipid described in any of [1] to [7] above as an active ingredient.
[17] A pharmaceutical composition comprising, as an active ingredient, the lipid nanoparticle containing the pH-sensitive cationic lipid described in any of [1] to [7] above and a nucleic acid.
[18] The pharmaceutical composition described in [16] or [17] above, which is used for gene therapy.
[19] A method for expressing a foreign gene, comprising administering to a subject animal the lipid nanoparticle described in [14] above, which encapsulates a foreign gene of interest to be expressed in a target cell, and expressing the foreign gene in the target cell of the subject animal.
[20] A method for expressing a foreign gene, comprising administering to a subject animal except for a human the lipid nanoparticle described in [14] above, which encapsulates a foreign gene of interest to be expressed in a target cell, and expressing the foreign gene in the target cell of the subject animal.

The pH-sensitive cationic lipid according to the present invention is relatively easy to synthesize and is suitable for large-scale synthesis. Therefore, the pH-sensitive cationic lipid is useful as a constituent lipid of lipid nanoparticles used as an active ingredient of a pharmaceutical.

### Brief Description of Drawings

Fig. 1 illustrates a diagram showing the results of measurement of Fluc activity (RLU/mg protein) in the liver, spleen, kidney, lung, heart, brain, and muscle of mice administered with Fluc mRNA-loaded lipid nanoparticles in Example 22.
Fig. 2A illustrates a diagram showing the results of measurement of Fluc activity (RLU/mg protein) in the liver of mice administered with Fluc mRNA-loaded lipid nanoparticles in Example 23.
Fig. 2B illustrates a diagram showing the results of measurement of Fluc activity (RLU/mg protein) in the spleen of mice administered with Fluc mRNA-loaded lipid nanoparticles in Example 23.
Fig. 2C illustrates a diagram showing the results of measurement of Fluc activity (RLU/mg protein) in the kidney of mice administered with Fluc mRNA-loaded lipid nanoparticles in Example 23.
Fig. 2D illustrates a diagram showing the results of measurement of Fluc activity (RLU/mg protein) in the lung of mice administered with Fluc mRNA-loaded lipid nanoparticles in Example 23.
Fig. 2E illustrates a diagram showing the results of measurement of Fluc activity (RLU/mg protein) in the heart of mice administered with Fluc mRNA-loaded lipid nanoparticles in Example 23.
Fig. 3A illustrates a diagram showing the results of measurement of Fluc activity (RLU/mg protein) in the liver of mice administered with each Fluc mRNA-loaded lipid nanoparticle in Example 24.
Fig. 3B illustrates a diagram showing the results of measurement of Fluc activity (RLU/mg protein) in the spleen of mice administered with each Fluc mRNA-loaded lipid nanoparticle in Example 24.
Fig. 3C illustrates a diagram showing the results of measurement of Fluc activity (RLU/mg protein) in the kidney of mice administered with each Fluc mRNA-loaded lipid nanoparticle in Example 24.
Fig. 3D illustrates a diagram showing the results of measurement of Fluc activity (RLU/mg protein) in the lung of mice administered with each Fluc mRNA-loaded lipid nanoparticle in Example 24.
Fig. 3E illustrates a diagram showing the results of measurement of Fluc activity (RLU/mg protein) in the heart of mice administered with each Fluc mRNA-loaded lipid nanoparticle in Example 24.
Fig. 4 illustrates a diagram showing the results of measurement of Fluc activity (RLU/mg protein) in the liver of mice administered with each Fluc mRNA-loaded lipid nanoparticle in Example 25.
Fig. 5 shows microscopic images of liver sections from mice administered with each EGFP mRNA-loaded lipid nanoparticle in Example 26 (upper row: EGFP, lower row: vascular endothelial cells).
Fig. 6 shows microscopic images of liver sections from mice administered with each EGFP mRNA-loaded lipid nanoparticle in Example 27 (upper row: EGFP, lower row: vascular endothelial cells).
Fig. 7 illustrates a diagram showing the results of measurement of Fluc activity (RLU/mg protein) in the liver of mice administered with Fluc mRNA-loaded lipid nanoparticles in Example 28.
Fig. 8 illustrates a diagram showing the results of measuring the expression level of human erythropoietin in the serum (hEPO activity) of mice administered with hEPO mRNA-loaded lipid nanoparticles in Example 29.
Fig. 9 illustrates a diagram showing the results of measurement of Fluc activity (RLU/mg protein) in the liver of mice administered with Fluc mRNA-loaded lipid nanoparticles in Example 30.
Fig. 10 illustrates a diagram showing the results of measurement of Fluc activity (RLU/mg protein) in the liver, spleen, and lung of mice administered with Fluc mRNA-loaded lipid nanoparticles in Example 31.
Fig. 11 illustrates a diagram showing the results of measurement of the TTR amount in the serum (µg/mL) and TTR knockdown efficiency (%) of mice administered with TTR-targeted genome editing lipid nanoparticles loaded with Cas9-mRNA and TTR-gRNA in Example 32.
Fig. 12 illustrates a diagram showing the results of measurement of the TTR amount in the serum (µg/mL) of mice administered with TTR-targeted genome editing lipid nanoparticles loaded with Cas9-mRNA and TTR-gRNA in Example 33.
Fig. 13 illustrates a diagram showing the results of measurement of the TTR amount in the serum (ng/mL) of mice administered with PCSK9-targeted genome editing lipid nanoparticles loaded with Cas9-mRNA and PCSK9-gRNA in Example 34.

### Description of Embodiments

Hereinafter, embodiments of the present invention will be specifically described. In this specification, "X1 to X2 (X1 and X2 are real numbers satisfying X1 < X2)" means "X1 or more and X2 or less." Also, "C_{X3-X4} (X3 and X4 are integers satisfying X3 < X4)" means "having X3 or more and X4 or less carbon atoms."

### [pH-Sensitive Cationic Lipid]

The pH-sensitive cationic lipid according to the present invention is a compound in which three hydrocarbon chains are each bonded to one of the three oxygen atoms of a tris skeleton via a spacer linkage group, and a hydrophilic group is bonded to the one nitrogen atom of the tris skeleton. The term "tris skeleton" as described herein refers to a structure derived from tris(hydroxymethyl)aminomethane, in which one hydrogen atom is removed from each of the three hydroxyl groups and the amino group. That is, the pH-sensitive cationic lipid according to the present invention is a compound having three hydrocarbon chains and a hydrophilic moiety, and due to its structural similarity, it exhibits high affinity with lipids such as phospholipids, making it suitable as a constituent lipid of lipid nanoparticles.

Specifically, the pH-sensitive cationic lipid according to the present invention comprises a compound represented by the following general formula (I) or general formula (I').

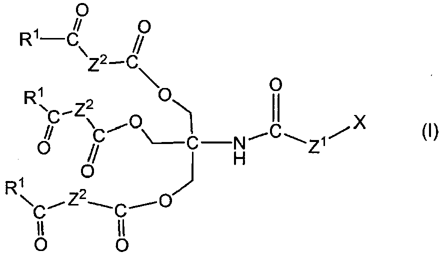

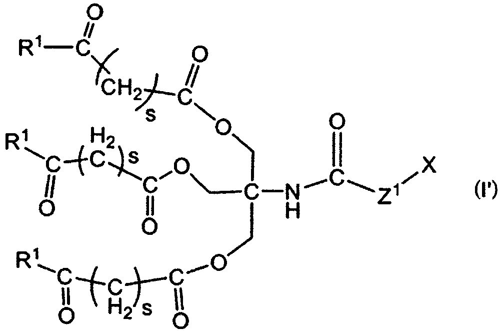

In the general formulae (I) and (I'), R¹ is a group represented by any of the general formulae (T-1) to (T-3). In the formulae, a black circle represents a bonding arm.

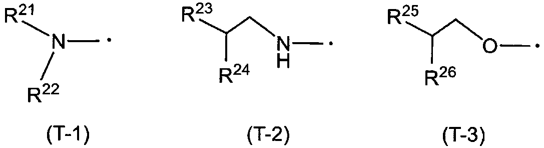

In the general formulae (T-1) to (T-3), R²¹ each independently is a hydrocarbon group having 1 to 22 carbon atoms (C₁₋₂₂ hydrocarbon group), R²² is a hydrocarbon group having 1 to 22 carbon atoms, R²³ is a hydrocarbon group having 1 to 22 carbon atoms, R²⁴ is a hydrogen atom or a hydrocarbon group having 1 to 22 carbon atoms, R²⁵ is a hydrocarbon group having 1 to 22 carbon atoms, and R²⁶ is a hydrogen atom or a hydrocarbon group having 1 to 22 carbon atoms. The hydrocarbon groups represented by R²¹ to R²⁶ may be alkyl groups or alkenyl groups. The alkenyl groups may contain one, two, or three unsaturated bonds. The C₁₋₂₂ hydrocarbon group may be linear or branched.

When R²¹ to R²⁶ are C₁₋₂₂ alkyl groups, examples of the alkyl groups include:
methyl group, ethyl group, n-propyl group, isopropyl group, n-butyl group, isobutyl group, sec-butyl group, tert-butyl group;
n-pentyl group, 1-methylbutyl group, 2-methylbutyl group, 3-methylbutyl group, 1-ethylpropyl group, 1,1-dimethylpropyl group, 2,2-dimethylpropyl group;
n-hexyl group, 1-methylpentyl group, 2-methylpentyl group, 3-methylpentyl group, 4-methylpentyl group, 1-ethylbutyl group, 1,1-dimethylbutyl group, 2,2-dimethylbutyl group, 3,3-dimethylbutyl group, 1,2-dimethylbutyl group, 1-methyl-2,2-dimethylbutyl group;
n-heptyl group, 1-methylhexyl group, 2-methylhexyl group, 3-methylhexyl group, 4-methylhexyl group, 5-methylhexyl group, 1-ethylpentyl group, 1,1-dimethylpentyl group, 2,2-dimethylpentyl group, 3,3-dimethylpentyl group, 4,4-dimethylpentyl group, 1-methyl-3,3-dimethylbutyl group, 2-methyl-3,3-dimethylbutyl group;
n-octyl group, 1-methylheptyl group, 2-methylheptyl group, 3-methylheptyl group, 4-methylheptyl group, 5-methylheptyl group, 6-methylheptyl group, 1-ethylhexyl group, 1,1-dimethylhexyl group, 2,2-dimethylhexyl group, 3,3-dimethylhexyl group, 4,4-dimethylhexyl group, 5,5-dimethylhexyl group, 1-methyl-4,4-dimethylpentyl group, 2-methyl-4,4-dimethylpentyl group, 3-methyl-4,4-dimethylpentyl group;
n-nonyl group, 1-methyloctyl group, 2-methyloctyl group, 3-methyloctyl group, 4-methyloctyl group, 5-methyloctyl group, 6-methyloctyl group, 7-methyloctyl group, 1-ethylheptyl group, 1,1-dimethylheptyl group, 2,2-dimethylheptyl group, 3,3-dimethylheptyl group, 4,4-dimethylheptyl group, 5,5-dimethylheptyl group, 6,6-dimethylheptyl group, 1-methyl-5,5-dimethylhexyl group, 2-methyl-5,5-dimethylhexyl group, 3-methyl-5,5-dimethylhexyl group, 4-methyl-5,5-dimethylhexyl group;
n-decyl group, 1-methylnonyl group, 2-methylnonyl group, 3-methylnonyl group, 4-methylnonyl group, 5-methylnonyl group, 6-methylnonyl group, 7-methylnonyl group, 8-methylnonyl group, 1-ethyloctyl group, 1,1-dimethyloctyl group, 2,2-dimethyloctyl group, 3,3-dimethyloctyl group, 4,4-dimethyloctyl group, 5,5-dimethyloctyl group, 6,6-dimethyloctyl group, 7,7-dimethyloctyl group, 1-methyl-6,6-dimethylheptyl group, 2-methyl-6,6-dimethylheptyl group, 3-methyl-6,6-dimethylheptyl group, 4-methyl-6,6-dimethylheptyl group, 5-methyl-6,6-dimethylheptyl group;
n-undecyl group, 1-methyldecyl group, 2-methyldecyl group, 3-methyldecyl group, 4-methyldecyl group, 5-methyldecyl group, 6-methyldecyl group, 7-methyldecyl group, 8-methyldecyl group, 9-methyldecyl group, 1-ethylnonyl group, 1,1-dimethylnonyl group, 2,2-dimethylnonyl group, 3,3-dimethylnonyl group, 4,4-dimethylnonyl group, 5,5-dimethylnonyl group, 6,6-dimethylnonyl group, 7,7-dimethylnonyl group, 8,8-dimethylnonyl group, 1-methyl-7,7-dimethyloctyl group, 2-methyl-7,7-dimethyloctyl group, 3-methyl-7,7-dimethyloctyl group, 4-methyl-7,7-dimethyloctyl group, 5-methyl-7,7-dimethyloctyl group, 6-methyl-7,7-dimethyloctyl group;
n-dodecyl group, 1-methylundecyl group, 2-methylundecyl group, 3-methylundecyl group, 4-methylundecyl group, 5-methylundecyl group, 6-methylundecyl group, 7-methylundecyl group, 8-methylundecyl group, 9-methylundecyl group, 10-methylundecyl group, 1-ethyldecyl group, 1,1-dimethyldecyl group, 2,2-dimethyldecyl group, 3,3-dimethyldecyl group, 4,4-dimethyldecyl group, 5,5-dimethyldecyl group, 6,6-dimethyldecyl group, 7,7-dimethyldecyl group, 8,8-dimethyldecyl group, 9,9-dimethyldecyl group, 1-methyl-8,8-dimethylnonyl group, 2-methyl-8,8-dimethylnonyl group, 3-methyl-8,8-dimethylnonyl group, 4-methyl-8,8-dimethylnonyl group, 5-methyl-8,8-dimethylnonyl group, 6-methyl-8,8-dimethylnonyl group, 7-methyl-8,8-dimethylnonyl group;
n-tridecyl group, 1-methyldodecyl group, 2-methyldodecyl group, 3-methyldodecyl group, 4-methyldodecyl group, 5-methyldodecyl group, 6-methyldodecyl group, 7-methyldodecyl group, 8-methyldodecyl group, 9-methyldodecyl group, 10-methyldodecyl group, 11-methyldodecyl group, 1-ethylundecyl group, 1,1-dimethylundecyl group, 2,2-dimethylundecyl group, 3,3-dimethylundecyl group, 4,4-dimethylundecyl group, 5,5-dimethylundecyl group, 6,6-dimethylundecyl group, 7,7-dimethylundecyl group, 8,8-dimethylundecyl group, 9,9-dimethylundecyl group, 10,10-dimethylundecyl group, 1-methyl-9,9-dimethyldecyl group, 2-methyl-9,9-dimethyldecyl group, 3-methyl-9,9-dimethyldecyl group, 4-methyl-9,9-dimethyldecyl group, 5-methyl-9,9-dimethyldecyl group, 6-methyl-9,9-dimethyldecyl group, 7-methyl-9,9-dimethyldecyl group, 8-methyl-9,9-dimethyldecyl group;
n-tetradecyl group, 1-methyltridecyl group, 2-methyltridecyl group, 3-methyltridecyl group, 4-methyltridecyl group, 5-methyltridecyl group, 6-methyltridecyl group, 7-methyltridecyl group, 8-methyltridecyl group, 9-methyltridecyl group, 10-methyltridecyl group, 11-methyltridecyl group, 12-methyltridecyl group, 1-ethyldodecyl group, 1,1-dimethyldodecyl group, 2,2-dimethyldodecyl group, 3,3-dimethyldodecyl group, 4,4-dimethyldodecyl group, 5,5-dimethyldodecyl group, 6,6-dimethyldodecyl group, 7,7-dimethyldodecyl group, 8,8-dimethyldodecyl group, 9,9-dimethyldodecyl group, 10,10-dimethyldodecyl group, 11,11-dimethyldodecyl group, 1-methyl-10,10-dimethylundecyl group, 2-methyl-10,10-dimethylundecyl group, 3-methyl-10,10-dimethylundecyl group, 4-methyl-10,10-dimethylundecyl group, 5-methyl-10,10-dimethylundecyl group, 6-methyl-10,10-dimethylundecyl group, 7-methyl-10,10-dimethylundecyl group, 8-methyl-10,10-dimethylundecyl group, 9-methyl-10,10-dimethylundecyl group;
n-pentadecyl group, 1-methyltetradecyl group, 2-methyltetradecyl group, 3-methyltetradecyl group, 4-methyltetradecyl group, 5-methyltetradecyl group, 6-methyltetradecyl group, 7-methyltetradecyl group, 8-methyltetradecyl group, 9-methyltetradecyl group, 10-methyltetradecyl group, 11-methyltetradecyl group, 12-methyltetradecyl group, 13-methyltetradecyl group, 1-ethyltridecyl group, 1,1-dimethyltridecyl group, 2,2-dimethyltridecyl group, 3,3-dimethyltridecyl group, 4,4-dimethyltridecyl group, 5,5-dimethyltridecyl group, 6,6-dimethyltridecyl group, 7,7-dimethyltridecyl group, 8,8-dimethyltridecyl group, 9,9-dimethyltridecyl group, 10,10-dimethyltridecyl group, 11,11-dimethyltridecyl group, 12,12-dimethyltridecyl group, 1-methyl-11,11-dimethyldodecyl group, 2-methyl-11,11-dimethyldodecyl group, 3-methyl-11,11-dimethyldodecyl group, 4-methyl-11,11-dimethyldodecyl group, 5-methyl-11,11-dimethyldodecyl group, 6-methyl-11,11-dimethyldodecyl group, 7-methyl-11,11-dimethyldodecyl group, 8-methyl-11,11-dimethyldodecyl group, 9-methyl-11,11-dimethyldodecyl group, 10-methyl-11,11-dimethyldodecyl group;
n-hexadecyl group, 1-methylpentadecyl group, 2-methylpentadecyl group, 3-methylpentadecyl group, 4-methylpentadecyl group, 5-methylpentadecyl group, 6-methylpentadecyl group, 7-methylpentadecyl group, 8-methylpentadecyl group, 9-methylpentadecyl group, 10-methylpentadecyl group, 11-methylpentadecyl group, 12-methylpentadecyl group, 13-methylpentadecyl group, 14-methylpentadecyl group; n-heptadecyl group, 1-methylhexadecyl group, 2-methylhexadecyl group, 3-methylhexadecyl group, 4-methylhexadecyl group, 5-methylhexadecyl group, 6-methylhexadecyl group, 7-methylhexadecyl group, 8-methylhexadecyl group, 9-methylhexadecyl group, 10-methylhexadecyl group, 11-methylhexadecyl group, 12-methylhexadecyl group, 13-methylhexadecyl group, 14-methylhexadecyl group, 15-methylhexadecyl group;
n-octadecyl group, 1-methylheptadecyl group, 2-methylheptadecyl group, 3-methylheptadecyl group, 4-methylheptadecyl group, 5-methylheptadecyl group, 6-methylheptadecyl group, 7-methylheptadecyl group, 8-methylheptadecyl group, 9-methylheptadecyl group, 10-methylheptadecyl group, 11-methylheptadecyl group, 12-methylheptadecyl group, 13-methylheptadecyl group, 14-methylheptadecyl group, 15-methylheptadecyl group, 16-methylheptadecyl group;
n-nonadecyl group, 1-methyloctadecyl group, 2-methyloctadecyl group, 3-methyloctadecyl group, 4-methyloctadecyl group, 5-methyloctadecyl group, 6-methyloctadecyl group, 7-methyloctadecyl group, 8-methyloctadecyl group, 9-methyloctadecyl group, 10-methyloctadecyl group, 11-methyloctadecyl group, 12-methyloctadecyl group, 13-methyloctadecyl group, 14-methyloctadecyl group, 15-methyloctadecyl group, 16-methyloctadecyl group, 17-methyloctadecyl group;
n-icosyl group, 1-methylnonadecyl group, 2-methylnonadecyl group, 3-methylnonadecyl group, 4-methylnonadecyl group, 5-methylnonadecyl group, 6-methylnonadecyl group, 7-methylnonadecyl group, 8-methylnonadecyl group, 9-methylnonadecyl group, 10-methylnonadecyl group, 11-methylnonadecyl group, 12-methylnonadecyl group, 13-methylnonadecyl group, 14-methylnonadecyl group, 15-methylnonadecyl group, 16-methylnonadecyl group, 17-methylnonadecyl group, 18-methylnonadecyl group;
n-henicosyl group, 1-methylicosyl group, 2-methylicosyl group, 3-methylicosyl group, 4-methylicosyl group, 5-methylicosyl group, 6-methylicosyl group, 7-methylicosyl group, 8-methylicosyl group, 9-methylicosyl group, 10-methylicosyl group, 11-methylicosyl group, 12-methylicosyl group, 13-methylicosyl group, 14-methylicosyl group, 15-methylicosyl group, 16-methylicosyl group, 17-methylicosyl group, 18-methylicosyl group, 19-methylicosyl group;
n-docosyl group, 1-methylhenicosyl group, 2-methylhenicosyl group, 3-methylhenicosyl group, 4-methylhenicosyl group, 5-methylhenicosyl group, 6-methylhenicosyl group, 7-methylhenicosyl group, 8-methylhenicosyl group, 9-methylhenicosyl group, 10-methylhenicosyl group, 11-methylhenicosyl group, 12-methylhenicosyl group, 13-methylhenicosyl group, 14-methylhenicosyl group, 15-methylhenicosyl group, 16-methylhenicosyl group, 17-methylhenicosyl group, 18-methylhenicosyl group, 19-methylhenicosyl group, 20-methylhenicosyl group; and the like.

When R²¹ to R²⁶ are C₂₋₂₂ alkenyl groups, examples of the alkenyl groups include groups in which 1 to 3 of the saturated bonds between carbon molecules in the groups listed as the C₂₋₂₂ alkyl groups are replaced by unsaturated bonds.

When the pH-sensitive cationic lipid represented by the general formula (I) or (I') is used as a constituent lipid of lipid nanoparticles, the three R¹ in the general formulae (I) and (I') groups represent regions that interact with hydrophobic portions of other lipid molecules constituting the lipid nanoparticles. When the hydrocarbon groups contained in R¹ are composed of a sufficient number of carbon atoms, the hydrophobic interactions between the pH-sensitive cationic lipid represented by the general formula (I) or (I') and other lipid molecules are enhanced, thereby enabling the resulting lipid nanoparticle to stably retain encapsulated substances such as nucleic acids. In this respect, the hydrocarbon groups contained in R¹ correspond to R²¹ and R²² in the general formula (T-1), R²³ and R²⁴ in the general formula (T-2), and R²⁵ and R²⁶ in the general formula (T-3).

In the general formula (T-1), the total number of carbon atoms in R²¹ and R²² is equal to or greater than 8, preferably equal to or greater than 10, and more preferably equal to or greater than 12. In the general formula (T-1), the total number of carbon atoms in R²¹ and R²² is preferably equal to or less than 28, more preferably equal to or less than 26, and even more preferably equal to or less than 24.

In the general formula (T-2), the total number of carbon atoms in R²³ and R²⁴ is equal to or greater than 6, preferably equal to or greater than 8, and more preferably equal to or greater than 10. In the general formula (T-2), the total number of carbon atoms in R²³ and R²⁴ is preferably equal to or less than 26, more preferably equal to or less than 24, and even more preferably equal to or less than 22.

In the general formula (T-3), the total number of carbon atoms in R²⁵ and R²⁶ is equal to or greater than 6, preferably equal to or greater than 8, and more preferably equal to or greater than 10. In the general formula (T-3), the total number of carbon atoms in R²⁵ and R²⁶ is preferably equal to or less than 26, more preferably equal to or less than 24, and even more preferably equal to or less than 22.

In the general formula (I), Z² is a linear alkylene group having 4 to 6 carbon atoms (linear C₄₋₆ alkylene group) or a cycloalkylene group. The number of carbon atoms in the cycloalkylene group is preferably from 3 to 7, more preferably from 4 to 6. As Z², a linear C₄₋₆ alkylene group is preferred, and a linear alkylene group having 4 or 5 carbon atoms (i.e., a n-butylene group or a n-pentylene group) is particularly preferred. In the pH-sensitive cationic lipid represented by the general formula (I), the hydrocarbon chains R¹ are linked to a tris skeleton via a spacer having the structure -CO-Z²-CO-, thereby providing the hydrocarbon chains R¹ with sufficient conformational flexibility.

In the general formula (I'), "s" is 2, 3, or 4, preferably 2 or 3. In an embodiment, "s" is 2. In another embodiment, "s" is 3. In yet another embodiment, "s" is 4. In the pH-sensitive cationic lipid represented by the general formula (I'), the hydrocarbon chains R¹ are linked to a tris skeleton via a spacer having the structure -CO-(CH₂)ₛ-CO-, thereby providing the hydrocarbon chains R¹ with sufficient conformational flexibility.

In the general formulae (I) and (I'), three R¹ groups in one molecule may be the same as or different from each other. In the pH-sensitive cationic lipid according to the present invention, from the viewpoint of ease of synthesis and quality stability, it is preferable that all three R¹ groups in one molecule are the same.

In the general formula (I), three Z² in one molecule may be the same as or different from each other. In the pH-sensitive cationic lipid according to the present invention, from the viewpoint of ease of synthesis and quality stability, it is preferable that all three Z² groups in one molecule are the same.

In the general formula (I'), three "s" in one molecule may be the same as or different from each other. In the pH-sensitive cationic lipid according to the present invention, from the viewpoint of ease of synthesis and quality stability, it is preferable that all three "s" in one molecule are the same.

In the pH-sensitive cationic lipid according to the present invention, it is preferable that R¹ is any group of the formula (A8), (A8β), (A10), (A12), (A12β), (A14), (A16), (A16β), (A16b), (A20), (A24), (E12), (E14), (A14β), (A24β), (A10L), (A12L), or (A14L).

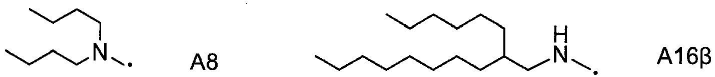

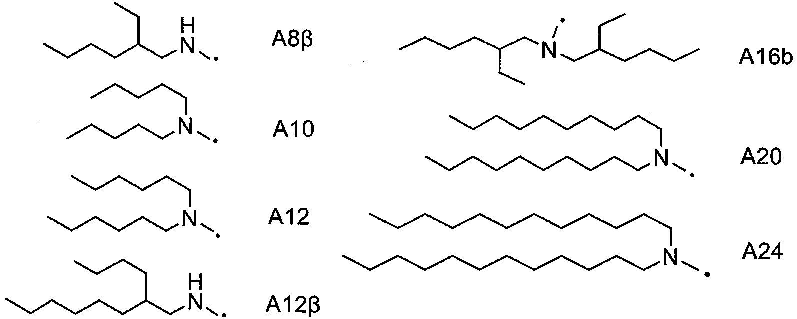

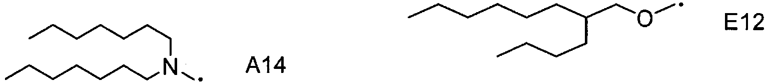

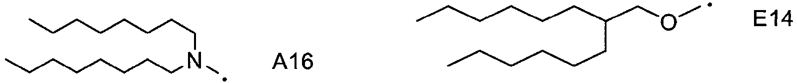

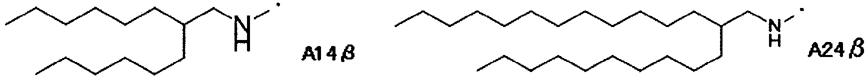

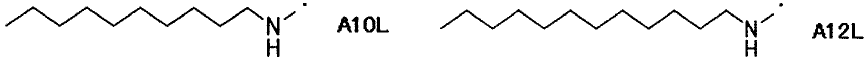

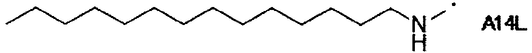

In one embodiment, R¹ is preferably a group represented by any one of the following formula (A8), (A8β), (A10), (A12), (A12β), (A14), (A16), (A16β), (A16b), (A20), (A24), (E12), or (E14).

In the general formulae (I) and (I'), Z¹ is a C₁₋₆ alkylene group which may be substituted with one hydroxyl group, or a single bond. The C₁₋₆ alkylene group may be linear or branched. Examples of the C₁₋₆ alkylene group include a methylene group, an ethylene group, a 1-methylmethylene group, a 1-hydroxymethylmethylene group, an n-propylene group, a 1-methylethylene group, a 1-hydroxymethylethylene group, a 2-methylethylene group, an n-butylene group, a 1-methylpropylene group, a 1-hydroxymethylpropylene group, a 2-methylpropylene group, a 3-methylpropylene group, a 1-ethylethylene group, a 2-ethylethylene group, an n-pentylene group, a 1-methylbutylene group, a 2-methylbutylene group, a 3-methylbutylene group, a 4-methylbutylene group, a 1-ethylpropylene group, a 2-ethylpropylene group, a 3-ethylpropylene group, an n-hexylene group, a 1-methylpentylene group, a 2-methylpentylene group, a 3-methylpentylene group, a 4-methylpentylene group, a 1-ethylbutylene group, a 2-ethylbutylene group, and a 3-ethylbutylene group. In the pH-sensitive cationic lipid according to the present invention, a compound in which Z¹ is a methylene group, an ethylene group, an n-propylene group, a 1-hydroxymethylmethylene group, a 1-methylmethylene group, a 1-methylethylene group, a 1-hydroxymethylethylene group, or a 2-methylethylene group is preferable, and a compound in which Z¹ is a methylene group, an ethylene group, an n-propylene group, or a 1-hydroxymethylmethylene group is more preferable.

In the general formulae (I) and (I'), X is -N(R²)(R³) or a 5- to 7-membered nonaromatic heterocyclic group. The 5- to 7-membered nonaromatic heterocyclic group represented by X is bonded to Z¹ via a carbon atom.

In the general formulae (I) and (I'), when X is -N(R²)(R³), R² and R³ are each independently a hydrogen atom or a C₁₋₄ hydrocarbon group in which one or two hydrogen atoms may be substituted with a phenyl group, a pyrrolyl group, a pyrimidyl group, or an indolyl group. The C₁₋₄ hydrocarbon group may be a linear or branched C₁₋₄ alkyl group or a linear or branched C₂₋₄ alkenyl group.

When R² and R³ are C₁₋₄ alkyl groups, examples of the C₁₋₄ alkyl groups include a methyl group, an ethyl group, a propyl group, an isopropyl group, an n-butyl group, an isobutyl group, and a tert-butyl group. When R² and R³ are C₂₋₄ alkenyl groups, examples of the C₂₋₄ alkenyl groups include a vinyl group, a 1-propenyl group, a 2-propenyl group, a 1-methylvinyl group, a 2-methyl-1-propenyl group, a 1-butenyl group, a 2-butenyl group, and a 3-butenyl group. In the pH-sensitive cationic lipid according to the present invention, in the general formulae (I) and (I'), when X is -N(R²)(R³), a compound in which R² and R³ are each independently a hydrogen atom or a linear or branched C₁₋₄ alkyl group is preferable, a compound in which R² and R³ are each independently a hydrogen atom or a linear C₁₋₄ alkyl group is more preferable, and a compound in which R² and R³ are each independently a hydrogen atom, a methyl group, an ethyl group, or an n-propyl group is still more preferable.

In the general formulae (I) and (I'), when X is -N(R²)(R³), R² and R³ may be bonded to each other to form a 5- to 7-membered nonaromatic heterocycle. Examples of the 5- to 7-membered nonaromatic heterocycle formed by R² and R³ being bonded to each other include a 1-pyrrolidinyl group, a 1-piperidinyl group, a 1-morpholinyl group, and a 1-piperazinyl group.

When X is -N(R²)(R³) and R² and R³ are bonded to each other to form a 5- to 7-membered nonaromatic heterocycle, one or more hydrogen atoms, preferably one or two hydrogen atoms, in the formed 5- to 7-membered nonaromatic heterocycle may be substituted with a C₁₋₄ hydrocarbon group, an amide group, or a nitrogen-containing cyclic group. The C₁₋₄ hydrocarbon group is preferably a C₁₋₄ alkyl group or a C₂₋₄ alkenyl group. When two hydrogen atoms in the ring are substituted with a C₁₋₄ alkyl group or a C₂₋₄ alkenyl group, they may be substituted with the same group or with different groups. The nitrogen-containing cyclic group is preferably a group derived from a ring in which a carbonyl group is adjacent to a nitrogen atom, and examples thereof include a group derived from a nitrogen-containing ring such as benzo[d]oxazol-2(3H)-one, 1,3-dihydro-2H-benzo[d]imidazol-2-one, indolin-2-one, 1,3-dihydro-2H-pyrrol-2-one, 1,3-dihydro-2H-imidazol-2-one, oxazol-2(3H)-one, oxazolidin-2-one, imidazolidin-2-one, pyrrolidin-2-one, 1,4-dihydroisoquinolin-3(2H)-one, 3,4-dihydro-2H-benzo[e][1,3]oxazin-2-one, 3,4-dihydroquinazolin-2(1H)-one, 3,4-dihydroquinolin-2(1H)-one, 1,4-dihydro-2H-benzo[d][1,3]oxazin-2-one, 3,4-dihydropyridin-2(1H)-one, 3,6-dihydropyridin-2(1H)-one, 3,6-dihydro-2H-1,3-oxazin-2-one, 3,4-dihydro-2H-1,3-oxazin-2-one, 3,4-dihydropyrimidin-2(1H)-one, piperidin-2-one, 1,3-oxazinan-2-one, or tetrahydropyrimidin-2(1H)-one.

When X is -N(R²)(R³) and R² and R³ are bonded to each other to form a 5- to 7-membered nonaromatic heterocycle, one of the carbon atoms constituting the 5- to 7-membered nonaromatic heterocycle may be a carbon atom constituting other nitrogen-containing cyclic group. The other nitrogen-containing cyclic group is preferably a group derived from a ring in which a carbonyl group is adjacent to a nitrogen atom, and specific examples thereof include the same groups as described above.

In the general formulae (I) and (I'), when X is a 5- to 7-membered nonaromatic heterocyclic group, examples of heteroatoms in the heterocyclic group include a nitrogen atom, an oxygen atom, and a sulfur atom. The heterocyclic group may have one heteroatom, or two or more heteroatoms which may be the same or different, constituting the heterocycle. The heterocycle in the heterocyclic group may be a saturated heterocycle or may contain one or two or more double bonds, but the heterocycle is not an aromatic ring.

When X is a 5- to 7-membered nonaromatic heterocyclic group, the heterocyclic group is bonded to Z¹ via a carbon atom, and the ring may be substituted to have one or two C₁₋₄ hydrocarbon groups or amide groups thereon. The C₁₋₄ hydrocarbon group may be a linear or branched C₁₋₄ alkyl group or a linear or branched C₂₋₄ alkenyl group. As the linear or branched C₁₋₄ alkyl group and the linear or branched C₂₋₄ alkenyl group, those mentioned above can be used.

In the general formulae (I) and (I'), -Z¹-X is preferably any one group of the following formulae (H-1) to (H-8) and (H-16) to (H-26). In the following formulae, black circles represent bonding arms.

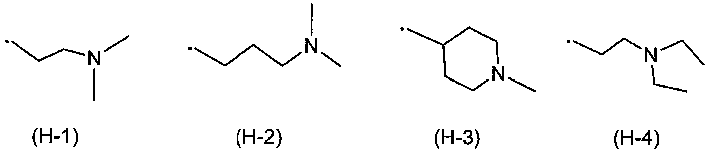

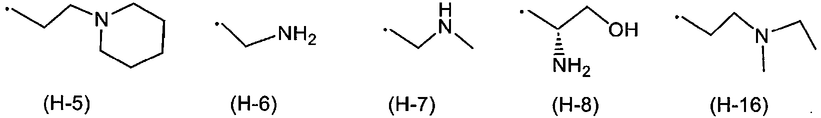

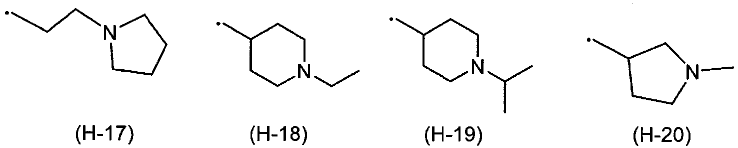

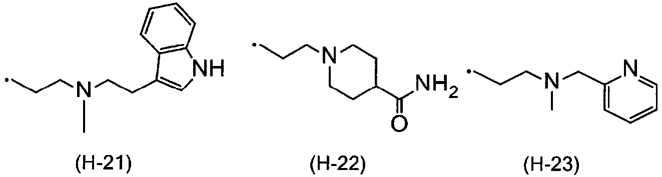

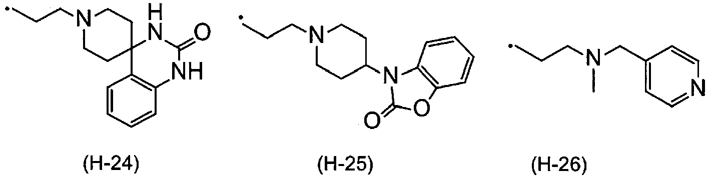

Preferred examples of the pH-sensitive cationic lipid according to the present invention include compounds represented by the general formula (I) in which -Z¹-X is a group represented by any one of the formulae (H-1) to (H-8) and (H-16) to (H-26); more preferably, in the general formula (I), R¹ is a group represented by any of the formula (A8), (A8β), (A10), (A12), (A12β), (A14), (A16), (A16β), (A16b), (A20), (A24), (E12), (E14), (A14β), (A24β), (A10L), (A12L), or (A14L), and -Z¹-X is a group represented by any one of the formulae (H-1) to (H-8) and (H-16) to (H-26); even more preferably, in the general formula (I), R¹ is a group represented by any of the formula (A8), (A8β), (A10), (A12), (A12β), (A14), (A16), (A16β), (A16b), (A20), (A24), (E12), (E14), (A14β), (A24β), (A10L), (A12L), or (A14L), Z² is a linear C₄₋₆ alkylene group, and -Z¹-X is a group represented by any one of the formulae (H-1) to (H-8) and (H-16) to (H-26); still more preferably, in the general formula (I), R¹ is a group represented by any of the formula (A8), (A8β), (A10), (A12), (A12β), (A14), (A16), (A16β), (Al6b), (A20), (A24), (E12), (E14), (A14β), (A24β), (A10L), (A12L), or (A14L), Z² is an n-butylene group, and -Z¹-X is a group represented by any one of the formulae (H-1) to (H-8) and (H-16) to (H-26), or R¹ is a group represented by any of the formula (A8), (A8β), (A10), (A12), (A12β), (A14), (A16), (A16β), (A16b), (A20), (A24), (E12), (E14), (A14β), (A24β), (A10L), (A12L), or (A14L), Z² is an n-pentylene group, and -Z¹-X is a group represented by any one of the formulae (H-1) to (H-8) and (H-16) to (H-26).

In one embodiment, more preferably, in the general formula (I), R¹ is a group represented by any of the formula (A8), (A8β), (A10), (A12), (A12β), (A14), (A16), (A16β), (A16b), (A20), (A24), (E12), or (E14), and -Z¹-X is a group represented by any one of formulae (H-1) to (H-8) and (H-16) to (H-26); even more preferably, in the general formula (I), R¹ is a group represented by any of the formula (A8), (A8β), (A10), (A12), (A12β), (A14), (A16), (A16β), (A16b), (A20), (A24), (E12), or (E14), Z² is a linear C₄₋₆ alkylene group, and -Z¹-X is a group represented by any one of the formulae (H-1) to (H-8) and (H-16) to (H-26); still more preferably, in the general formula (I), R¹ is a group represented by any of the formula (A8), (A8β), (A10), (A12), (A12β), (A14), (A16), (A16β), (A16b), (A20), (A24), (E12), or (E14), Z² is an n-butylene group, and -Z¹-X is a group represented by any one of the formulae (H-1) to (H-8) and (H-16) to (H-26), or R¹ is a group represented by any of the formula (A8), (A8β), (A10), (A12), (A12β), (A14), (A16), (A16β), (A16b), (A20), (A24), (E12), or (E14), Z² is an n-pentylene group, and - Z¹-X is a group represented by any one of the formulae (H-1) to (H-8) and (H-16) to (H-26).

Preferred examples of the pH-sensitive cationic lipid according to the present invention include compounds represented by the general formula (I') in which -Z¹-X is a group represented by any one of the formulae (H-1) to (H-8) and (H-16) to (H-26); more preferably, in the general formula (I'), R¹ is a group represented by any of the formula (A8), (A8β), (A10), (A12), (A12β), (A14), (A16), (A16β), (A16b), (A20), (A24), (E12), (E14), (A14β), (A24β), (A10L), (A12L), or (A14L), and -Z¹-X is a group represented by any one of the formulae (H-1) to (H-8) and (H-16) to (H-26); even more preferably, in the general formula (I'), R¹ is a group represented by any of the formula (A8), (A8β), (A10), (A12), (A12β), (A14), (A16), (A16β), (A16b), (A20), (A24), (E12), (E14), (A14β), (A24β), (A10L), (A12L), or (A14L), "s" is 2 or 3, and -Z¹-X is a group represented by any one of the formulae (H-1) to (H-8) and (H-16) to (H-26); still more preferably, in the general formula (I'), R¹ is a group represented by any of the formula (A8), (A8β), (A10), (A12), (A12β), (A14), (A16), (A16β), (A16b), (A20), (A24), (E12), (E14), (A14β), (A24β), (A10L), (A12L), or (A14L), "s" is 2, and -Z¹-X is a group represented by any one of the formulae (H-1) to (H-8) and (H-16) to (H-26), or R¹ is a group represented by any of the formula (A8), (A8β), (A10), (A12), (A12β), (A14), (A16), (A16β), (A16b), (A20), (A24), (E12), (E14), (A14β), (A24β), (A10L), (A12L), or (A14L), "s" is 3, and - Z¹-X is a group represented by any one of the formulae (H-1) to (H-8) and (H-16) to (H-26).

In one embodiment, more preferably, in the general formula (I'), R¹ is a group represented by any of the formula (A8), (A8β), (A10), (A12), (A12β), (A14), (A16), (A16β), (A16b), (A20), (A24), (E12), or (E14), and -Z¹-X is a group represented by any one of the formulae (H-1) to (H-8) and (H-16) to (H-26); even more preferably, in the general formula (I'), R¹ is a group represented by any of the formula (A8), (A8β), (A10), (A12), (A12β), (A14), (A16), (A16β), (A16b), (A20), (A24), (E12), or (E14), "s" is 2 or 3, and -Z¹-X is a group represented by any one of the formulae (H-1) to (H-8) and (H-16) to (H-26); even more preferably, in the general formula (I'), R¹ is a group represented by any of the formula (A8), (A8β), (A10), (A12), (A12β), (A14), (A16), (A16β), (A16b), (A20), (A24), (E12), or (E14), "s" is 2, and -Z¹-X is a group represented by any one of the formulae (H-1) to (H-8) and (H-16) to (H-26), or R¹ is a group represented by any of the formula (A8), (A8β), (A10), (A12), (A12β), (A14), (A16), (A16β), (A16b), (A20), (A24), (E12), or (E14), "s" is 3, and -Z¹-X is a group represented by any one of the formulae (H-1) to (H-8) and (H-16) to (H-26).

In the pH-sensitive cationic lipid according to the present invention, three hydrocarbon chains composed of R¹ extend from one Tris skeleton via a spacer, and a hydrophilic group composed of X is further linked to the Tris skeleton via Z¹. Therefore, the pH-sensitive cationic lipid according to the present invention can take an orientation in which the hydrophilic group composed of X is sterically shielded by the three hydrocarbon chains composed of R¹. On the other hand, the pH-sensitive cationic lipid described in Patent Literature 1 such as CL4H6 has two hydrophobic chains and a hydrophilic group, and the hydrophilic group and the hydrophobic chains are in a positional relationship such that they are separated from each other. Because of these differences in steric structure, the pH-sensitive cationic lipid according to the present invention can be expected to have different properties from the conventional pH-sensitive cationic lipid such as CL4H6 when used as a constituent lipid of a lipid nanoparticle.

The pKa of the pH-sensitive cationic lipid represented by the general formulae (I) and (I') is not particularly limited, but can be selected, for example, in the range of about 4.0 to about 9.0, preferably about 4.5 to about 8.5, more preferably about 5.0 to about 8.5, and it is preferable to select the type of each substituent so as to give a pKa within this range. The pKa of the pH-sensitive cationic lipid is particularly affected by -Z¹-X in the general formulae (I) and (I'). For example, a pH-sensitive cationic lipid having a desired pKa can be obtained by setting -Z¹-X in the general formulae (I) and (I') to a group having a pKa close to the desired pKa from among the formulae (H-1) to (H-8) and (H-16) to (H-26).

The pH-sensitive cationic lipid according to the present invention can be produced, for example, by condensing a compound represented by the following general formula (A) (hereinafter sometimes referred to as "compound (A)"), a compound represented by the following general formula (B) (hereinafter sometimes referred to as "compound (B)"), and tris(hydroxymethyl)aminomethane. R¹ and Z² in the general formula (A) are the same as R¹ and Z² in the general formula (I) , respectively. Z¹ and X in the general formula (B) are the same as Z¹ and X in the general formula (I), respectively.

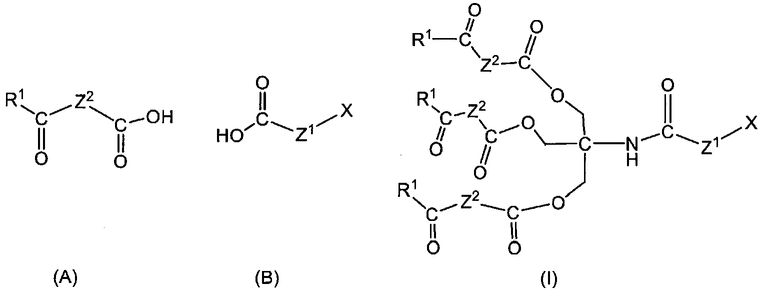

The pH-sensitive cationic lipid represented by the general formula (I) is synthesized by subjecting the carboxylic acid group of the compound (A) and the hydroxyl group of Tris to dehydration condensation to form an ester bond, and further subjecting the carboxylic acid group of the compound (B) and the amino group of Tris to dehydration condensation to form an amide bond. The dehydration condensation reaction of the carboxylic acid group of the compound (A) with the hydroxyl group of Tris can be generally carried out under the same reaction conditions as the dehydration condensation reaction in forming an ester of a carboxylic acid and an alcohol. The dehydration condensation reaction of the carboxylic acid group of the compound (B) with the amino group of Tris can be generally carried out under the same reaction conditions as the dehydration condensation reaction in forming an amide of a carboxylic acid and an amine. That is, the pH-sensitive cationic lipid represented by the general formula (I) can be relatively easily synthesized by a general dehydration condensation reaction using Tris as a linker.

The pH-sensitive cationic lipid represented by the general formula (I) can be synthesized by reacting Tris with the compound (A) to carry out a dehydration condensation reaction with the hydroxyl group of Tris, and then reacting the resulting ester with the compound (B) to carry out a dehydration condensation reaction with the Tris-derived amino group. The pH-sensitive cationic lipid represented by the general formula (I) can also be synthesized by reacting Tris with the compound (B) to carry out a dehydration condensation reaction with the amino group of Tris, and then reacting the resulting amide with compound (A) to carry out a dehydration condensation reaction with the Tris-derived hydroxyl group.

The compound (A) wherein Z² is a linear C₂₋₄ alkylene group, i.e., a compound represented by the following general formula (A') (hereinafter sometimes referred to as "compound (A')") can be produced by ring-opening condensation of a compound represented by the following general formula (A-0) with a dicarboxylic acid anhydride represented by the following general formula (D) (hereinafter sometimes referred to as "compound (D)"). In the general formula (A-0), R¹ is the same as defined in the general formula (A). In the general formula (A'), "s" represents 2, 3, or 4, and "s" in the general formula (D) is the same as defined in the general formula (A').

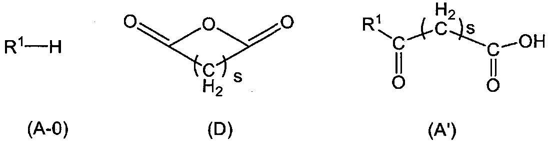

The ring-opening condensation reaction of the compound (A-0) with the compound (D) can be generally carried out under the same reaction conditions as the dehydration condensation reaction in forming an amide of an acid anhydride and a primary or secondary amine or an ester of an acid anhydride and an alcohol.

The pH-sensitive cationic lipid represented by the general formula (I') can be synthesized by forming an ester bond through dehydration condensation between the carboxy group of the produced compound (A') and the hydroxyl group of Tris, followed by forming an amide bond through dehydration condensation between the carboxy group of the compound (B) and the amino group of Tris.

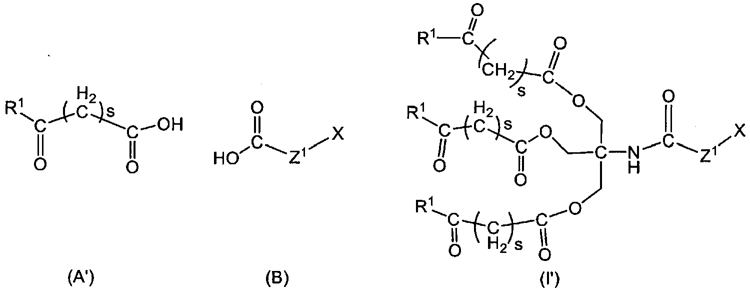

Among the pH-sensitive cationic lipids represented by the general formula (I) or (I'), compounds in which Z¹ is an ethylene group and X is -N(R²)(R³) (i.e., compounds represented by the general formula (I-a) or (I'-a)) can be more easily synthesized by using N-[tris(hydroxymethyl)methyl]acrylamide. Specifically, these compounds can be produced by adding N-[tris(hydroxymethyl)methyl]acrylamide to a compound represented by the general formula (C), and then condensing the resulting compound represented by the general formula (E) with a compound represented by the general formula (A) or (A').

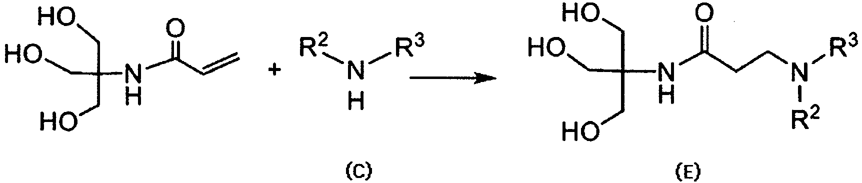

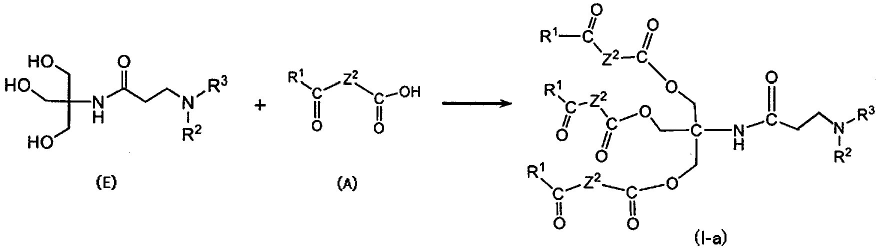

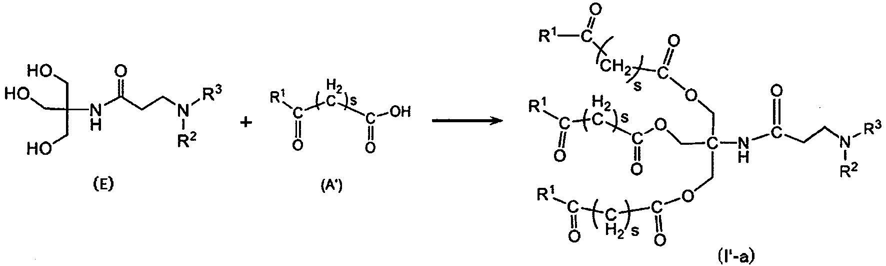

In the general formula (C), R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group. R² and R³ in the general formula (E) are the same as R² and R³ in the general formula (C), respectively.

R¹ and Z² in the general formula (A) are the same as R¹ and Z² in the general formula (I), respectively. Z¹ and "s" in the general formula (A') are the same as Z¹ and "s" in the general formula (I'), respectively.

In the general formula (I-a), R¹ and Z² are the same as R¹ and Z² in the general formula (I), respectively, and R² and R³ are the same as R² and R³ in the general formula (C), respectively. In the general formula (I'-a), R¹ and "s" are the same as R¹ and "s" in the general formula (I'), respectively, and R² and R³ are the same as R² and R³ in the general formula (C), respectively.

The addition reaction of the compound represented by the general formula (C) to the unsaturated bond in N-[tris(hydroxymethyl)methyl]acrylamide can be generally carried out under the same reaction conditions as the addition reaction in adding a secondary amine to an alkene. The dehydration condensation reaction of the carboxylic acid group of the compound represented by the general formula (A) or (A') with the hydroxyl group of the compound represented by the general formula (E) can be generally carried out under the same reaction conditions as the dehydration condensation reaction in forming an ester of a carboxylic acid and an alcohol. That is, the pH-sensitive cationic lipid represented by the general formula (I-a) or (I'-a) can be relatively easily synthesized by a general addition reaction and dehydration condensation reaction using N-[tris(hydroxymethyl)methyl]acrylamide.

The pH-sensitive cationic lipid represented by the general formula (I) or (I') can be easily produced, for example, by the method specifically shown in the Examples of the present specification. By referring to this production method and appropriately selecting starting compounds, reagents, reaction conditions, and the like, those skilled in the art can easily produce any lipid included in the scope of the general formula (I) or (I').

### [Lipid Nanoparticle]

The lipid nanoparticle according to the present invention comprises the pH-sensitive cationic lipid according to the present invention as a constituent lipid. The lipid nanoparticle according to the present invention may comprise only one kind or two or more kinds of the pH-sensitive cationic lipid according to the present invention. When the lipid nanoparticle according to the present invention comprises two or more kinds of the pH-sensitive cationic lipid according to the present invention, the amount of the pH-sensitive cationic lipid according to the present invention means the total amount of lipid molecules corresponding to the pH-sensitive cationic lipid according to the present invention among the lipid molecules constituting the lipid nanoparticle.

The proportion of the pH-sensitive cationic lipid according to the present invention in the lipid molecules constituting the lipid nanoparticle is not particularly limited. For example, in the lipid nanoparticle according to the present invention, the ratio of the amount of the pH-sensitive cationic lipid according to the present invention to the total amount of lipids constituting the lipid nanoparticle ([amount of pH-sensitive cationic lipid according to the present invention (mol)]/([total amount of lipid constituting the lipid nanoparticle (mol)])×100%) is preferably 5 mol% or more, more preferably 10 mol% or more, and further preferably 20 mol% or more. On the other hand, if the proportion of the pH-sensitive cationic lipid in the lipid molecules constituting the lipid nanoparticle is too high, the content of other lipids for imparting desired properties to the lipid nanoparticle may be too low. Therefore, the ratio of the amount of the pH-sensitive cationic lipid according to the present invention to the total amount of lipids constituting the lipid nanoparticle in the lipid nanoparticle according to the present invention is preferably 90 mol% or less, more preferably 80 mol% or less, and further preferably 70 mol% or less.

As the lipid other than the pH-sensitive cationic lipid according to the present invention among the constituent lipids of the lipid nanoparticle according to the present invention, lipids generally used in forming liposomes can be used. Examples of such lipids include phospholipids, sterols, glycolipids, and saturated or unsaturated fatty acids. These can be used alone or in combination of two or more.

Examples of phospholipids include glycerophospholipids such as phosphatidylserine, phosphatidylinositol, phosphatidylglycerol, phosphatidylethanolamine, phosphatidylcholine, cardiolipin, plasmalogen, ceramide phosphorylglycerol phosphate, and phosphatidic acid; and sphingophospholipids such as sphingomyelin, ceramide phosphorylglycerol, and ceramide phosphoryl ethanolamine. Naturally occurring phospholipids such as egg yolk lecithin and soybean lecithin can also be used. The fatty acid residues in glycerophospholipids and sphingophospholipids are not particularly limited, but examples thereof include saturated or unsaturated fatty acid residues having 12 to 24 carbon atoms, and saturated or unsaturated fatty acid residues having 14 to 20 carbon atoms are preferable. Specific examples include acyl groups derived from fatty acids such as lauric acid, myristic acid, palmitic acid, palmitoleic acid, stearic acid, oleic acid, linoleic acid, linolenic acid, arachidic acid, arachidonic acid, behenic acid, and lignoceric acid. When these glycerolipids or sphingolipids have two or more fatty acid residues, all the fatty acid residues may be the same group or different groups.

Examples of sterols include animal-derived sterols such as cholesterol, cholesterol hemisuccinate, lanosterol, dihydrolanosterol, desmosterol, and dihydrocholesterol; plant-derived sterols (phytosterols) such as stigmasterol, sitosterol, campesterol, and brassicasterol; and microorganism-derived sterols such as timosterol and ergosterol. Examples of glycolipids include glycosylglycerols such as sulfoxyribosyl glyceride, diglycosyl diglyceride, digalactosyl diglyceride, galactosyl diglyceride, and glycosyl diglyceride; and glycosphingolipids such as galactosylceramide, lactosylceramide, and gangliosides. Examples of saturated or unsaturated fatty acids include saturated or unsaturated fatty acids having 12 to 20 carbon atoms such as palmitic acid, oleic acid, stearic acid, arachidonic acid, and myristic acid.

In addition to the pH-sensitive cationic lipid according to the present invention, it is preferable that the constituent lipids of the lipid nanoparticle according to the present invention include a neutral lipid, more preferably a phospholipid or a sterol, further preferably a sterol, and still more preferably cholesterol.

The lipid nanoparticle according to the present invention preferably contains a polyalkylene glycol-modified lipid as a lipid component. Since polyalkylene glycol is a hydrophilic polymer, the surface of a lipid nanoparticle can be modified with polyalkylene glycol by constructing the lipid nanoparticle using a polyalkylene glycol-modified lipid as a constituent lipid of a lipid membrane. Surface modification with polyalkylene glycol may enhance the stability of the lipid nanoparticle such as the blood retention property.

As the polyalkylene glycol, for example, polyethylene glycol, polypropylene glycol, polytetramethylene glycol, polyhexamethylene glycol, or the like can be used. The molecular weight of the polyalkylene glycol is, for example, about 300 to about 10,000, preferably about 500 to about 10,000, and further preferably about 1,000 to about 5,000.

For example, stearylated polyethylene glycol (e.g., stearic acid PEG45 (STR-PEG45)) can be used for modification of a lipid with polyethylene glycol. In addition, polyethylene glycol derivatives such as N-[carbonyl-methoxypolyethylene glycol-2000]-1,2-dipalmitoyl-sn-glycero-3-phosphoethanolamine, N-[carbonyl-methoxypolyethylene glycol-5000]-1,2-dipalmitoyl-sn-glycero-3-phosphoethanolamine, N-[carbonyl-methoxypolyethylene glycol-750]-1,2-distearoyl-sn-glycero-3-phosphoethanolamine, N-[carbonyl-methoxypolyethylene glycol-2000]-1,2-distearoyl-sn-glycero-3-phosphoethanolamine, N-[carbonyl-methoxypolyethylene glycol-5000]-1,2-distearoyl-sn-glycero-3-phosphoethanolamine, and 1,2-dimyristoyl-rac-glycero-3-methoxypolyethylene glycol-2000 (PEG-DMG) can also be used, but polyalkylene glycolated lipids are not limited thereto.

The proportion of the polyalkylene glycol-modified lipid to the total amount of lipids constituting the lipid nanoparticle according to the present invention is not particularly limited as long as it does not impair the selectivity of the pH-sensitive cationic lipid according to the present invention to the target tissue, specifically, the target tissue-specific gene expression activity when the lipid nanoparticle according to the present invention is used as a gene expression carrier. For example, the proportion of the polyalkylene glycol-modified lipid to the total amount of lipids constituting the lipid nanoparticle is preferably 0.5 to 3 mol%.

The lipid nanoparticle according to the present invention can be subjected to appropriate surface modification or the like as necessary. The blood retention property of the lipid nanoparticle according to the present invention can be enhanced by modifying the surface with a hydrophilic polymer or the like. Surface modification may be performed by using a lipid modified with these modifying groups as a constituent lipid of the lipid nanoparticle.

In producing the lipid nanoparticle according to the present invention, for example, glycophorin, ganglioside GM1, phosphatidylinositol, ganglioside GM3, glucuronic acid derivative, glutamic acid derivative, polyglycerin phospholipid derivative, or the like can also be used as a lipid derivative for enhancing the blood retention property. In addition to polyalkylene glycol, dextran, pullulan, Ficoll, polyvinyl alcohol, styrenemaleic anhydride alternating copolymer, divinyl ether-maleic anhydride alternating copolymer, amylose, amylopectin, chitosan, mannan, cyclodextrin, pectin, carrageenan, or the like can also be used for surface modification as the hydrophilic polymer for enhancing the blood retention property.

Further, in order to promote nuclear translocation of the lipid nanoparticle according to the present invention, for example, the surface of the lipid nanoparticle can be modified with an oligosaccharide compound having three or more saccharides. The type of the oligosaccharide compound having three or more saccharides is not particularly limited, but for example, an oligosaccharide compound in which about 3 to about 10 sugar units are bonded can be used, and preferably an oligosaccharide compound in which about 3 to about 6 sugar units are bonded can be used. Among them, preferably, an oligosaccharide compound which is a trimer to hexamer of glucose can be used, and more preferably, an oligosaccharide compound which is a trimer or tetramer of glucose can be used. More specifically, isomaltotriose, isopanose, maltotriose, maltotetraose, maltopentaose, or maltohexaose can be preferably used, and among them, maltotriose, maltotetraose, maltopentaose, or maltohexaose is more preferable, in which glucoses are bonded through an α1-4 linkage. Particularly preferable is maltotriose or maltotetraose, and most preferable is maltotriose. The amount of the surface modification of the lipid nanoparticle with the oligosaccharide compound is not particularly limited, but is, for example, about 1 to about 30 mol%, preferably about 2 to about 20 mol%, and more preferably about 5 to about 10 mol% with respect to the total lipid amount.

The method for modifying the surface of the lipid nanoparticle with an oligosaccharide compound is not particularly limited; for example, a liposome in which the surface of the lipid nanoparticle is modified with a monosaccharide such as galactose or mannose (International Publication No. WO 2007/102481) is known, and thus the surface modification method described in this publication can be employed. All of the disclosure of the above-mentioned publication is incorporated into the disclosure of this specification by reference.

Further, the lipid nanoparticle according to the present invention can be imparted with, for example, any one or two or more functions such as a temperature change-sensitive function, a membrane permeation function, a gene expression function, and a pH-sensitive function. By appropriately adding these functions, it is possible to improve the retention property of the lipid nanoparticle in blood, efficiently release the lipid nanoparticle from the endosome after endocytosis in the target cell, and more efficiently express the encapsulated nucleic acid in the target cell.

The lipid nanoparticle according to the present invention may contain one or more substances selected from the group consisting of antioxidants such as tocopherol, propyl gallate, ascorbyl palmitate, or butylated hydroxytoluene, charged substances, and membrane polypeptides. Examples of the charged substances for imparting a positive charge include saturated or unsaturated aliphatic amines such as stearylamine and oleylamine. Examples of the charged substances for imparting a negative charge include dicetyl phosphate, cholesteryl hemisuccinate, phosphatidylserine, phosphatidylinositol, and phosphatidic acid. Examples of membrane polypeptides include peripheral membrane polypeptides and integral membrane polypeptides. The blending amount of these substances is not particularly limited and can be appropriately selected depending on the purposes.

The size of the lipid nanoparticle according to the present invention is preferably an average particle size of 450 nm or less, more preferably an average particle size of 300 nm or less, further preferably an average particle size of 200 nm or less, and still more preferably 160 nm or less, since a high delivery efficiency to target cells in vivo can be easily obtained. The average particle size of the lipid nanoparticle means a number average particle size measured by dynamic light scattering (DLS). Measurement by dynamic light scattering can be carried out by a conventional method using a commercially available DLS apparatus or the like.

The polydispersity index (PDI) of the lipid nanoparticle according to the present invention is about 0.01 to about 0.7, preferably about 0.01 to about 0.6, further preferably about 0.01 to about 0.45, and still more preferably about 0.01 to about 0.3. The zeta potential can be in the range of -50 mV to 5 mV, preferably in the range of -40 mV to 5 mV, more preferably in the range of -20 mV to 5 mV, and further preferably in the range of -12 mV to 5 mV.

The form of the lipid nanoparticle according to the present invention is not particularly limited, and examples thereof include a unilamellar liposome, a multilamellar liposome, a spherical micelle, and an amorphous layered structure, as a form dispersed in an aqueous solvent. The lipid nanoparticle according to the present invention is preferably a unilamellar liposome or a multilamellar liposome.

The lipid nanoparticle according to the present invention preferably encapsulates a component of interest to be delivered into a target cell inside the particle covered with a lipid membrane. The component encapsulated inside the particle by the lipid nanoparticle according to the present invention is not particularly limited as long as it has a size that can be encapsulated. Any substance such as a nucleic acid, a saccharide, a peptide, a low molecular weight compound, and a metal compound can be encapsulated in the lipid nanoparticle according to the present invention.

A nucleic acid is preferable as the component to be encapsulated in the lipid nanoparticle according to the present invention. The nucleic acid may be DNA, RNA, or an analog or derivative thereof (e.g., peptide nucleic acid (PNA) or phosphorothioate DNA). The nucleic acid to be encapsulated in the lipid nanoparticle according to the present invention may be a single-stranded nucleic acid or a double-stranded nucleic acid, and may be linear or circular.

The nucleic acid to be encapsulated in the lipid nanoparticle according to the present invention preferably contains a foreign gene to be expressed in a target cell, and more preferably is a nucleic acid that functions to express the foreign gene in a cell by being taken up into the cell. The foreign gene may be a gene originally contained in the genomic DNA of the target cell or a gene not contained in the genomic DNA. Examples of such nucleic acids include gene expression vectors containing a nucleic acid comprising a base sequence encoding a gene to be expressed. The gene expression vector may exist as an extrachromosomal gene in a transfected cell, or may be integrated into genomic DNA by homologous recombination.

The gene expression vector to be encapsulated in the lipid nanoparticle according to the present invention is not particularly limited, and a vector generally used in gene therapy or the like can be used. The gene expression vector to be encapsulated in the lipid nanoparticle according to the present invention is preferably a nucleic acid vector such as a plasmid vector. The plasmid vector may be in a circular form, or may be encapsulated in the lipid nanoparticle according to the present invention in a state of being linearly cleaved in advance. The gene expression vector can be designed by a conventional method with generally used molecular biological tools based on the base sequence information of the target gene to be expressed, and can be produced by various known methods.

The nucleic acid to be encapsulated in the lipid nanoparticle according to the present invention is also preferably a functional nucleic acid that controls the expression of a target gene present in a target cell. Examples of the functional nucleic acid include antisense oligonucleotides, antisense DNA, antisense RNA, siRNA, microRNA, mRNA, and gRNA. It may also be plasmid DNA (pDNA) serving as an expression vector such as an siRNA expression vector that expresses siRNA in a cell. The expression vector can be prepared from a commercial product and can be modified appropriately. For example, the siRNA expression vector can be prepared from a commercially available siRNA expression vector, or can be appropriately modified. The nucleic acid to be encapsulated in the lipid nanoparticle according to the present invention is preferably mRNA or pDNA because of its particularly good selectivity for tissues in vivo.

The method for producing the lipid nanoparticle according to the present invention is not particularly limited, and any method available to those skilled in the art can be employed. It can be produced as follows: for example, all lipid components are dissolved in an organic solvent such as chloroform, and a lipid membrane is formed by drying under reduced pressure using an evaporator or spray drying using a spray dryer, and then a component to be encapsulated in the lipid nanoparticle, for example, an aqueous solvent containing a nucleic acid or the like, is added to the dried mixture, and further emulsified by an emulsifier such as a homogenizer, an ultrasonic emulsifier, or a high-pressure jet emulsifier. It can also be produced by a method well known as a method for producing liposomes, such as a reverse phase evaporation method. When it is desired to control the size of the lipid nanoparticle, extrusion through a membrane filter having a uniform pore size or the like under high pressure may be performed.

The composition of the aqueous solvent (dispersion medium) is not particularly limited, but examples thereof include buffer solutions such as phosphate buffer, citrate buffer, and phosphate buffered saline, physiological saline, and cell culture media. These aqueous solvents (dispersion media) can stably disperse the lipid nanoparticles, but further, sugars (aqueous solutions) such as monosaccharides such as glucose, galactose, mannose, fructose, inositol, ribose, and xylose, disaccharides such as lactose, sucrose, cellobiose, trehalose, and maltose, trisaccharides such as raffinose and melezitose, polysaccharides such as cyclodextrin, and sugar alcohols such as erythritol, xylitol, sorbitol, mannitol, and maltitol, and polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol may be added. In order to stably store the lipid nanoparticles dispersed in this aqueous solvent for a long period of time, it is desirable to exclude electrolytes in the aqueous solvent as much as possible from the viewpoint of physical stability such as aggregation suppression. From the viewpoint of chemical stability of the lipid, it is desirable to set the pH of the aqueous solvent to near neutral from weakly acidic (about pH 3.0 to 8.0) and/or to remove dissolved oxygen by nitrogen bubbling or the like.

The lipid nanoparticle according to the present invention can also be produced by an alcohol dilution method using a flow path. The method is a method for producing lipid nanoparticles by introducing a solution in which lipid components are dissolved in an alcohol solvent and a solution in which a water-soluble component to be included in the lipid nanoparticle is dissolved in an aqueous solvent from separate flow paths and merging them. By using a microchannel with a built-in three-dimensional micromixer capable of achieving instantaneous mixing of two liquids, lipid nanoparticles having a diameter of about 30 nm can be reproducibly produced (Non-patent Literature 5). As the flow path used for production, it is preferable to use a simple two-dimensional flow path structure in which baffles of a certain width are arranged alternately from both sides in a micro-sized flow path through which a raw material solution flows, as described in Patent Literature 2, since it can form a nano-sized lipid particle formation system with high particle size controllability. As the aqueous solvent used in the alcohol dilution method, the solvents described above can be used.

When the resulting aqueous dispersion of the lipid nanoparticle is freeze-dried or spray-dried, for example, a use of sugars (aqueous solutions) such as monosaccharides such as glucose, galactose, mannose, fructose, inositol, ribose, and xylose, disaccharides such as lactose, sucrose, cellobiose, trehalose, and maltose, trisaccharides such as raffinose and melezitose, polysaccharides such as cyclodextrin, and sugar alcohols such as erythritol, xylitol, sorbitol, mannitol, and maltitol may improve stability. Further, when the aqueous dispersion is frozen, for example, a use of the above-mentioned sugars or polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol may improve stability.

The lipid nanoparticle according to the present invention functions as a gene expression carrier to a target tissue. By encapsulating a foreign gene of interest to be expressed in a target cell in the lipid nanoparticle according to the present invention and administering the lipid nanoparticle to a subject animal, the foreign gene is expressed in the target tissue of the subject animal. The lipid nanoparticle according to the present invention can also encapsulate a medicinal ingredient other than a nucleic acid to introduce the medicinal ingredient into a target tissue and is also useful as a drug delivery carrier. Therefore, the lipid nanoparticle according to the present invention is useful as an active ingredient of a pharmaceutical composition used for various treatments including gene therapy.

Highly liver-selective lipid nanoparticles can be produced by using a compound having high tissue specificity for the liver as a constituent lipid among the pH-sensitive cationic lipids according to the present invention and selecting and using other constituent lipids that are easily taken up into the liver. Similarly, highly spleen-selective lipid nanoparticles can be produced by using a compound having high tissue specificity for the spleen as a constituent lipid among the pH-sensitive cationic lipids according to the present invention and selecting and using other constituent lipids that are easily taken up into the spleen. When such highly liver-selective lipid nanoparticles or highly spleen-selective lipid nanoparticles encapsulating a gene expression vector are administered to an animal, the gene expression vector encapsulated in the lipid nanoparticles is selectively expressed in the liver or spleen rather than in other organs. Similarly, when highly liver-selective lipid nanoparticles or highly spleen-selective lipid nanoparticles among the lipid nanoparticles according to the present invention, encapsulating an siRNA expression vector, are administered to an animal, the siRNA expression vector encapsulated in the lipid nanoparticles is selectively expressed in the liver or spleen rather than in other organs, and the expression of the gene targeted by the expression vector is suppressed.

Highly liver-selective lipid nanoparticles include those in which phosphatidylcholine has a content ratio of 10 mol% or less, preferably 2 to 9 mol%, more preferably 2 to 5 mol%, or further preferably 2 to 4 mol% based on the total constituent lipids.

The animal to which the lipid nanoparticle according to the present invention is administered is not particularly limited, and may be a human or an animal other than a human. Examples of non-human animals include mammals such as cattle, pigs, horses, sheep, goats, monkeys, dogs, cats, rabbits, mice, rats, hamsters, and guinea pigs, and birds such as chickens, quails, and ducks. The administration route for administering the lipid nanoparticle according to the present invention to an animal is not particularly limited, but is preferably parenteral administration such as intravenous administration, enteral administration, intramuscular administration, subcutaneous administration, transdermal administration, nasal administration, and pulmonary administration.

In one embodiment, the lipid nanoparticle according to the present invention is a lipid nanoparticle for use as a medicament, comprising the pH-sensitive cationic lipid described herein. In another embodiment, the lipid nanoparticle according to the present invention is a lipid nanoparticle for use as a medicament, comprising the pH-sensitive cationic lipid described herein and a nucleic acid.

In one embodiment, the lipid nanoparticle according to the present invention is a lipid nanoparticle for use in gene therapy, comprising the pH-sensitive cationic lipid described herein. In another embodiment, the lipid nanoparticle according to the present invention is a lipid nanoparticle for use in gene therapy, comprising the pH-sensitive cationic lipid described herein and a nucleic acid.

In one embodiment, the lipid nanoparticle according to the present invention comprises the pH-sensitive cationic lipid described herein and encapsulates a foreign gene of interest to be expressed in a target cell, and a method for expressing the foreign gene in the target cell of a subject animal, comprising administering the lipid nanoparticle to the subject animal, is provided.

In one embodiment, the lipid nanoparticle according to the present invention comprises the pH-sensitive cationic lipid described herein and encapsulates a nucleic acid capable of suppressing a function of a target gene, and a method for suppressing the function of the target gene, comprising administering to a subject animal, is provided.

In one embodiment, the lipid nanoparticle according to the present invention comprising the pH-sensitive cationic lipid described herein and a nucleic acid can be used in a method for treating or preventing a disease associated with an abnormality in a gene. Specifically, a method for treating or preventing a disease associated with an abnormality in a gene is provided, the method comprising administering to a subject in need thereof the lipid nanoparticle comprising the pH-sensitive cationic lipid described herein and a nucleic acid, wherein the nucleic acid suppresses or substitutes for the function of the gene.

In one embodiment, the lipid nanoparticle according to the present invention can be used in the manufacture of a medicament for gene therapy. Specifically, a use of the lipid nanoparticle comprising the pH-sensitive cationic lipid described herein in the manufacture of a medicament for gene therapy is provided. Also provided is a use of the lipid nanoparticle comprising the pH-sensitive cationic lipid described herein and a nucleic acid in the manufacture of a medicament for gene therapy.

### Examples

Next, the present invention will be described in more detail with reference to Examples, but the present invention is not limited to the following Examples.

### <NMR>

In the following experiments, unless otherwise specified, ¹H and ¹³C NMR spectra were obtained using an NMR apparatus manufactured by JEOL Ltd. (ECA500 apparatus or ECS400 apparatus) with tetramethylsilane as an internal standard (0 ppm), wherein d is an abbreviation for doublet, t is an abbreviation for triplet, and m is an abbreviation for multiplet. Chemical shifts of the ¹H NMR spectra were reported in ppm on the σ scale, downfield from tetramethylsilane.

### <Thin Layer Chromatography (TLC)>

In the following experiments, unless otherwise specified, the reactants of all reactions were monitored by TLC using pre-coated TLC plates (manufactured by Millipore). TLC visualization was performed by UV light (254 nm), phosphomolybdic acid staining, or staining with p-anisaldehyde staining, iodine, or Dragendorff s reagent. Reaction products were purified by a Selekt system (manufactured by Biotage).

### <Constituent Lipids of Lipid Nanoparticles>

SM-102 manufactured by Cayman Chemical, ALC-0315 manufactured by Ambeed, DLin-MC3-DMA (MC3) manufactured by Selleck Biotech, and cholesterol (Chol) manufactured by Sigma-Aldrich were respectively used. 1,2-Distearoyl-sn-glycero-3-phosphocholine (DSPC) and methoxyethylene glycol 2000-modified 2-dimyristoyl-rac-glycerol (PEG-DMG) manufactured by NOF CORPORATION were used.

### <mRNA>

For the nucleic acids to be encapsulated in lipid nanoparticles, mRNA encoding firefly luciferase (Fluc) (CleanCap FLuc mRNA, 5moU), mRNA encoding enhanced green fluorescent protein EGFP (EGFP mRNA, 5moU), and mRNA encoding human erythropoietin hEPO (hEPO mRNA, 5moU) manufactured by TriLink Biotechnologies were used.

### <Preparation of Lipid Nanoparticles>

In the following experiments, unless otherwise specified, lipid nanoparticles were prepared by an alcohol dilution method using a flow path. The baffle mixer-integrated microchannel iLiNP (manufactured by Lilac Pharma) was used as the flow path.

Specifically, first, 13.75 µL of a 1 mg/mL mRNA solution was diluted with 50 mM citrate buffer (pH 4.0) to a volume of 250 µL, and the resulting mRNA solution was dispensed into a 1 mL syringe (manufactured by HAMILTON). Further, in the case of pH-responsive cationic lipid/chol/DSPC/PEG-DSG=50/35/15/1.5 (mol%), 200 µL of a 10 mM pH-responsive cationic lipid/ethanol solution, 140 µL of a 10 mM cholesterol/ethanol solution, 60 µL of a 10 mM DSPC/ethanol solution, 60 µL of a 1 mM PEG-DSG/ethanol solution, and 40 µL of ethanol were mixed to make a total volume of 500 µL, and the resulting lipid solution (final lipid concentration: 8 mM, 100% ethanol) was dispensed into a 1 mL syringe (manufactured by HAMILTON). Each syringe was mounted on a syringe pump, the baffle mixer-integrated microchannel and the pump were connected, the RNA solution and the lipid solution were fed into the microchannel at a flow rate ratio of 3:1 (N/P=9.3) and a total flow rate of 500 µL/min, and a lipid nanoparticle solution discharged from the flow path was collected. The lipid nanoparticle solution was placed in a dialysis membrane (MWCO 12,000-14,000, manufactured by Spectrum Laboratories), dialyzed against PBS(-) (pH 7.4) as an external aqueous phase at 4°C for 2 hours or more to remove ethanol and exchange the buffer, and then the lipid nanoparticle solution was collected from the dialysis membrane.

### <Measurement of Average Particle Size and Zeta Potential of Lipid Nanoparticles>

The average particle size (number average value), ζ-average particle size, and polydispersity index (PdI) of the lipid nanoparticles in PBS(-) (pH 7.4), and the zeta potential in 10 mM HEPES buffer (pH 7.4) were measured using an analyzer "Zetasizer Nano ZS ZEN3600" (manufactured by Malvern) utilizing dynamic light scattering.

### <Encapsulation Rate (Encapsulation Efficiency) of Lipid Nanoparticles>

The encapsulation rate of the lipid nanoparticles was determined by measuring the amount of encapsulated nucleic acid (mRNA) using Ribogreen (manufactured by Life Technologies), which is an RNA intercalator. The mRNA amount was quantified based on a calibration curve prepared from a series of diluted mRNA solutions at known concentrations.

Specifically, 10 mM HEPES buffer (pH 7.4), 10 w/v% Triton X-100, and Ribogreen were mixed at 978.8 µL, 20 µL, and 1.25 µL per 10 wells, respectively, to obtain a Triton(+) buffer. A Triton(-) buffer was obtained in replacing Triton X-100 with 10 mM HEPES buffer (pH 7.4). 100 µL/well of the sample was added to a black 96-well plate for fluorescence measurement, and 100 µL/well of Triton(+) buffer or Triton(-) buffer was added. After the plate was gently shaken on a shaking incubator (SI-300, manufactured by AS ONE) (700 rpm, 30 seconds), the fluorescence intensity of each well was measured using a microplate reader (VARIOSKAN LUX, manufactured by Thermo Fisher Scientific) (wavelength Ex/Em=500 nm/525 nm), and the nucleic acid encapsulation rate (%) was calculated.

### <Measurement of Apparent pKa of Lipid Nanoparticles>

The apparent pKa of the lipid nanoparticles was measured using p-toluenesulfonic acid (TNS). First, TNS (final concentration: 0.75 µM) and lipid nanoparticles (final concentration: 30 µM) were mixed in a buffer (200 mL) adjusted to each pH (pH 3.5 to 9.5). As the buffer, 20 mM citrate buffer, 20 mM sodium phosphate buffer, or 20 mM Tris-HCl buffer containing 130 mM NaCl was used. The fluorescence intensity of the prepared mixture was measured using a fluorescence spectrophotometer (Varioskan Lux, manufactured by Thermo Fisher Scientific) with the settings of λex=321 nm and λem=447 nm. Among the measured values, the highest value and the lowest value were set as 100% and 0% ionization degrees, respectively, and the pH showing a 50% ionization degree was calculated as the apparent pKa. The content ratio (%) of cationically charged pH-sensitive cationic lipid was calculated using the Henderson-Hasselbalch equation.

### <Administration of Lipid Nanoparticles to Mice>

Lipid nanoparticles were administered to mice by intravenous injection into the tail vein using a 27G needle. Unless otherwise specified, BALB/c mice (4 to 5 weeks old, female) were used.

### <Measurement of Fluc activity>

First, BALB/c mice (female, 4 weeks old) were intravenously injected with lipid nanoparticles encapsulating Fluc mRNA (Fluc mRNA-loaded lipid nanoparticles) at a dose of 0.1 mg mRNA/kg. Six hours after intravenous injection, the mice were euthanized, each tissue was collected, frozen in liquid nitrogen, and stored at -80°C. The tissues were homogenized in passive lysis buffer (manufactured by Promega) using a bead-type cell disruptor (Micro Smash MS-100R, manufactured by TOMY Seiko). Tissue debris was removed by centrifugation, and the supernatant was collected. Fluc activity in the supernatant was measured using the Luciferase Assay System (E1500, manufactured by Promega) according to the manufacturer's protocol. Luminescence was measured using a luminometer (Luminescencer-PSN, manufactured by ATTO). Protein concentration was determined using a commercially available assay kit (BCA Protein Assay Kit, manufactured by Pierce). Fluc activity was expressed as relative light units (RLU) per mg of protein.

### [Example 1]

2-(((4-(didecylamino)-4-oxobutanoyl)oxy)methyl)-2-(3-(piperidin-1-yl)propanamido)propane-1,3-diyl bis(4-(didecylamino)-4-oxobutanoate) (TxT-5-4A20) was synthesized.

### (1) Synthesis of 4-(didecylamino)-4-oxobutanoic acid (4A20)

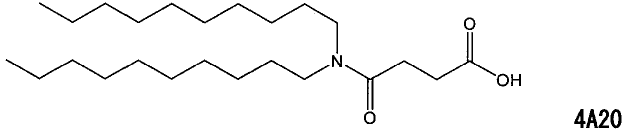

Succinic anhydride (500.4 mg, 5 mmol) dissolved in anhydrous DCM (10 ml) was added dropwise to solution of didecylamine (1.49 mg, 5 mmol) in pyridine (605.4 µL) and anhydrous DCM (10 mL). The reaction mixture was stirred at 40°C overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (4A20) (1.97 g, yield 99.0%) as an yellow oil. The crude product was used for next reaction without further purification.

### (2) Synthesis of N-(1,3-dihydroxy-2-(hydroxymethyl)propan-2-yl)-3-(piperidin-1-yl)propanamide (TxT-5 head).

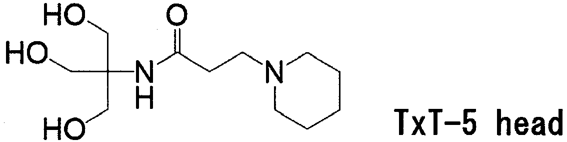

Piperidine (1.98 mL, 20.0 mmol) was added to N-[tris(hydroxymethyl)methyl]acrylamide (93%, 1.884 g, 10.0 mmol) dissolved in methanol (10 mL). The resulting reaction mixture was stirred at 40°C overnight. The residue was loaded on normal-phase column (Sfär Silica HCD, Biotage) and purified by flash chromatography with a gradient mobile phase of DCM and methanol. This gave TxT-5 head (2.01 g, yield 81%) as a white powder.

### (3) Synthesis of TxT-5-4A20.

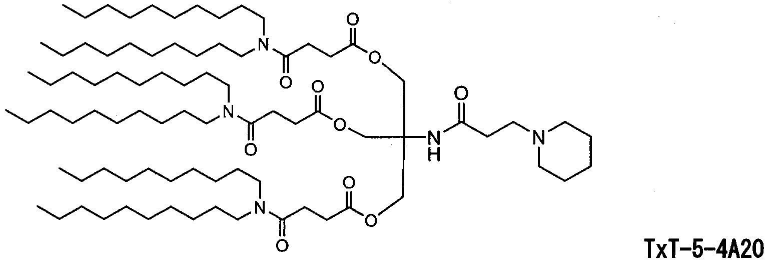

Compound (4A20) (656.1 mg, 1.65 mmol) was dissolved in anhydrous DCM (2.5 ml). To this solution, TxT-5 head (130.2 mg, 0.5 mmol), DMAP (6.11 mg, 0.05 mmol), and EDCI-HCl (335.5 mg, 1.75 mmol) were added. The resulting reaction mixture was stirred at 40°C overnight and then suspended with EtOAc. The suspension was washed with 0.5 N aqueous NaOH solution, water, and brine, and then dried over anhydrous Na₂SO₄. The obtained solution was filtered, and filtrate was evaporated. The residue was loaded on normal-phase column (Sfär Silica HCD, Biotage) and purified by flash chromatography with a gradient mobile phase of hexane with 0.5% TEA and EtOAc with 0.5% TEA. This gave Compound (TxT-5-4A20) (634.1 mg, yield 79.5%) as an yellow oil.

### [Example 2]

2-(((5-(dibutylamino)-5-oxopentanoyl)oxy)methyl)-2-(3-(piperidin-1-yl)propanamido)propane-1,3-diyl bis(5-(dibutylamino)-5-oxopentanoate) (TxT-5-5A8) was synthesized.

### (1) Synthesis of 5-(dibutylamino)-5-oxopentanoic acid (5A8)

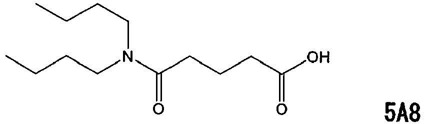

Glutaric anhydride (570.5 mg, 5 mmol) dissolved in anhydrous DCM (5 mL) was added dropwise to solution of dibutylamine (851 µL, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A8) (1.21 g, yield 99.8%) as an yellow oil. The crude product was used for next reaction without further purification.

### (2) Synthesis of TxT-5-5A8.

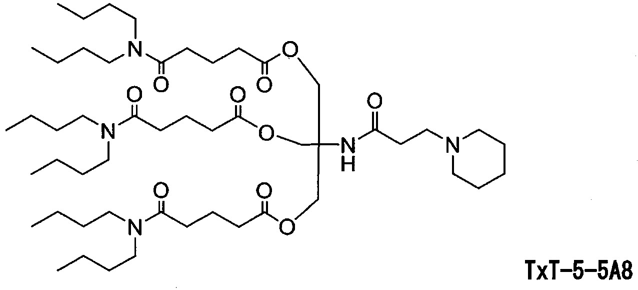

Compound (5A8) (335.5 mg, 1.65 mmol) was dissolved in anhydrous DCM (2.5 ml). To this solution, TxT-5 head (130.2 mg, 0.5 mmol), DMAP (6.11 mg, 0.05 mmol), and EDCI-HCl (335.5 mg, 1.75 mmol) were added. The resulting reaction mixture was stirred at 40°C overnight and then suspended with EtOAc. The suspension was washed with 0.5 N aqueous NaOH solution, water, and brine, and then dried over anhydrous Na₂SO₄. The obtained solution was filtered, and filtrate was evaporated. The residue was loaded on normal-phase column (Sfär Silica HCD, Biotage) and purified by flash chromatography with a gradient mobile phase of hexane with 0.5% TEA and EtOAc with 0.5% TEA. The residue was then loaded on reverse-phase column (Sfär C18, Biotage) and purified by flash chromatography with a gradient mobile phase of water with 0.1% TFA and acetonitrile/2-propanol (1:1) with 0.1% TFA. This gave Compound (TxT-5-5A8) (321.5 mg, yield 68.7%) as an yellow oil.

### [Example 3]

2-(((5-((2-ethylhexyl)amino)-5-oxopentanoyl)oxy)methyl)-2-(3-(piperidin-1-yl)propanamido)propane-1,3-diyl bis(5-((2-ethylhexyl)amino)-5-oxopentanoate) (TxT-5-5A8β) was synthesized.

### (1) Synthesis of 5-((2-ethylhexyl)amino)-5-oxopentanoic acid (5A8β)

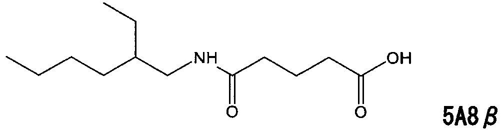

Glutaric anhydride (570.5 mg, 5 mmol) dissolved in anhydrous DCM (5 mL) was added dropwise to solution of 2-ethylhexylamine (818.7 µL, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A8β) (1.22 g yield 100.7%) as an yellow oil. The crude product was used for next reaction without further purification.

### (2) Synthesis of TxT-5-5A8β

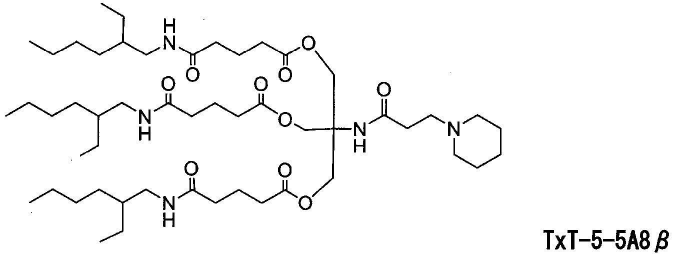

Compound (TxT-5-5A8β) (157.9 mg, yield 33.7%) was obtained as an yellow oil in the same manner as the synthesis of Compound (TxT-5-4A20), except that Compound (5A8β) (335.5 mg, 1.65 mmol) was used instead of Compound (4A20) and reacted with TxT-5 head followed by purification.

### [Example 4]

2-(((5-(dipentylamino)-5-oxopentanoyl)oxy)methyl)-2-(3-(piperidin-1-yl)propanamido)propane-1,3-diyl bis(5-(dipentylamino)-5-oxopentanoate) (TxT-5-5A10) was synthesized.

### (1) Synthesis of 5-(dipentylamino)-5-oxopentanoic acid (5A10)

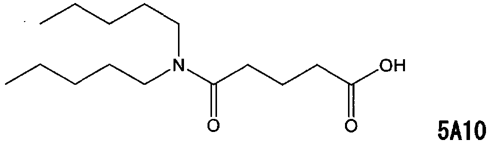

Glutaric anhydride (570.5 mg, 5 mmol) dissolved in anhydrous DCM (5 mL) was added dropwise to solution of diamylamine (1.01 mL, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A10) (1.38 g, yield 102%) as an yellow oil. The crude product was used for next reaction without further purification.

### (2) Synthesis of TxT-5-5A10

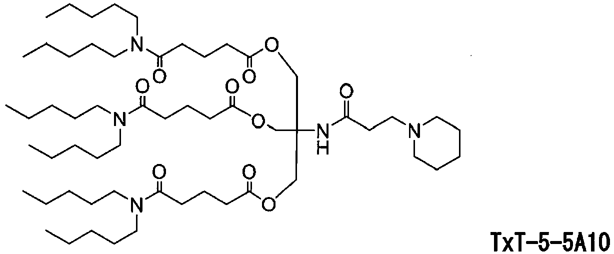

Compound (TxT-5-5A10) (194.5 mg, yield 38.1%) was obtained as an yellow oil in the same manner as the synthesis of Compound (TxT-5-5A8), except that Compound (5A10) (447.8 mg, 1.65 mmol) was used instead of Compound (5A8) and reacted with TxT-5 head followed by purification.

### [Example 5]

2-(((5-(dihexylamino)-5-oxopentanoyl)oxy)methyl)-2-(3-(piperidin-1-yl)propanamido)propane-1,3-diyl bis(5-(dihexylamino)-5-oxopentanoate) (TxT-5-5A12) was synthesized.

### (1) Synthesis of 5-(dihexylamino)-5-oxopentanoic acid (5A12)

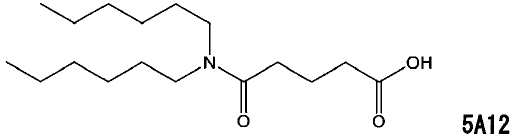

Glutaric anhydride (570.5 mg, 5 mmol) dissolved in anhydrous DCM (5 mL) was added dropwise to solution of dihexylamine (1.17 mL, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A12) (1.55 g yield 103.5%) as an yellow oil. The crude product was used for next reaction without further purification.

### (2) Synthesis of TxT-5-5A12

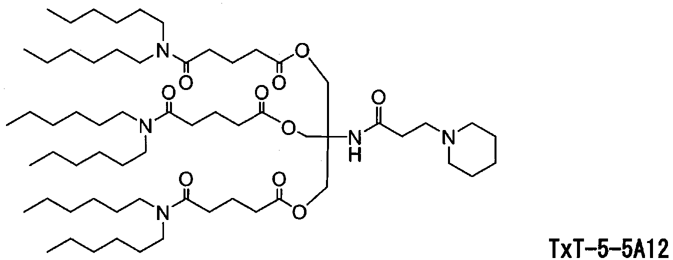

Compound (TxT-5-5A12) (343.4 mg, yield 62.2%) was obtained as an yellow oil in the same manner as the synthesis of Compound (TxT-5-4A20), except that Compound (5A12) (494.1 mg, 1.65 mmol) was used instead of Compound (4A20) and reacted with TxT-5 head followed by purification.

### [Example 6]

2-(((5-(diheptylamino)-5-oxopentanoyl)oxy)methyl)-2-(3-(piperidin-1-yl)propanamido)propane-1,3-diyl bis(5-(diheptylamino)-5-oxopentanoate) (TxT-5-5A14) was synthesized.

### (1) Synthesis of 5-(diheptylamino)-5-oxopentanoic acid (5A14)

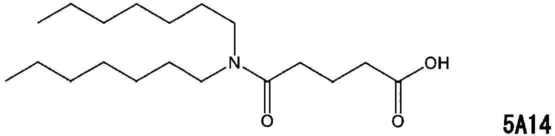

Glutaric anhydride (570.5 mg, 5 mmol) dissolved in anhydrous DCM (5 mL) was added dropwise to solution of diheptylamine (1.35 mL, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A14) (1.70 g, yield 104%) as an yellow oil. The crude product was used for next reaction without further purification.

### (2) Synthesis of Compound (TxT-5-5A14)

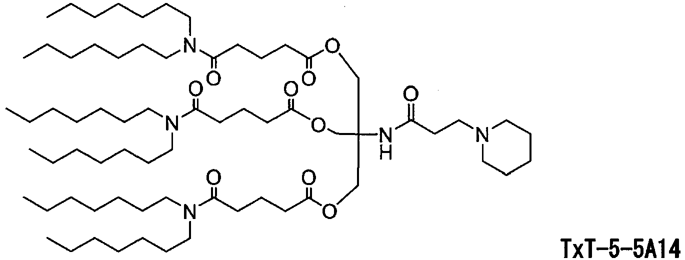

Compound (TxT-5-5A14) (393.0 mg, yield 66.1%) was obtained as an yellow oil in the same manner as the synthesis of Compound (TxT-5-4A20), except that Compound (5A14) (517.2 mg, 1.65 mmol) was used instead of Compound (4A20) and reacted with TxT-5 head followed by purification.

### [Example 7]

2-(((5-(dioctylamino)-5-oxopentanoyl)oxy)methyl)-2-(3-(piperidin-1-yl)propanamido)propane-1,3-diyl bis(5-(dioctylamino)-5-oxopentanoate) (TxT-5-5A16) was synthesized.

### (1) Synthesis of 5-(dioctylamino)-5-oxopentanoic acid (5A16)

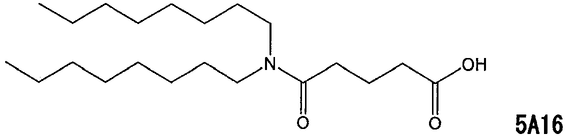

Glutaric anhydride (570.5 mg, 5 mmol) dissolved in anhydrous DCM (5 mL) was added dropwise to solution of di-n-octylamine (1.51 mL, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A16) (2.05 g, yield 115.2%) as an yellow oil. The crude product was used for next reaction without further purification.

### (2) Synthesis of Compound (TxT-5-5A16)

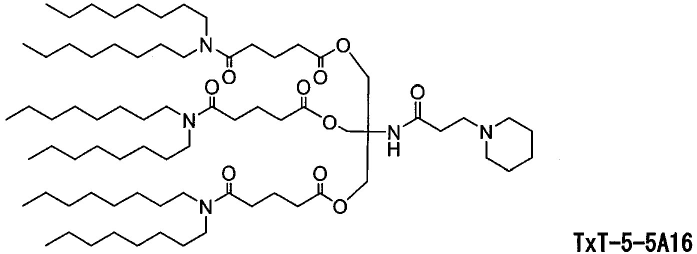

Compound (TxT-5-5A16) (527.5 mg, yield 75.4%) was obtained as an yellow oil in the same manner as the synthesis of Compound (TxT-5-5A8), except that Compound (5A16) (586.7 mg, 1.65 mmol) was used instead of Compound (5A8) and reacted with TxT-5 head followed by purification.

### [Example 8]

2-(6-(bis(2-ethylhexyl)amino)-2,6-dioxohexyl)-2-(3-(piperidin-1-yl)propanamido)propane-1,3-diyl bis(5-(bis(2-ethylhexyl)amino)-5-oxopentanoate) (TxT-5-5A16b) was synthesized.

### (1) Synthesis of 5-(bis(2-ethylhexyl)amino)-5-oxopentanoic acid (5A16b)

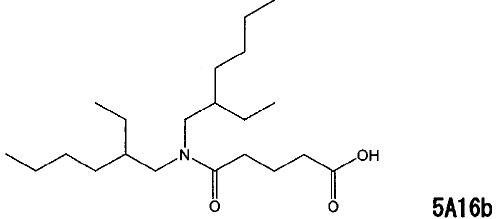

Glutaric anhydride (600.6 mg, 5.25 mmol) dissolved in anhydrous DCM (5 mL) was added dropwise to solution of di(2-ethylhexyl)amine (1.51 mL, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A16b) (1.76 g, yield 99.1%) as an yellow oil. The crude product was used for next reaction without further purification.

### (2) Synthesis of Compound (TxT-5-5A16b)

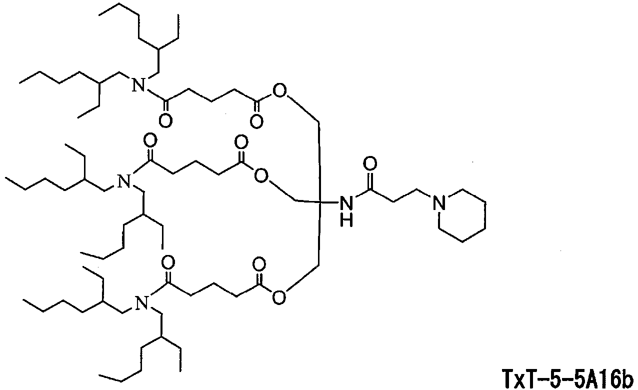

Compound (TxT-5-5A16b) (455.0 mg, yield 72.4%) was obtained as an yellow oil in the same manner as the synthesis of Compound (TxT-5-4A20), except that Compound (5A16b) (586.7 mg, 1.65 mmol) was used instead of Compound (4A20) and reacted with TxT-5 head followed by purification.

### [Example 9]

2-(((5-((2-hexyldecyl)amino)-5-oxopentanoyl)oxy)methyl)-2-(3-(piperidin-1-yl)propanamido)propane-1,3-diyl bis(5-((2-hexyldecyl)amino)-5-oxopentanoate) (TxT-5-5A16β) was synthesized.

### (1) Synthesis of 5-((2-hexyldecyl)amino)-5-oxopentanoic acid (5A16β)

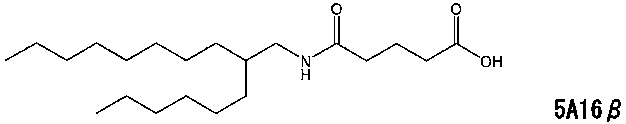

Glutaric anhydride (239.6 mg, 2.1 mmol) dissolved in anhydrous DCM (3 mL) was added dropwise to solution of 2-hexyldecane-1-amine (588.9 µL, 2 mmol) in pyridine (242.1 µL) and anhydrous DCM (9 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A16β) (728.5 mg, yield 41.5%) as an yellow oil. The crude product was used for next reaction without further purification.

### (2) Synthesis of Compound (TxT-5-5A16β)

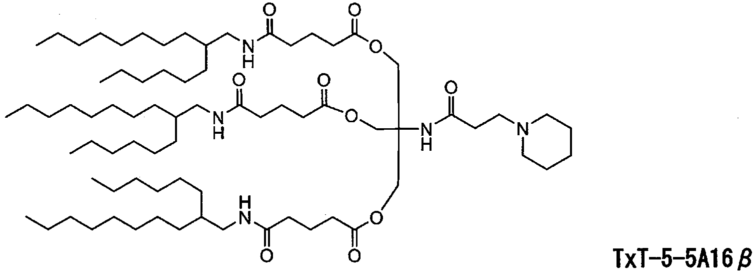

Compound (5A16β) (352.0 mg, 0.99 mmol) was dissolved in anhydrous DCM (2.5 mL). To this solution, TxT-5 head (78.1 mg, 0.3 mmol), DMAP (3.67 mg, 0.03 mmol), and EDCI-HCl (201.3 mg, 1.05 mmol) were added. The resulting reaction mixture was stirred at 40°C overnight and then suspended with EtOAc. The suspension was washed with 0.5 N aqueous NaOH solution, water, and brine, and then dried over anhydrous Na₂SO₄. The obtained solution was filtered, and filtrate was evaporated. The residue was loaded on normal-phase column (Sfär Silica HCD, Biotage) and purified by flash chromatography with a gradient mobile phase of hexane with 0.5% TEA and EtOAc with 0.5% TEA. This gave Compound (TxT-5-5A16β) (281.2 mg, yield 73.6%) as an yellow oil.

### [Example 10]

2-(6-(didecylamino)-2,6-dioxohexyl)-2-(3-(piperidin-1-yl)propanamido)propane-1,3-diyl bis(5-(didecylamino)-5-oxopentanoate) (TxT-5-5A20)was synthesized.

### (1) Synthesis of 5-(didecylamino)-5-oxopentanoic acid (5A20)

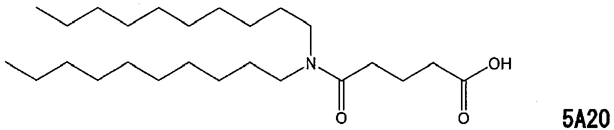

Glutaric anhydride (570.5 mg, 5 mmol) dissolved in anhydrous DCM (5 mL) was added dropwise to solution of didecylamine (1.49 mg, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na2_{S}O₄. The obtained solution was filtered, and filtrate was evaporated. This gave Compound (5A20) (1.98 g, yield 96.1%) as an yellow oil. The crude product was used for next reaction without further purification.

### (2) Synthesis of Compound (TxT-5-5A20)

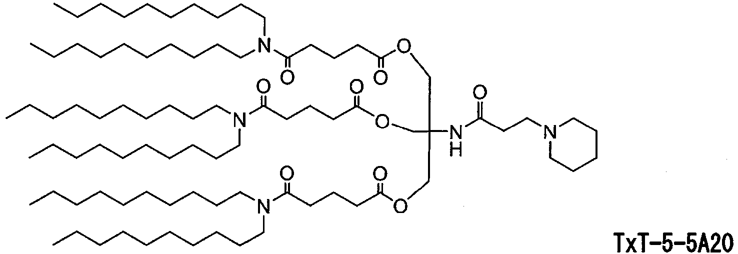

Compound (TxT-5-5A20) (448.6 mg, yield 62.9%) was obtained as an yellow oil in the same manner as the synthesis of Compound (TxT-5-4A20), except that Compound (5A20) (679.3 mg, 1.65 mmol) was used instead of Compound (4A20) and reacted with TxT-5 head followed by purification.

### [Example 11]

2-(6-(didodecylamino)-2,6-dioxohexyl)-2-(3-(piperidin-1-yl)propanamido)propane-1,3-diyl bis(5-(didodecylamino)-5-oxopentanoate) (TxT-5-5A24) was synthesized.

### (1) Synthesis of 5-(didodecylamino)-5-oxopentanoic acid (5A24).

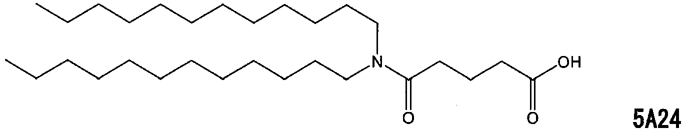

Glutaric anhydride (600.6 mg, 5.25 mmol) dissolved in anhydrous DCM (5 mL) was added dropwise to solution of didodecylamine (1.77 g, 5 mmol) in pyridine (605.4 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A24) (2.26 g, yield 96.5%) as a colorless oil. The crude product was used for next reaction without further purification.

### (2) Synthesis of Compound (TxT-5-5A24)

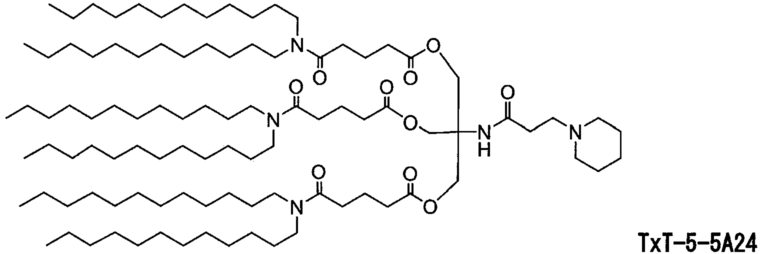

Compound (TxT-5-5A24) (634.1 mg, yield 79.5%) was obtained as an yellow oil in the same manner as the synthesis of Compound (TxT-5-4A20), except that Compound (5A24) (771.8 mg, 1.65 mmol) was used instead of Compound (4A20) and reacted with TxT-5 head followed by purification.

### [Example 12]

bis(2-hexyloctyl) O,O'-(2-(((5-((2-hexyloctyl)oxy)-5-oxopentanoyl)oxy)methyl)-2-(3-(piperidin-1-yl)propanamido)propane-1,3-diyl) diglutarate (TxT-5-5E14) was synthesized.

### (1) Synthesis of 5-((2-hexyloctyl)oxy)-5-oxopentanoic acid (5E14)

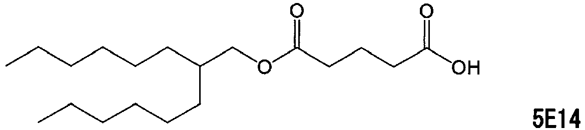

Glutaric anhydride (600.6 mg, 5.25 mmol) dissolved in anhydrous DCM (5 mL) was added dropwise to solution of 2-hexyl-1-n-octanol (1.29 mL, 5 mmol) in TEA (1.29 mL) and anhydrous DCM (15 mL). The reaction mixture was stirred at 40°C overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5E14) (1.71 g, yield 104.3%) as an yellow oil. The crude product was used for next reaction without further purification.

### (2) Synthesis of Compound (TxT-5-5E14)

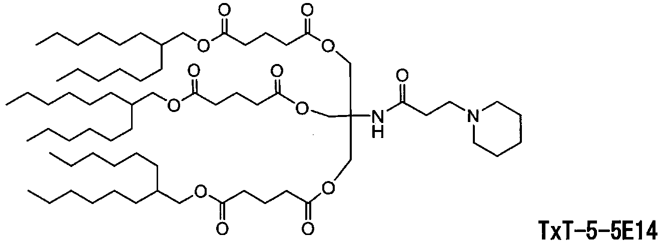

Compound (5E14) (599.5 mg, 1.83 mmol) was dissolved in anhydrous DCM (3.0 mL). To this solution, TxT-5 head (130.2 mg, 0.5 mmol), DMAP (6.72 mg, 0.055 mmol) and EDCI-HCl (359.0 mg, 1.93 mmol) were added. The resulting reaction mixture was stirred at 40°C overnight and then suspended with EtOAc. The suspension was washed with 0.5 N aqueous NaOH solution, water, and brine, and then dried over anhydrous Na₂SO₄. The obtained solution was filtered, and filtrate was evaporated. The residue was loaded on normal-phase column (Sfär Silica HCD, Biotage) and purified by flash chromatography with a gradient mobile phase of hexane with 0.5% TEA and EtOAc with 0.5% TEA. This gave Compound (TxT-5-5E14) (411.5 mg, yield 69.1%) as an yellow oil.

### [Example 13]

bis(2-butyloctyl) O,O'-(2-(((5-((2-hexyloctyl)oxy)-5-oxopentanoyl)oxy)methyl)-2-(3-(piperidin-1-yl)propanamido)propane-1,3-diyl) diglutarate (TxT-5-5E12) was synthesized.

### (1) Synthesis of 5-((2-butyloctyl)oxy)-5-oxopentanoic acid (5E12)

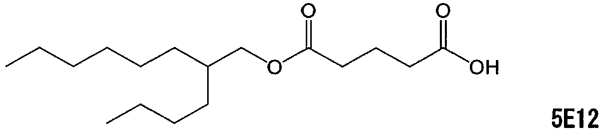

Glutaric anhydride (600.6 mg, 5.25 mmol) dissolved in anhydrous DCM (5 mL) was added dropwise to solution of 2-butyl-1-n-octanol (1.15 mL, 5 mmol) in TEA (1.04 mL) and anhydrous DCM (15 mL). The reaction mixture was stirred at 40°C overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5E12) (1.65 g, yield 109.7%) as an yellow oil. The crude product was used for next reaction without further purification.

### (2) Synthesis of Compound (TxT-5-5E12)

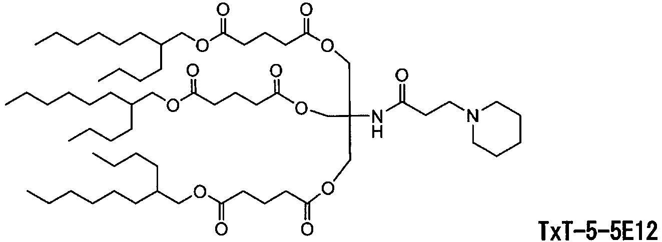

Compound (TxT-5-5E12) is obtained as an yellow oil in the same manner as the synthesis of Compound (TxT-5-5E14), except that Compound (5E12) is used instead of Compound (5E14) and reacted with TxT-5 head followed by purification.

### [Example 14]

2-(((5-((2-butyloctyl)amino)-5-oxopentanoyl)oxy)methyl)-2-(3-(piperidin-1-yl)propanamido)propane-1,3-diyl bis(5-((2-butyloctyl)amino)-5-oxopentanoate) (TxT-5-5A12β) was synthesized.

### (1) Synthesis of 5-((2-butyloctyl)amino)-5-oxopentanoic acid (5A12β)

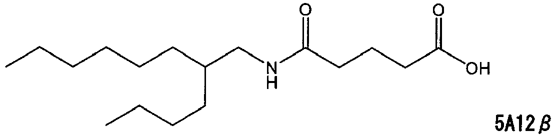

Glutaric anhydride (300.3 mg, 2.63 mmol) dissolved in anhydrous DCM (7.5 mL) was added dropwise to solution of 2-butyl-n-octan-1-amine (579 µL, 2.5 mmol) in TEA (522 µL, 3.75 mmol) and anhydrous DCM (2.5 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A12β) (764.4 mg, yield 102%) as an yellow oil. The crude product was used for next reaction without further purification.

### (2) Synthesis of Compound (TxT-5-5A12β)

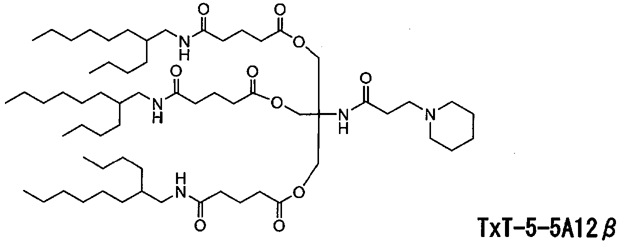

Compound (TxT-5-5A12β) (223.6 mg, yield 62.7%) was obtained as an yellow oil in the same manner as the synthesis of Compound (TxT-5-5A16β), except that Compound (5A12β) (324.2 mg, 0.99 mmol) was used instead of Compound (5A16β) and reacted with TxT-5 head followed by purification, and hexane and EtOAc with 0.5% TEA was used instead of hexane with 0.5% TEA and EtOAc with 0.5% TEA as a gradient mobile phase for flash chromatography.

### [Example 15]

2-(((5-((2-hexyloctyl)amino)-5-oxopentanoyl)oxy)methyl)-2-(3-(piperidin-1-yl)propanamido)propane-1,3-diyl bis(5-((2-hexyloctyl)amino)-5-oxopentanoate) (TxT-5-5A14β) was synthesized.

### (1) Synthesis of 2-hexyloctyl methanesulfonate

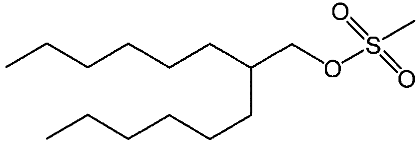

2-hexyl-1-octanol (2.58 mL, 10mmol), suspended in anhydrous DCM (20 mL) was added methanesulfonyl chloride (854.2 µL, 11mmol) and TEA (1.67 mL). The reaction mixture was stirred at room temperature for 16h and then evaporated. The resulting residue was suspended with EtOAc, washed with water of neutral, and brine, and dried over anhydrous Na₂SO₄. The obtained solution was filtered, and filtrate was evaporated. This gave 2-hexyloctyl methanesulfonate (2.58 g, yield 90%) as an yellow oil. The crude product was used for next reaction without further purification.

### (2) Synthesis of 2-hexyloctan-1-amine

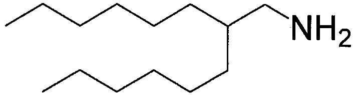

2-hexyloctyl methanesulfonate (1.32 g, 4.5 mmol) was solubilized in 7N NH₃ solution in MeOH (64.3 mL, 450 mol) and stirred at 40°C for 2 weeks. The reaction mixture was carried out a bubbling treatment with argon gas and then evaporated. The resulting residue was suspended with DCM, washed with 1 N aqueous HCl solution, water, and brine, and dried over anhydrous Na₂SO₄. The obtained solution was filtered, and filtrate was evaporated. The residue was loaded on normal-phase column (Sfär Silica HCD, Biotage) and purified by flash chromatography with a gradient mobile phase of DCM and MeOH. This gave 2-hexyloctan-1-amine (426.7 mg, yield 44.4%) as an yellow oil.

### (3) Synthesis of 5-((2-hexyloctyl)amino)-5-oxopentanoic acid (5A14β)

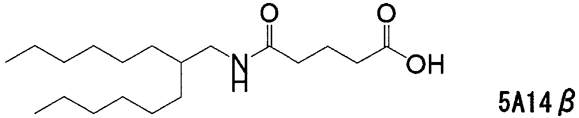

Glutaric anhydride (239.6 mg, 2.1 mmol) dissolved in anhydrous DCM (3 mL) was added dropwise to solution of 2-hexyloctan-1-amine (426.8 mg, 2 mmol) in pyridine (242.1 µL) and anhydrous DCM (7 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A14β) (617.8 mg, yield 94.3%) as an yellow oil. The crude product was used for next reaction without further purification.

### (4) Synthesis of Compound (TxT-5-5A14β)

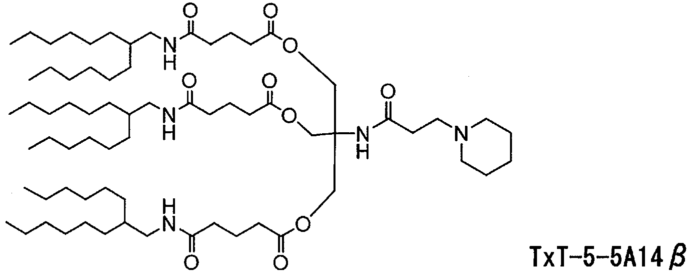

Compound (TxT-5-5A14β) (211.7 mg, yield 35.6%) was obtained as an yellow oil in the same manner as the synthesis of Compound (TxT-5-4A20), except that Compound (5A14β) (540.4 mg, 1.65 mmol) was used instead of Compound (4A20) and reacted with TxT-5 head followed by purification.

### [Example 16]

2-(((5-((2-decyltetradecyl)amino)-5-oxopentanoyl)oxy)methyl)-2-(3-(piperidin-1-yl)propanamido)propane-1,3-diyl bis(5-((2-decyltetradecyl)amino)-5-oxopentanoate) (TxT-5-5A24β) was synthesized.

### (1) Synthesis of 5-((2-decyltetradecyl)amino)-5-oxopentanoic acid (5A24β)

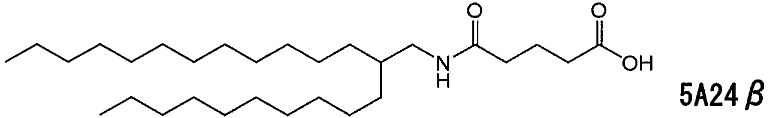

Glutaric anhydride (299.5 mg, 2.63 mmol) dissolved in anhydrous DCM (5 mL) was added dropwise to solution of 2-decyltetradecan-1-amine (884 mg, 2.5 mmol) in pyridine (302.7 µL) and anhydrous DCM (15 mL). The reaction mixture was stirred at ambient temperature overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A24β) (1.05 g, yield 89.9%) as an yellow oil. The crude product was used for next reaction without further purification.

### (2) Synthesis of Compound (TxT-5-5A24β)

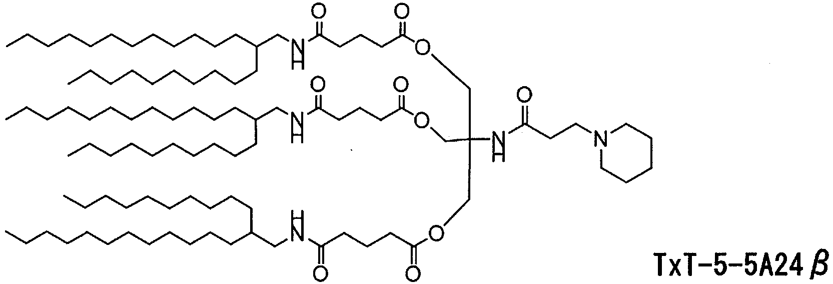

Compound (TxT-5-5A24β) (548.2 mg, yield 68.1%) was obtained as an yellow oil in the same manner as the synthesis of Compound (TxT-5-4A20), except that Compound (5A24β) (884 mg, 1.65 mmol) was used instead of Compound (4A20) and reacted with TxT-5 head followed by purification.

### [Example 17]

2-((2-(2-((diheptylamino)methyl)cyclohexyl)acetoxy)methyl)-2-(3-(piperidin-1-yl)propanamido)propane-1,3-diyl bis(2-(diheptylcarbamoyl)cyclohexane-1-carboxylate) (TxT-5-4(6)A14) was synthesized.

### (1) Synthesis of 2-(diheptylcarbamoyl)cyclohexane-1-carboxylic acid (4(6)A14)

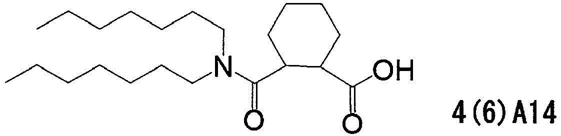

(±)-trans-1,2-cyclohexanedicarboxylic anhydride (809.4 mg, 5.25 mmol) dissolved in anhydrous DCM (5 mL) was added dropwise to solution of diheptylamine (1.35 mL, 5 mmol) in pyridine (605.4 µL) and anhydrous DCM (15 mL). The reaction mixture was stirred at 40°C overnight and then evaporated. The resulting residue was suspended with EtOAc, washed with 1 N aqueous HCl solution, water, and brine, and dried over anhydrous Na₂SO₄. The obtained solution was filtered, and filtrate was evaporated. This gave Compound (4(6)A14) (1.93 g, yield 104.9%) as a colorless oil. The crude product was used for next reaction without further purification.

### (2) Synthesis of Compound (TxT-5-4(6)A14)

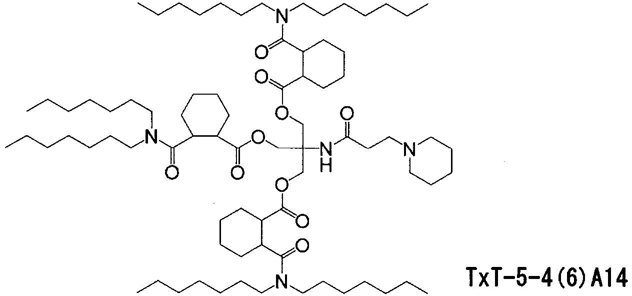

Compound (4(6)A14) (599.5 mg, 1.83 mmol) was dissolved in anhydrous DCM (2.5 mL). To this solution, TxT-5 head (130.2 mg, 0.5 mmol), DMAP (6.11 mg, 0.05 mmol), and EDCI-HCl (335.5 mg, 1.75 mmol) were added. The resulting reaction mixture was stirred at 40°C overnight and then suspended with EtOAc. The suspension was washed with 0.5 N aqueous NaOH solution, water, and brine, and dried over anhydrous Na₂SO₄. The obtained solution was filtered, and filtrate was evaporated. The residue was loaded on normal-phase column (Sfär Silica HCD, Biotage) and purified by flash chromatography with a gradient mobile phase of hexane and EtOAc. This gave Compound (TxT-5-4(6)A14) (293.5 mg, yield 44.8%) as a colorless oil.

### [Example 18]

2-(((5-(decylamino)-5-oxopentanoyl)oxy)methyl)-2-(3-(piperidin-1-yl)propanamido)propane-1,3-diyl bis(5-(decylamino)-5-oxopentanoate) (TxT-5-5A10L) was synthesized.

### (1) Synthesis of 5-(decylamino)-5-oxopentanoic acid (5A10L)

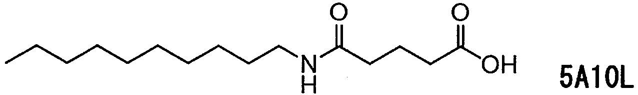

Glutaric anhydride (600.6 mg, 5.25 mmol) dissolved in anhydrous DCM (5 mL) was added dropwise to solution of 1-aminodecane (983.1 mL, 5 mmol) in pyridine (605.4 µL) and anhydrous DCM (15 mL). The reaction mixture was stirred at 40°C overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A10L) (1.17 g, yield 86.2%) as a white powder. The crude product was used for next reaction without further purification.

### (2) Synthesis of Compound (TxT-5-5A10L)

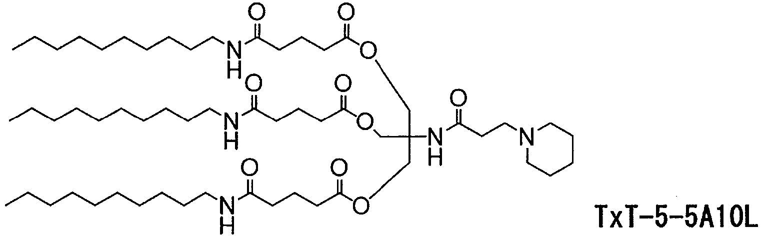

Compound (TxT-5-5A10L) (410.6 mg, yield 80.5%) was obtained as a white solid in the same manner as the synthesis of Compound (TxT-5-4(6)A14), except that Compound (5A10L) (447.8 mg, 1.65 mmol) was used instead of Compound (4(6)A14) and reacted with TxT-5 head followed by purification.

### [Example 19]

2-(((5-(dodecylamino)-5-oxopentanoyl)oxy)methyl)-2-(3-(piperidin-1-yl)propanamido)propane-1,3-diyl bis(5-(dodecylamino)-5-oxopentanoate) (TxT-5-5A12L) was synthesized.

### (1) Synthesis of 5-(dodecylamino)-5-oxopentanoic acid (5A12L)

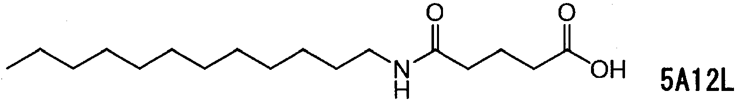

Glutaric anhydride (600.6 mg, 5.25 mmol) dissolved in anhydrous DCM (5 mL) was added dropwise to solution of dodecylamine (926.8 mL, 5 mmol) in pyridine (605.4 µL) and anhydrous DCM (15 mL). The reaction mixture was stirred at 40°C overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A12L) (968.1 mg, yield 64.7%) as a white powder. The crude product was used for next reaction without further purification.

### (2) Synthesis of Compound (TxT-5-5A12L)

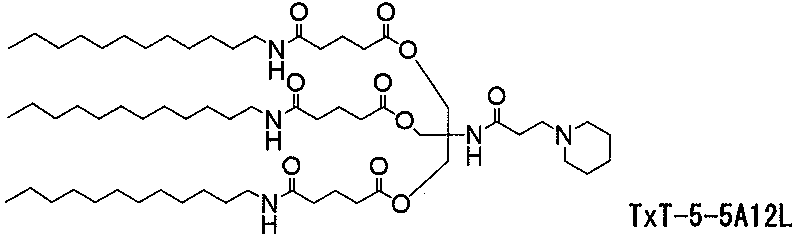

Compound (TxT-5-5A12L) (220.1 mg, yield 39.8%) was obtained as a white solid in the same manner as the synthesis of Compound (TxT-5-4(6)A14), except that Compound (5A12L) (494.1 mg, 1.65 mmol) was used instead of Compound (4(6)A14) and reacted with TxT-5 head followed by purification.

### [Example 20]

2-(((5-oxo-5-(tetradecylamino)pentanoyl)oxy)methyl)-2-(3-(piperidin-1-yl)propanamido)propane-1,3-diyl bis(5-oxo-5-(tetradecylamino)pentanoate) (TxT-5-5A14L) was synthesized.

### (1) Synthesis of 5-oxo-5-(tetradecylamino)pentanoic acid (5A14L)

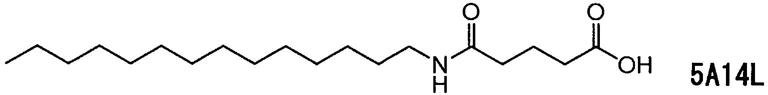

Glutaric anhydride (600.6 mg, 5.25 mmol) dissolved in anhydrous DCM (5 mL) was added dropwise to solution of tetradecylamine (1.07 g, 5 mmol) in pyridine (605.4 µL) and anhydrous DCM (15 mL). The reaction mixture was stirred at 40°C overnight and then evaporated. The resulting residue was suspended with EtOAc, washed with 1 N aqueous HCl solution, water, and brine, and then dried over anhydrous Na₂SO₄. The obtained solution was filtered, and filtrate was evaporated. This gave Compound (5A14L) (337.2 mg, yield 20.6%) as a white powder. The crude product was used for next reaction without further purification.

### (2) Synthesis of Compound (TxT-5-5A14L)

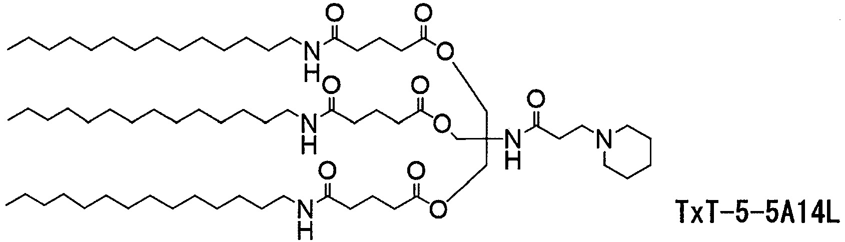

Compound (5A14L) (270.2 mg, 0.825 mmol) was dissolved in anhydrous DCM (2.5 mL). To this solution, TxT-5 head (65.1 mg, 0.25 mmol), DMAP (3.05 mg, 0.025 mmol), and EDCI-HCl (167.74 mg, 0.875 mmol) were added. The resulting reaction mixture was stirred at 40°C overnight and then suspended with EtOAc. The suspension was washed with 0.5 N aqueous NaOH solution, water, and brine, and then dried over anhydrous Na₂SO₄. The obtained solution was filtered, and filtrate was evaporated. The residue was loaded on normal-phase column (Sfär Silica HCD, Biotage) and purified by flash chromatography with a gradient mobile phase of hexane and EtOAc. This gave Compound (TxT-5-5A14L) (97.2 mg, yield 32.6%) as a white powder.

### [Example 21]

2-(((5-(diheptylamino)-5-oxopentanoyl)oxy)methyl)-2-(1-isopropylpiperidine-4-carboxamido)propane-1,3-diyl bis(5-(diheptylamino)-5-oxopentanoate) (TxT-19-5A14) was synthesized.

### (1) Synthesis of N-(1,3-dihydroxy-2-(hydroxymethyl)propan-2-yl)-1-isopropylpiperidine-4-carboxamide (TxT-19 head)

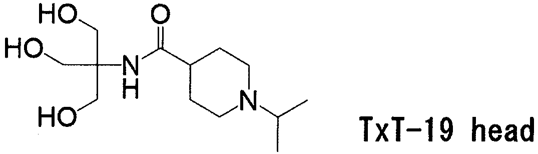

Under an argon atmosphere, ethyl chloroformate (434.0 mg, 4.0 mmol) was added dropwise to 1-(propan-2-yl)piperidine-4-carboxylic acid hydrochloride (747.7 mg, 3.6 mmol) and TEA (809.5 mg, 8.0 mmol) in THF (10 mL) at 0°C and the mixture was stirred for 30 min at 0°C. The resulting reaction mixture was added to tris(hydroxymethyl)aminomethane (436.1 mg, 3.6 mmol) and DMF (10 mL) and then stirred for overnight at 60°C. The reaction was monitored with TLC, and the reaction mixture was added TEA (2.23 mL, 15.8 mmol). The solid was filtered off and DMF and THF were evaporated in vacuo. The obtained residue was loaded on normal-phase column (Sfär Amino D, Biotage) and purified by flash chromatography with a gradient mobile phase of DCM and MeOH. This gave TxT-19 head (508.2 mg, yield 51.4%) as a yellow powder.

### (2) Synthesis of TxT-19-5A14

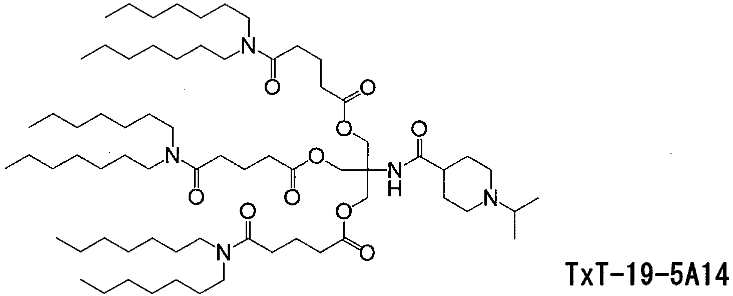

Compound (5A14) (310.3 mg, 0.95 mmol) was dissolved in anhydrous DCM (1.5 mL). To this solution, TxT-19 head (82.3 mg, 0.3 mmol), DMAP (3.70 mg, 0.03 mmol), and EDCI-HCl (201.3 mg, 1.05 mmol) were added. The resulting reaction mixture was stirred at 40°C under argon atmosphere overnight. After concentrating in vacuo, the obtained mixture was diluted with MeOH with 20% water and extracted with hexane. The aqueous layer was made alkaline using Na₂CO₃. The organic layer was washed with MeOH with 20% water and dried over anhydrous Na₂SO₄. The obtained solution was filtered, and filtrate was evaporated. The residue was loaded on normal-phase column (Sfär Amino D, Biotage) and purified by flash chromatography with a gradient mobile phase of hexane and EtOAc. This gave Compound (TxT-19-5A14) (106.3 mg, yield 29.3%) as a viscous yellow oil.

### [Example 22]

Lipid nanoparticles were prepared using TxT lipids, any of TxT-5-5A8, TxT-5-5A8β, TxT-5-5A10, TxT-5-5A12, TxT-5-5A12β, TxT-5-5A14, and TxT-5-5A16, as constituent lipids.

Phosphatidylcholine (PC), cholesterol, and PEG-DMG were used as lipids other than TxT lipids. DSPC was used as PC.

Specifically, TxT, DSPC, cholesterol, and PEG-DMG were used in a composition of TxT/DSPC/chol/PEG-DMG=50/15/35/1.5 (mol%) to prepare lipid nanoparticles loaded with Fluc mRNA (Fluc mRNA-loaded lipid nanoparticles) by the alcohol dilution method. Table 1 shows the ζ-average particle size (nm), number average particle size (nm), polydispersity index (PdI), ζ-potential (mV), mRNA encapsulation rate (%), mRNA concentration (µg/µL), and pKa of each Fluc mRNA-loaded lipid nanoparticle.

In Table 1, "nd" means not determined.

**Table 1**

| TxT | ζ-average particle size [nm] | number average particle size [nm] | Pdl | ζ-potential [mV] | mRNA encapsulation rate [%] | mRNA concentration (µg/µL) | pKa |
|---|---|---|---|---|---|---|---|
| TxT-5-5A16 | 85.6 | 61.0 | 0.11 | -3.3 | 96.7 | 0.020 | 6.27 |
| TxT-5-5A14 | 106.2 | 72.4 | 0.11 | -3.4 | 98.8 | 0.025 | 4.99 |
| TxT-5-5A12 | 155.2 | 124.5 | 0.08 | -6.7 | 85.9 | 0.019 | 6.44 |
| TxT-5-5A12β | 111.9 | 82.8 | 0.08 | -6.0 | 97.0 | 0.024 | 4.90 |
| TxT-5-5A10 | 426.0 | 213.0 | 0.42 | -36.4 | 56.5 | 0.012 | 7.94 |
| TxT-5-5A8 | 92.9 | 61.6 | 0.19 | -5.3 | 23.7 | 0.005 | nd |
| TxT-5-5A8β | 255.9 | 110.3 | 0.30 | -18.0 | 73.4 | 0.009 | nd |

As shown in Table 1, lipid nanoparticles encapsulating mRNA were successfully produced regardless of which TxT lipid was used. Furthermore, the physical properties of these lipid nanoparticles were all suitable for use as gene carriers.

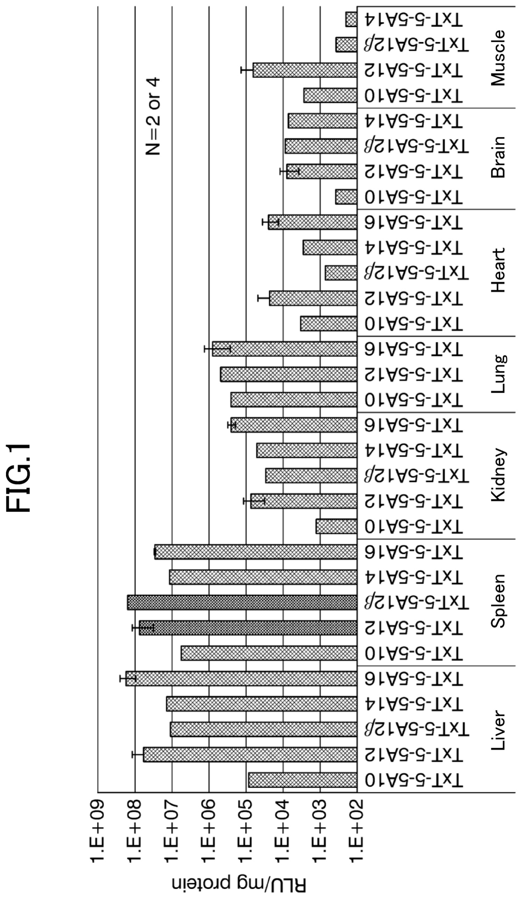

Subsequently, lipid nanoparticles encapsulating Fluc mRNA prepared using TxT-5-5A10, TxT-5-5A12, TxT-5-5A12β, TxT-5-5A14, and TxT-5-5A16 were administered to mice to evaluate luciferase expression levels (Fluc activity) in various tissues. Specifically, each Fluc mRNA-loaded lipid nanoparticle was administered to Balb/c mice at a dose of 0.1 mg mRNA/kg. Six hours after administration, the mice were euthanized, and their organs were collected to measure Fluc activity (RLU/mg protein) (N = 2 or 4). The results are shown in Figure 1.

As shown in Figure 1, Fluc activity was confirmed in the liver, spleen, kidney, lung, heart, brain, and muscle in all mice administered with any of the lipid nanoparticles. These results confirmed that lipid nanoparticles comprising TxT lipids as constituent lipids are useful as gene carriers targeting various organs. Furthermore, the Fluc mRNA-loaded lipid nanoparticles exhibited particularly high Fluc activity in the liver and spleen. Among them, the lipid nanoparticle comprising TxT-5-5A12 or TxT-5-5A12β as a constituent lipid showed the highest Fluc activity in the spleen, indicating their potential as carriers targeting the spleen.

### [Example 23]

Using TxT-5-5A16, which exhibited the highest Fluc activity in the liver in Example 22, lipid nanoparticles were prepared with varying ratios of DSPC, and luciferase expression levels (Fluc activity) were evaluated in various tissues.

Specifically, TxT-5-5A16, DSPC, cholesterol, and PEG-DMG were used in a composition of TxT-5-5A16/DSPC/chol/PEG-DMG=50/X/50-X/1.5 (mol%) (X = 3, 6, 9, 12, or 15) to prepare Fluc mRNA-loaded lipid nanoparticles in the same manner as Example 22. Table 2 shows the ζ-average particle size (nm), number average particle size (nm), polydispersity index (PdI), ζ-potential (mV), mRNA encapsulation rate (%), mRNA concentration (µg/µL), and pKa of each Fluc mRNA-loaded lipid nanoparticle.

**Table 2**

| DSPC concentration [mol %] | ζ-average particle size [nm] | number average particle size [nm] | Pdl | ζ-potential [mV] | mRNA encapsulation rate [%] | mRNA concentration (µg/µL) | pKa |
|---|---|---|---|---|---|---|---|
| 15 | 88.3 | 60.8 | 0.14 | -4.1 | 95.6 | 0.020 | 6.36 |
| 12 | 85.1 | 64.4 | 0.07 | -1.9 | 97.0 | 0.024 | 6.24 |
| 9 | 90.2 | 66.3 | 0.08 | -2.2 | 98.3 | 0.024 | 5.41 |
| 6 | 96.8 | 78.6 | 0.04 | -2.4 | 98.9 | 0.024 | 5.88 |
| 3 | 114.0 | 93.3 | 0.0 | -2.7 | 98.8 | 0.023 | 6.28 |

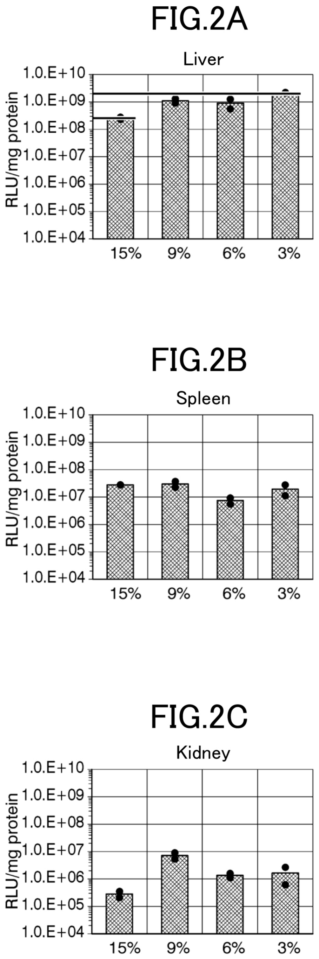

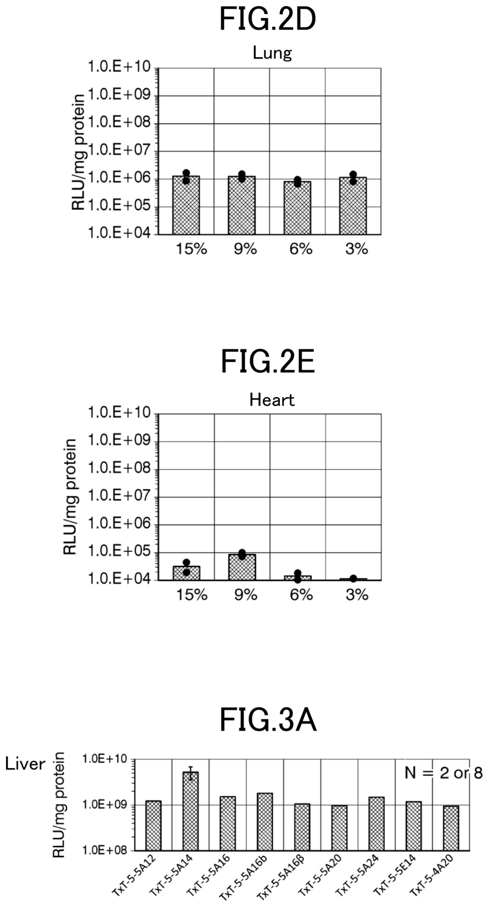

Subsequently, in the same manner as Example 22, each Fluc mRNA-loaded lipid nanoparticle was administered to mice to evaluate luciferase expression levels (Fluc activity) in various tissues (N = 2). The results are shown in Figs. 2A to 2E. In the figures, "3%", "6%", "9%", and "15%" indicate the results from mice administered with Fluc mRNA-loaded lipid nanoparticles containing 3 mol%, 6 mol%, 9 mol%, and 15 mol% DSPC, respectively. In the spleen (Fig. 2B), kidney (Fig. 2C), lung (Fig. 2D), and heart (Fig. 2E), no effect of DSPC content on Fluc activity was observed. In the liver (Fig. 2A), Fluc activity increased as the DSPC content decreased; specifically, the lipid nanoparticles containing 3 mol% DSPC exhibited approximately 10 times higher activity compared to those containing 15 mol% DSPC.

### [Example 24]

Lipid nanoparticles containing 3 mol% DSPC were prepared using the following TxT lipids as constituent lipids: TxT-5-5A12, TxT-5-5A12β, TxT-5-5A14, TxT-5-5A16, TxT-5-5A16b, TxT-5-5A16β, TxT-5-5A20, TxT-5-5A24, TxT-5-5E14, and TxT-5-4A20.

Specifically, TxT, DSPC, cholesterol, and PEG-DMG were used in a composition of TxT/DSPC/chol/PEG-DMG=50/3/47/1.5 (mol%) to prepare Fluc mRNA-loaded lipid nanoparticles by the alcohol dilution method. Table 3 shows the ζ-average particle size (nm), number average particle size (nm), polydispersity index (PdI), ζ-potential (mV), mRNA encapsulation rate (%), mRNA concentration (µg/µL), and pKa of each Fluc mRNA-loaded lipid nanoparticle.

**Table 3**

| TxT | ζ-average particle size [nm] | number average particle size [nm] | Pdl | ζ-potential [mV] | mRNA encapsulation rate [%] | mRNA concentration (µg/µL) | pKa |
|---|---|---|---|---|---|---|---|
| TxT-5-5A12 | 155.2 | 124.5 | 0.10 | -6.7 | 85.9 | 0.019 | 6.58 |
| TxT-5-5A12β | 96.0 | 72.8 | 0.08 | -5.6 | 99.5 | 0.023 | 6.53 |
| TxT-5-5A14 | 101.0 | 78.9 | 0.08 | -7.5 | 98.6 | 0.022 | 6.46 |
| TxT-5-5A16 | 114.0 | 93.3 | 0.03 | -2.7 | 98.8 | 0.023 | 6.28 |
| TxT-5-5A16b | 103.7 | 61.6 | 0.22 | -11.2 | 96.0 | 0.019 | 4.99 |
| TxT-5-5A16β | 89.4 | 69.3 | 0.07 | -7.4 | 99.2 | 0.018 | 4.90 |
| TxT-5-5A20 | 136.2 | 95.1 | 0.11 | -4.4 | 98.2 | 0.018 | 6.53 |
| TxT-5-5A24 | 133.1 | 92.2 | 0.14 | -8.9 | 96.5 | 0.022 | 6.68 |
| TxT-5-5E14 | 95.7 | 60.2 | 0.17 | -9.7 | 98.8 | 0.022 | 5.38 |
| TxT-5-4A20 | 136.8 | 98.6 | 0.08 | -3.7 | 98.4 | 0.019 | 6.49 |

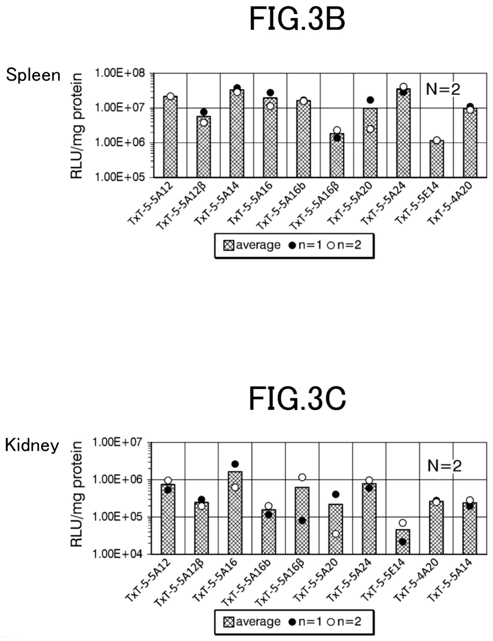

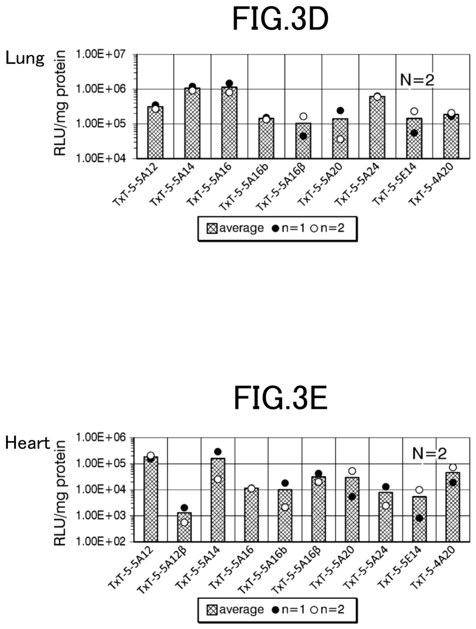

Subsequently, in the same manner as Example 22, each Fluc mRNA-loaded lipid nanoparticle was administered to mice to evaluate luciferase expression levels (Fluc activity) in various tissues (N = 2 or 8). The results are shown in Figs. 3A to 3E. In all mice administered with any of the lipid nanoparticles, Fluc activity in the liver (Fig. 3A) was observed to be more than ten times higher compared to that in the spleen (Fig. 3B), kidney (Fig. 3C), lung (Fig. 3D), and heart (Fig. 3E). In particular, the highest Fluc activity was observed in mice administered with lipid nanoparticles comprising TxT-5-5A14 as a constituent lipid, indicating that lipid nanoparticles comprising TxT-5-5A14 as a constituent lipid are promising as carriers targeting the liver.

### [Example 25]

The hepatic uptake efficiency of lipid nanoparticles comprising TxT-5-5A14 as a constituent lipid was compared with that of Fluc mRNA-loaded lipid nanoparticles formulated using SM-102, ALC-0315, or MC3 as a cationic lipid.

Specifically, TxT-5-5A14, DSPC, cholesterol, and PEG-DMG were used in a composition of TxT-5-SA14/DSPC/chol/PEG-DMG=50/3/47/1.5 (mol%) to prepare Fluc mRNA-loaded lipid nanoparticles by the alcohol dilution method. In addition, when SM-102, ALC-0315, or MC3 is used as a cationic lipid, they were used in a composition of cationic lipid/DSPC/chol/PEG-DMG=50/10/40/1.5 (mol%) to prepare Fluc mRNA-loaded lipid nanoparticles by the alcohol dilution method. Table 4 shows the ζ-average particle size (nm), number average particle size (nm), polydispersity index (PdI), ζ-potential (mV), mRNA encapsulation rate (%), mRNA concentration (µg/µL), and pKa of each Fluc mRNA-loaded lipid nanoparticle.

**Table 4**

| Cationic lipid | ζ-average particle size [nm] | number average particle size [nm] | Pdl | ζ-potential [mV] | mRNA encapsulation rate [%] | mRNA concentration (µg/µL) | pKa |
|---|---|---|---|---|---|---|---|
| TxT-5-5A14 | 113.8 | 82.4 | 0.08 | -8.9 | 99.4 | 0.022 | 6.53 |
| MC3 | 83.6 | 51.8 | 0.20 | -6.8 | 98.8 | 0.021 | 6.23 |
| SM-102 | 70.4 | 42.0 | 0.21 | -11.0 | 99.6 | 0.018 | 6.50 |
| ALC-0315 | 86.0 | 57.0 | 0.13 | -2.1 | 98.4 | 0.025 | 6.27 |

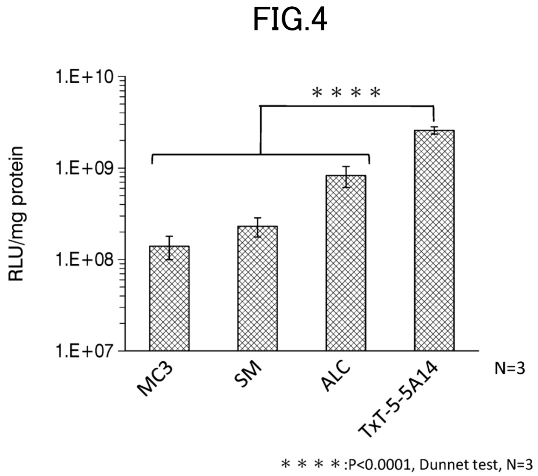

Subsequently, in the same manner as Example 22, each Fluc mRNA-loaded lipid nanoparticle was administered to mice to evaluate luciferase expression levels (Fluc activity) in the liver (N = 3). The results are shown in Fig. 4. In mice administered with lipid nanoparticles comprising TxT-5-5A14 as a constituent lipid, significantly higher Fluc activity was observed compared to those administered with lipid nanoparticles comprising other cationic lipids as constituent lipids. These findings suggest that lipid nanoparticles comprising TxT-5-5A14 as a constituent lipid may serve as superior gene carriers compared to existing ones.

### [Example 26]

The cellular uptake of lipid nanoparticles comprising TxT-5-5A14 as a constituent lipid in various types of cells in the liver was compared with that of Fluc mRNA-loaded lipid nanoparticles formulated using SM-102, ALC-0315, or MC3 as a cationic lipid.

Specifically, in the same manner as Example 25 except for the use of EGFP mRNA in place of Fluc mRNA, EGFP mRNA-loaded lipid nanoparticles consisting of a composition of TxT-5-SA14/DSPC/chol/PEG-DMG=50/3/47/1.5 (mol%) or of SM-102 or ALC-0315 or MC3/DSPC/chol/PEG-DMG=50/10/40/1.5 (mol%) were prepared.

Subsequently, each EGFP mRNA-loaded lipid nanoparticle was administered to Balb/c mice at a dose of 0.2 mg mRNA/kg. At 23 hours and 50 minutes post-administration, fluorescently labeled lectin (DyLight-649 Lycopersicon (Tomato) Lectin) was intravenously injected via the tail vein at 40 µg/mouse to fluorescently label vascular endothelial cells. At 24 hours after administration of the lipid nanoparticles, the mice were euthanized, and their livers were collected to prepare tissue sections. For nuclear staining, the tissue sections were immersed in a nuclear staining solution (1 µg/mL Hoechst 33342 dissolved in PBS(-)), cooled on ice, and incubated for at least 30 minutes in the dark. The stained tissue sections were then observed using a confocal laser scanning microscope.

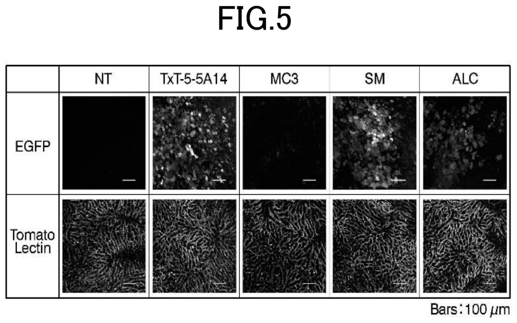

Microscopic images of liver tissue sections from each mouse are shown in Fig. 5. In the figure, the upper panels show fluorescence images of EGFP, and the lower panels show fluorescence images of vascular endothelial cells (fluorescently labeled lectin). As shown in Fig. 5, in the liver of mice administered with EGFP mRNA-loaded lipid nanoparticles comprising TxT-5-5A14 as a constituent lipid, GFP expression was observed not only in hepatocytes but also in vascular endothelial cells. These results confirmed that lipid nanoparticles containing TxT lipids are taken up not only by hepatocytes but also by vascular endothelial cells in the liver.

### [Example 27]

Each of EGFP mRNA-loaded lipid nanoparticles comprising TxT lipids as constituent lipids was administered to mice and the expression efficiency in hepatocytes was evaluated. As the TxT lipids, TxT-5-5A12, TxT-5-5A12β, TxT-5-5A14, TxT-5-5A16, TxT-5-5A16b, TxT-5-5A16β, TxT-5-5A20, TxT-5-5A24, TxT-5-5E14, and TxT-5-4A20 were used.

Specifically, in the same manner as Example 24 except that EGFP mRNA was used in place of Fluc mRNA and that the total flow rate during liquid delivery into the microchannel was set to 3.0 mL/min, EGFP mRNA-loaded lipid nanoparticles consisting of a composition of TxT/DSPC/chol/PEG-DMG=50/3/47/1.5 (mol%) were prepared. Table 5 shows the ζ-average particle size (nm), number average particle size (nm), polydispersity index (PdI), ζ-potential (mV), mRNA encapsulation rate (%), and mRNA concentration (µg/µL) of each EGFP mRNA-loaded lipid nanoparticle. As shown in Table 5, uniform particles were successfully prepared regardless of the TxT lipids.

**Table 5**

| TxT | ζ-average particle size [nm] | number average particle size [nm] | Pdl | ζ-potential [mV] | mRNA encapsulation rate [%] | mRNA concentration (µg/µL) |
|---|---|---|---|---|---|---|
| TxT-5-5A12 | 114.4 | 80.2 | 0.13 | -9.4 | 95.5 | 0.019 |
| TxT-5-5A12β | 104.0 | 81.9 | 0.05 | -7.8 | 99.1 | 0.016 |
| TxT-5-5A14 | 106.0 | 80.9 | 0.06 | -13.0 | 99.0 | 0.023 |
| TxT-5-5A16 | 132.4 | 112.5 | 0.04 | -8.4 | 99.2 | 0.022 |
| TxT-5-5A16b | 81.4 | 59.1 | 0.07 | -6.0 | 97.9 | 0.020 |
| TxT-5-5A16β | 101.1 | 80.1 | 0.06 | -8.9 | 99.1 | 0.016 |
| TxT-5-5A20 | 136.2 | 108.4 | 0.08 | -8.7 | 97.8 | 0.022 |
| TxT-5-5A24 | 129.5 | 85.3 | 0.10 | -4.2 | 97.3 | 0.021 |
| TxT-5-5E14 | 103.3 | 71.1 | 0.14 | -11.6 | 98.3 | 0.024 |
| TxT-5-4A20 | 140.9 | 110.1 | 0.08 | -7.4 | 96.2 | 0.021 |

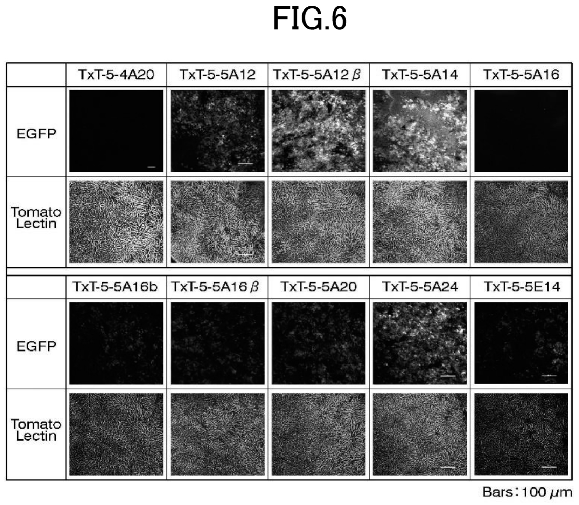

Subsequently, in the same manner as Example 26, each EGFP mRNA-loaded lipid nanoparticle was administered to Balb/c mice. At 23 hours and 50 minutes post-administration, fluorescently labeled lectin was administered. At 24 hours after administration of the lipid nanoparticles, the mice were euthanized, and their livers were collected to prepare tissue sections. Each tissue section was observed using a confocal laser scanning microscope. Microscopic images of liver tissue sections from each mouse are shown in Fig. 6. In the figure, the upper panels show fluorescence images of EGFP, and the lower panels show fluorescence images of vascular endothelial cells (fluorescently labeled lectin).

As shown in Fig. 6, fluorescence of EGFP was observed in the liver tissues of mice administered with EGFP mRNA-loaded lipid nanoparticles containing TxT-5-5A12, TxT-5-5A12β, TxT-5-5A14, TxT-5-5A16b, TxT-5-5A16β, TxT-5-5A24, TxT-5-5E14, or TxT-5-5A20. In particular, hepatocytes of mice administered with lipid nanoparticles containing TxT-5-5A12β, TxT-5-5A14, or TxT-5-5A24 exhibited notably high levels of gene expression. These results confirmed that lipid nanoparticles comprising TxT lipids as constituent lipids are useful as gene carriers targeting the liver. In contrast, in the liver tissues of mice administered with EGFP mRNA-loaded lipid nanoparticles containing TxT-5-4A20 or TxT-5-5A16, EGFP expression was not detectable under the observation conditions (24 hours post-administration), suggesting that the time required to reach peak gene expression from administration may vary depending on the specific TxT lipid used.

### [Example 28]

The time-dependent changes in gene expression in the liver were evaluated for lipid nanoparticles comprising TxT-5-5A12β or TxT-5-5A14 as a constituent lipid.

Specifically, TxT5-5A12β or TxT-5-5A14, DSPC, cholesterol, and PEG-DMG were used in a composition of TxT-5-5A12β or TxT-5-5A14/DSPC/chol/PEG-DMG=50/3/47/1.5 (mol%) to prepare Fluc mRNA-loaded lipid nanoparticles by the alcohol dilution method. Table 6 shows the ζ-averageparticle size (nm), number average particle size (nm), polydispersity index (PdI), ζ-potential (mV), mRNA encapsulation rate (%), and mRNA concentration (µg/µL) of each Fluc mRNA-loaded lipid nanoparticle.

**Table 6**

| TxT | ζ-average particle size [nm] | number average particle size [nm] | Pdl | ζ-potential [mV] | mRNA encapsulation rate [%] | mRNA concentration (µg/µL) |
|---|---|---|---|---|---|---|
| TxT-5-5A12β | 92.0 | 71.2 | 0.06 | -8.4 | 98.0 | 0.020 |
| TxT-5-5A14 | 107.7 | 79.6 | 0.09 | -6.4 | 98.2 | 0.021 |

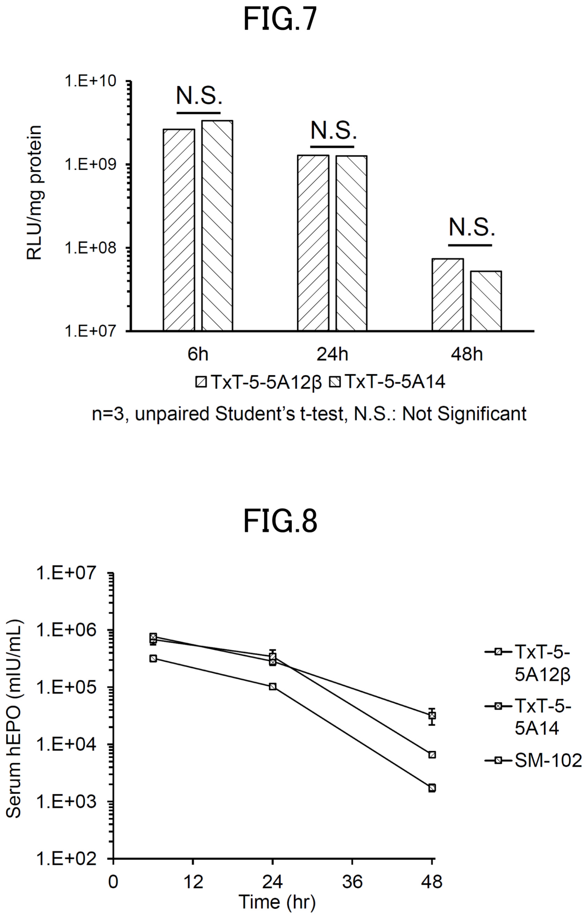

Subsequently, in the same manner as Example 22, each Fluc mRNA-loaded lipid nanoparticle was administered to mice to evaluate luciferase expression levels (Fluc activity) in the liver at 6, 24, and 48 hours after administration (N = 3). The results are shown in Fig. 7. Lipid nanoparticles comprising TxT-5-5A12β as a constituent lipid and those comprising TxT-5-5A14 as a constituent lipid exhibited comparable levels of gene expression at all time points.

### [Example 29]

The time-dependent changes in expression efficiency of hEPO with lipid nanoparticles comprising TxT-5-5A12β or TxT-5-5A14 as a constituent lipid were compared to those with hEPO mRNA-loaded lipid nanoparticles prepared using SM-102.

Specifically, TxT-5-5A12β or TxT-5-5A14, DSPC, cholesterol, and PEG-DMG were used in a composition of TxT-5-5A12β or TxT-5-5A14/DSPC/chol/PEG-DMG=50/3/47/1.5 (mol%) to prepare hEPO mRNA-loaded lipid nanoparticles by the alcohol dilution method. In addition, when SM-102 is used as a cationic lipid, they were used in a composition of SM-102/DSPC/chol/PEG-DMG=50/10/40/1.5 (mol%) to prepare hEPO mRNA-loaded lipid nanoparticles by the alcohol dilution method. Table 7 shows the ζ-average particle size (nm), number average particle size (nm), polydispersity index (PdI), ζ-potential (mV), mRNA encapsulation rate (%), and mRNA concentration (µg/µL) of each hEPO mRNA-loaded lipid nanoparticle.

**Table 7**

| Cationic lipid | ζ-average particle size [nm] | number average particle size [nm] | Pdl | ζ-potential [mV] | mRNA encapsulation rate [%] | mRNA concentration (µg/µL) |
|---|---|---|---|---|---|---|
| TxT-5-5A12β | 88.9 | 66.9 | 0.10 | -3.0 | 99.5 | 0.017 |
| TxT-5-5A14 | 103.4 | 76.6 | 0.04 | -1.4 | 99.3 | 0.022 |
| SM-102 | 78.8 | 38.6 | 0.19 | 0.0 | 97.5 | 0.015 |

Subsequently, each hEPO mRNA-loaded lipid nanoparticle was administered to mice, and blood samples were collected from the tail vein at 6, 24, and 48 hours after administration (N = 3). The collected blood was allowed to stand at room temperature for 90 minutes, followed by centrifugation at 25°C, 1,000 x g for 15 minutes. The supernatant was collected to obtain serum. The hEPO activity in the serum was measured using a commercially available ELISA kit (Human EPO ELISA Kit, Invitrogen). Calibration samples were prepared by serial dilution from 100 mIU/mL to obtain solutions of 50, 25, 12.5, 6.3, and 3.1 ng/mL, each in a volume of 450 µL. Serum samples were diluted 10- to 10,000-fold to fall within the calibration range. A volume of 100 µL of each calibration sample and serum sample was applied to the plate, followed by the addition of 450 µL of Biotin-Conjugate and incubation at 18 to 25°C for 1 hour. The wells were washed six times, and 100 µL of Streptavidin-HRP was added to each well and incubated at 18 to 25°C for 15 minutes. After another six washes, 100 µL of TMB Substrate Solution was added to each well, and the plate was shielded from light using aluminum foil and incubated at 18 to 25°C for 10 minutes. Finally, 100 µL of Stop Solution was added, and absorbance at 450 nm was measured using a spectrofluorometer (Varioskan Lux, Thermo Fisher Scientific).

The results are shown in Fig. 8. In both mice administered with lipid nanoparticles comprising TxT-5-5A12β as a constituent lipid and those administered with lipid nanoparticles comprising TxT-5-5A14 as a constituent lipid, clearly higher hEPO concentrations were observed compared to mice administered with lipid nanoparticles comprising SM-102 as a constituent lipid. These findings suggest that lipid nanoparticles comprising TxT-5-5A12β or TxT-5-5A14 as a constituent lipid may serve as superior gene carriers compared to existing gene carriers.

### [Example 30]

Lipid nanoparticles (Fluc mRNA-loaded lipid nanoparticles) containing 3 mol% DSPC and using as constituent lipids, TxT-5-5A12, TxT-5-5A12β, TxT-5-5A14, TxT-5-5A14β, TxT-5-5A16, TxT-5-5A16b, TxT-5-5A16β, TxT-5-5A20, TxT-5-5A24, TxT-5-5A24β, TxT-5-5E14, and TxT-5-4A20, were prepared, and luciferase expression levels (Fluc activity) in the liver were evaluated.

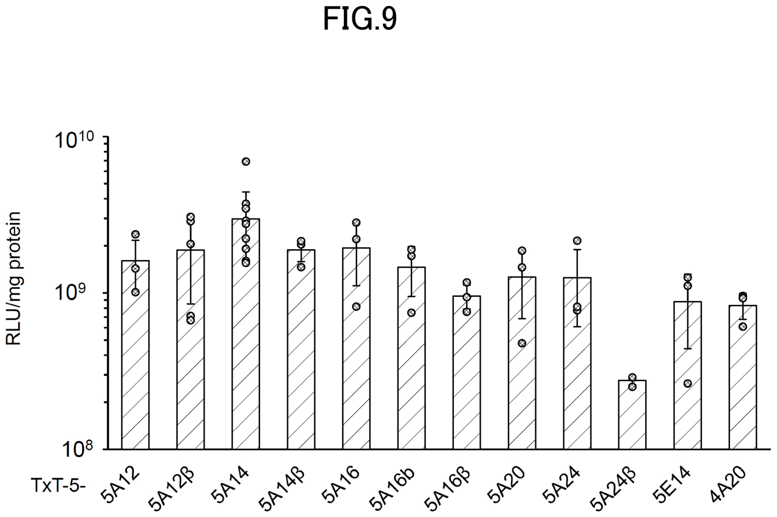

For each of the prepared Fluc mRNA-loaded lipid nanoparticles, tests were conducted in the same manner as Example 24 (N = 3 to 9). The Fluc activity (RLU/mg protein) in the liver of mice administered with the Fluc mRNA-loaded lipid nanoparticles was measured, and the results are shown in Fig. 9. Fluc activity was confirmed in all mice administered with the respective lipid nanoparticles.

### [Example 31]

Lipid nanoparticles (Fluc mRNA-loaded lipid nanoparticles) containing 3 mol% DSPC and using TxT-5-4(6)A14 as a constituent lipid were prepared, and luciferase expression levels (Fluc activity) in various tissues were evaluated.

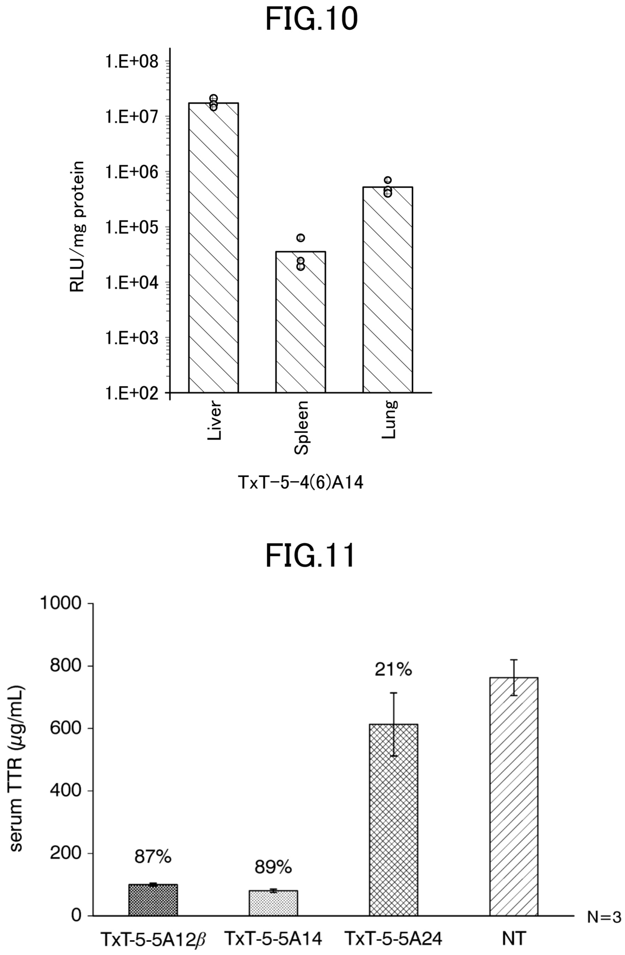

For the prepared Fluc mRNA-loaded lipid nanoparticles, tests were conducted in the same manner as Example 24 (however, N = 3). The Fluc activity (RLU/mg protein) in the liver, spleen, and lung of mice administered with the Fluc mRNA-loaded lipid nanoparticles was measured, and the results are shown in Fig. 10. Fluc activity was observed in the organs, i.e., liver, spleen, and lungs, with particularly high activity confirmed in the liver.

### [Example 32]

Lipid nanoparticles comprising various TxT lipids as constituent lipids were used as DDS carriers for genome editing. As TxT lipids, TxT-5-5A12β, TxT-5-5A14, and TxT-5-5A24, which exhibited high gene expression in Example 27, were employed.

Specifically, an RNA solution was first prepared to contain Cas9-mRNA (TriLink, SmoU-modified) and gRNA targeting transthyretin (TTR) at a mass ratio of 1:2 (N/P = 6.0). Using this RNA solution, in the same manner as Example 27, lipid nanoparticles encapsulating Cas9-mRNA and TTR-gRNA (SEQ ID NO: 1) and having a composition of TxT/DSPC/chol/PEG-DMG = 50/3/47/1.5 (mol%) were prepared (lipid nanoparticles for TTR-targeted genome editing). Table 8 shows the ζ-averageparticle diameter (nm), number-average particle diameter (nm), polydispersity index (PdI), ζ-potential (mV), mRNA encapsulation efficiency (%), and mRNA concentration (µg/µL) of each lipid nanoparticle for TTR-targeted genome editing.

**Table 8**

| TxT | ζ-average particle size [nm] | number average particle size [nm] | Pdl | ζ-potential [mV] | mRNA encapsulation rate [%] | mRNA concentration (µg/µL) |
|---|---|---|---|---|---|---|
| TxT-5-5A12β | 87.8 | 71.3 | 0.02 | -3.8 | 99.1 | 0.412 |
| TxT-5-5A14 | 82.2 | 66.4 | 0.05 | -5.5 | 96.5 | 0.467 |
| TxT-5-5A24 | 73.8 | 53.1 | 0.12 | -6.5 | 98.4 | 0.541 |

As shown in Table 8, uniform particles were successfully prepared using all TxT lipids. Among them, lipid nanoparticles containing TxT-5-5A24 exhibited the smallest average particle diameter, while those containing TxT-5-5A12β exhibited the largest. These results suggest a tendency for particles composed of lipids with shorter scaffold carbon chains (i.e., lipids in which the number of carbon atoms in R¹ of general formula (I) is smaller) to have larger average particle diameters. Furthermore, lipid nanoparticles for TTR-targeted genome editing encapsulating Cas9 mRNA tended to exhibit smaller average particle diameters compared to lipid nanoparticles encapsulating Flue mRNA (Example 24) or EGFP mRNA (Example 27), even when the lipid composition was the same.

Subsequently, each of the prepared lipid nanoparticles for TTR-targeted genome editing was administered via tail vein injection to female Balb/c mice (4 weeks old) at a dose of 1.5 mg Cas9 mRNA/kg. One week after administration, the mice were euthanized, and blood was collected from the inferior vena cava (N = 3). The collected blood was allowed to stand at room temperature for 1 hour, followed by centrifugation at 25°C, 1,000 × g for 15 minutes. The supernatant was collected to obtain serum. The TTR concentration in the serum was measured using a commercially available ELISA kit (Prealbumin ELISA Kit Mouse, Aviva Systems Biology). Calibration samples were prepared by serial dilution from 500 ng/mL to obtain solutions of 250, 125, 62.5, 31.25, and 15.63 ng/mL, each in a volume of 300 µL. Serum samples were diluted 10,000-fold. A volume of 100 µL of each calibration sample and serum sample was applied to the plate and incubated at 25°C for 30 minutes. The wells were washed four times, and 100 µL of Enzyme-Antibody Conjugate was added to each well. The plate was shielded from light using aluminum foil and incubated at 25°C for 20 minutes. After another four washes, 100 µL of TMB Substrate Solution (Aviva Systems Biology) was added to each well, and the plate was shielded from light using aluminum foil and incubated at 25°C for 10 minutes. Finally, 100 µL of Stop Solution (Aviva Systems Biology) was added, and absorbance at 450 nm was measured using a spectrofluorometer (Varioskan Lux, Thermo Fisher Scientific).

Figure 11 shows the measurement results of serum TTR levels (µg/mL) and TTR knockdown efficiency (%). In the figure, "NT" indicates the measurement result of serum from mice that were not administered lipid nanoparticles. Lipid nanoparticles for TTR-targeted genome editing containing TxT-5-5A12β and those containing TxT-5-5A14 exhibited very high TTR knockout activity, exceeding 85%. These results suggest that the use of lipid nanoparticles comprising TxT-5-5A12β or TxT-5-5A14 as a constituent lipid enables efficient genome editing via mRNA delivery.

### [Example 33]

Lipid nanoparticles comprising TxT-5-5A14 as a constituent lipid were used as DDS carriers for genome editing.

Specifically, an RNA solution was first prepared to contain Cas9-mRNA (TriLink, 5moU-modified) and gRNA targeting transthyretin (TTR) at a mass ratio of 1:2 (N/P = 6.0). Using this RNA solution, in the same manner as Example 32, lipid nanoparticles encapsulating Cas9-mRNA and TTR-gRNA and having a composition of TxT-5-5A14/DSPC/chol/PEG-DMG = 50/3/47/1.5 (mol%) were prepared (lipid nanoparticles for TTR-targeted genome editing). Table 9 shows the ζ-average particle diameter (nm), number-average particle diameter (nm), polydispersity index (PdI), ζ-potential (mV), mRNA encapsulation efficiency (%), and mRNA concentration (µg/µL) of the lipid nanoparticle for TTR-targeted genome editing.

**Table 9**

| TxT | ζ-average particle size [nm] | number average particle size [nm] | Pdl | ζ-potential [mV] | mRNA encapsulation rate [%] | mRNA concentration (µg/µL) |
|---|---|---|---|---|---|---|
| TxT-5-5A14 | 91.8 | 67.7 | 0.10 | -4.0 | 99.1 | 0.037 |

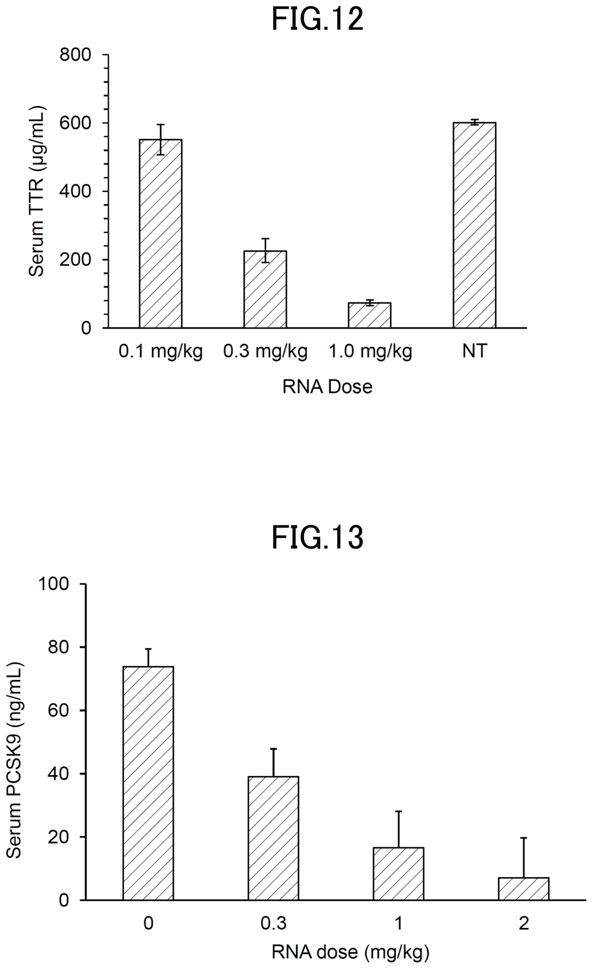

Subsequently, the lipid nanoparticles for TTR-targeted genome editing were administered to mice and serum TTR levels (µg/mL) were measured (N = 3) in the same manner as in Example 32, except that a dose of Cas9-mRNA was adjusted to 0.1, 0.3, and 1.0 mg/kg. The results are shown in Fig. 12. In the figure, "NT" indicates the measurement result of serum from mice that were not administered lipid nanoparticles. A dose-dependent increase in TTR knockout efficiency was observed, with no less than 60% TTR knockout activity achieved at a dose of 0.3 mg RNA/kg. These results suggest that efficient genome editing by mRNA can be achieved by using as a carrier lipid nanoparticle comprising TxT-5-5A14 as a constituent lipid.

### [Example 34]

Lipid nanoparticles comprising TxT-5-5A14 as a constituent lipid were used as DDS carriers for genome editing.

Specifically, an RNA solution was first prepared to contain Cas9-mRNA (TriLink, 5moU-modified) and gRNA targeting proprotein convertase subtilisin/kexin type 9 (PCSK9) (SEQ ID NO: 3) at a mass ratio of 1:2 (N/P = 6.0). Using this RNA solution, in the same manner as Example 32, lipid nanoparticles encapsulating Cas9-mRNA and PCSK9-gRNA and having a composition of TxT-5-SA14/DSPC/chol/PEG-DMG = 50/3/47/1.5 (mol%) were prepared (lipid nanoparticles for PCSK9-targeted genome editing). Table 10 shows the ζ-average particle diameter (nm), number-average particle diameter (nm), polydispersity index (PdI), ζ-potential (mV), mRNA encapsulation efficiency (%), and mRNA concentration (µg/µL) of the lipid nanoparticle for PCSK9-targeted genome editing.

**Table 10**

| TxT | ζ-average particle size [nm] | number average particle size [nm] | Pdl | ζ-potential [mV] | mRNA encapsulation rate [%] | mRNA concentration (µg/µL) |
|---|---|---|---|---|---|---|
| TxT-5-5A14 | 99.4 | 73.6 | 0.10 | -1.6 | 97.1 | 0.311 |

Subsequently, the prepared lipid nanoparticles for PCSK9-targeted genome editing were administered via tail vein injection to female Balb/c mice (4 weeks old) at doses of 0.3, 1.0, and 2.0 mg RNA/kg. Ten days after administration, the mice were euthanized, and blood was collected from the inferior vena cava (N = 3 to 6). The collected blood was allowed to stand at room temperature for 1 hour, followed by centrifugation at 4°C, 1,000 × g for 10 minutes. The supernatant was collected to obtain serum. The concentration of PCSK9 in the serum was measured using a commercially available ELISA kit (Mouse/Rat PCSK9 ELISA Kit, CircuLex). Calibration samples were prepared by serial dilution from 6 ng/mL to obtain solutions of 3, 1.5, 0.75, 0.375, 0.188, and 0.094 ng/mL, each in 300 µL volumes. Serum samples were diluted 100-fold. A volume of 100 µL of each calibration sample and serum sample was applied to the plate and incubated at 25°C for 60 minutes with shaking at 300 rpm. The wells were washed four times, and 100 µL of HRP-conjugated Detection Antibody (CircuLex) was added to each well and incubated again at 25°C for 60 minutes with shaking at 300 rpm. The wells were washed four more times, and 100 µL of Substrate Reagent (CircuLex) was added to each well. The plate was shielded from light using aluminum foil and incubated at 25°C for 10 to 20 minutes with shaking at 300 rpm. Finally, 100 µL of Stop Solution (CircuLex) was added, and absorbance at 450 nm and 540 nm was measured using a spectrophotometer (Varioskan Lux, Thermo Fisher Scientific).

Fig. 13 shows the measurement results of PCSK9 concentration in serum (ng/mL). A dose-dependent increase in PCSK9 knockout efficiency was observed, with no less than 90% knockout activity achieved at a dose of 2.0 mg RNA/kg. These results suggest that efficient genome editing by mRNA can be achieved by using as a carrier lipid nanoparticle comprising TxT-5-5A14 as a constituent lipid.

The sequences of the gRNAs used in the above examples are as follows.

**Table 11**

| Name (SEQ ID NO) | Sequence |
|---|---|
| sgTTR (SEQ ID NO: 1) | mU*mU*mA*CAGCCACGUCUACAGCAGUUUUAGAGCUAGAAAUAG CAAGUUAAAAUAAGGCUAGUCCGUUAUCAACUUGAAAAAGUGG CACCGAGUCGGUGCmU*mU*mU*U |
| Highly modified sgTTR (SEQ ID NO: 2) | mU*mU*mA*CAGCCACGUCUACAGCAGUUUUAGAmGmCmUmAmG mAmAmAmUmAmGmCAAGUUAAAAUAAGGCUAGUCCGUUAUCAm AmCmUmUmGmAmAmAmAmAmGmUmGmGmCmAmCmCmGmAmGm UmCmGmGmUmGmCmU*mU*mU*mU |
| sgPCSK9 (SEQ ID NO: 3) | mC*mC*mC*AUACCUUGGAGCAACGGGUUUUAGAGCUAGAAAUAG CAAGUUAAAAUAAGGCUAGUCCGUUAUCAACUUGAAAAAGUGG CACCGAGUCGGUGCmU*mU*mU*U |

| | |
|---|---|
| mU represents 2'-O-methyluridine, mA represents 2'-O-methyladenosine, mC represents 2'-O-methylcytidine, mG represents 2'-O-methylguanosine, and * denotes a phosphorothioate linkage. Specifically, mU* represents 2'-O-methyluridine with a 3'-phosphorothioate linkage, mA* represents 2'-O-methyladenosine with a 3'-phosphorothioate linkage, and mC* represents 2'-O-methylcytidine with a 3'-phosphorothioate linkage. | |

## Claims

1. A pH-sensitive cationic lipid comprising a compound represented by the following general formula (I) or (I'): 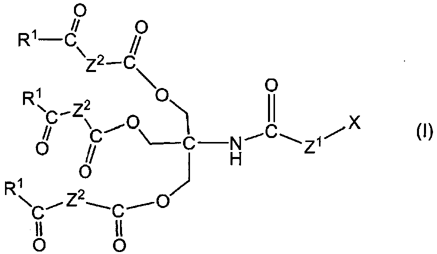 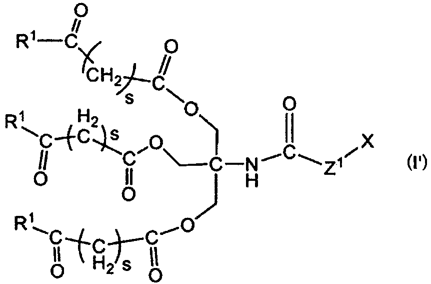
wherein, in the formulae (I) and (I'), R¹ is a group represented by any of the general formulae (T-1) to (T-3): 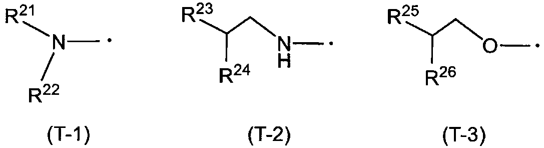
wherein, in the general formulae, R²¹ is a hydrocarbon group having 1 to 22 carbon atoms; R²² is a hydrocarbon group having 1 to 22 carbon atoms; R²³ is a hydrocarbon group having 1 to 22 carbon atoms; R²⁴ is a hydrogen atom or a hydrocarbon group having 1 to 22 carbon atoms; R²⁵ is a hydrocarbon group having 1 to 22 carbon atoms; and R²⁶ is a hydrogen atom or a hydrocarbon group having 1 to 22 carbon atoms, provided that the total number of carbon atoms in R²¹ and R²² is equal to or greater than 8, the total number of carbon atoms in R²³ and R²⁴ is equal to or greater than 6, and the total number of carbon atoms in R²⁵ and R²⁶ is equal to or greater than 6; and a black circle represents a bonding arm; and
three R¹ groups in one molecule may be the same as or different from each other; Z¹ is an alkylene group having 1 to 6 carbon atoms which may be substituted with one hydroxyalkyl group having 1 to 3 carbon atoms, or a single bond; X is -N(R²)(R³) or a 5-to 7-membered nonaromatic heterocyclic group, wherein the group is bonded to Z¹ via a carbon atom, and the ring may be substituted to have one or two hydrocarbon groups having 1 to 4 carbon atoms or amide groups thereon; and R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group,
in the formula (I), Z² is a linear alkylene group having 4 to 6 carbon atoms or a cycloalkylene group; three Z² in one molecule may be the same as or different from each other, and
in formula (I'), "s" is 2, 3, or 4; three "s" in one molecule may be the same as or different from each other.

2. The pH-sensitive cationic lipid according to claim 1, wherein -Z¹-X in the following general formula (I) or (I') is selected from the following formulae (H-1) to (H-8) and (H-16) to (H-26): 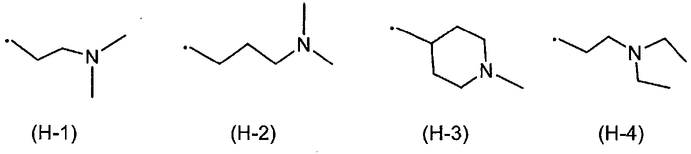 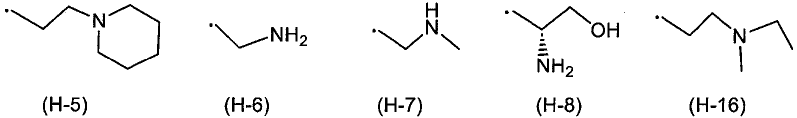 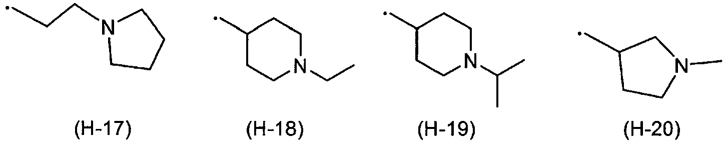 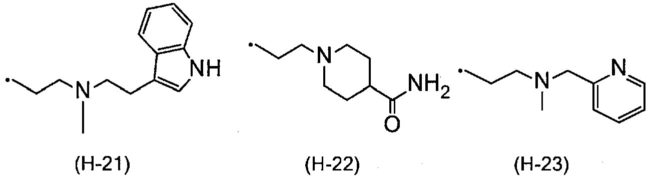 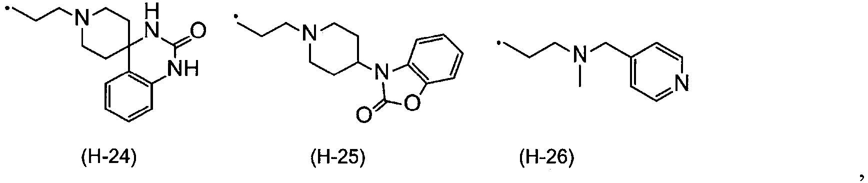 wherein a black circle represents a bonding arm.

3. The pH-sensitive cationic lipid according to claim 1, wherein the three R¹ groups in one molecule are all the same.

4. The pH-sensitive cationic lipid according to claim 1, which is represented by the general formula (I') wherein "s" is 2 or 3.

5. The pH-sensitive cationic lipid according to claim 1, wherein R¹ is any group of the formula (A8), (A8β), (A10), (A12), (A12β), (A14), (A16), (A16β), (A16b), (A20), (A24), (E12), (E14), (A14β), (A24β), (A10L), (A12L), or (A14L):

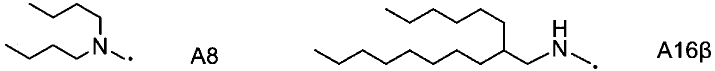

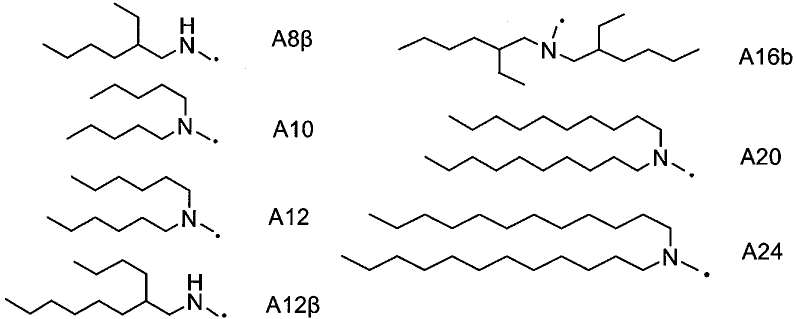

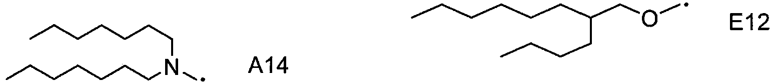

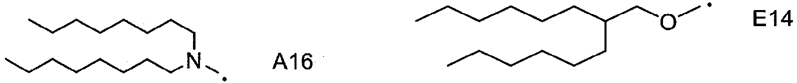

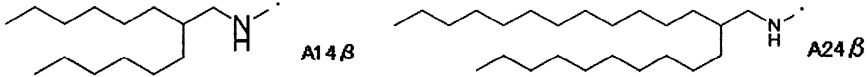

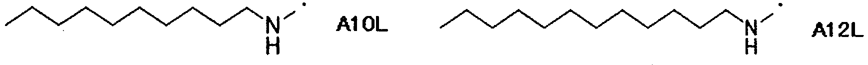

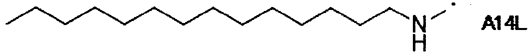

6. The pH-sensitive cationic lipid according to claim 1, wherein pKa is between 4.0 and 9.0.

7. The pH-sensitive cationic lipid according to claim 1 which is selected from the following compounds:

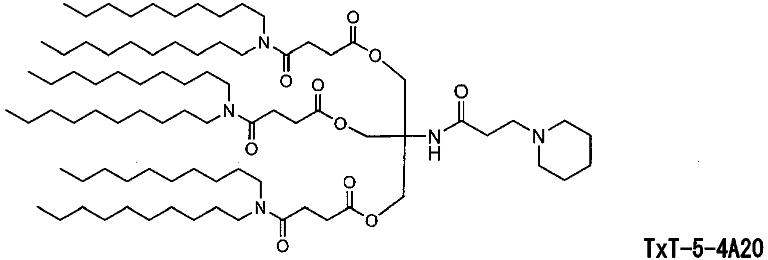

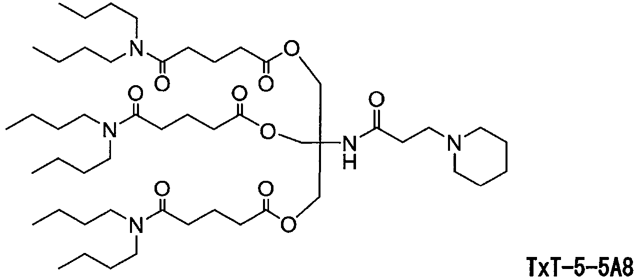

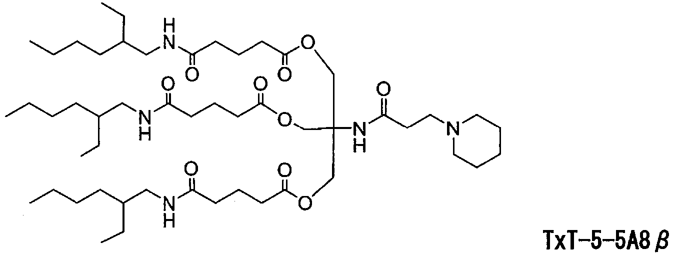

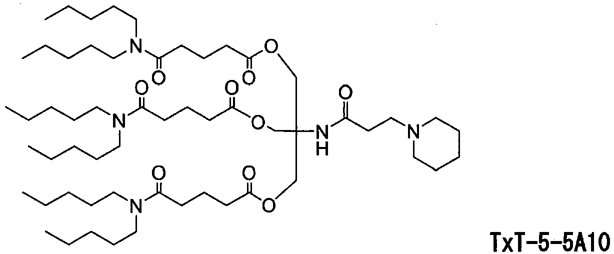

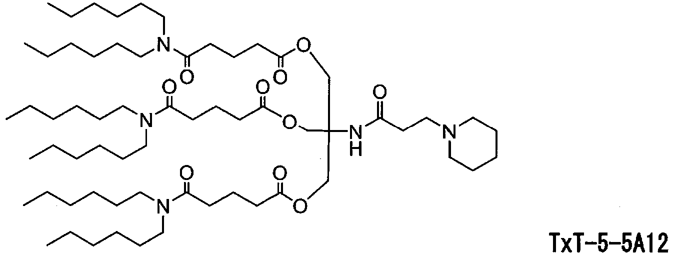

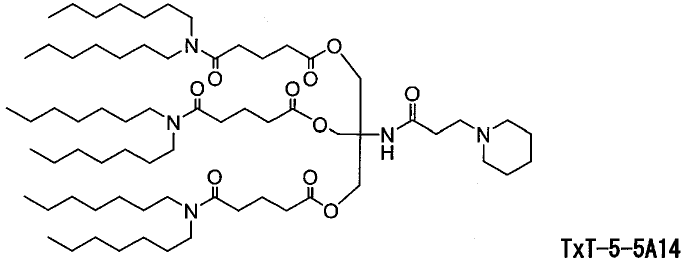

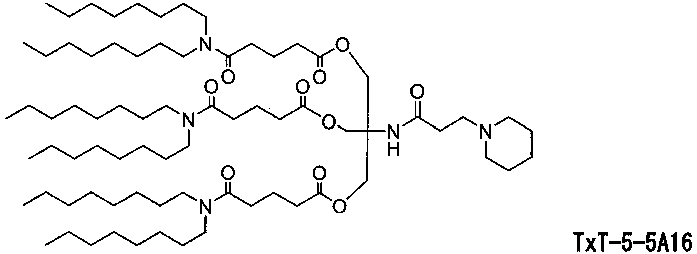

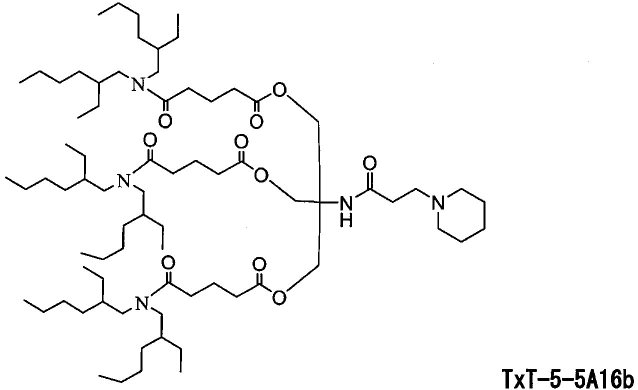

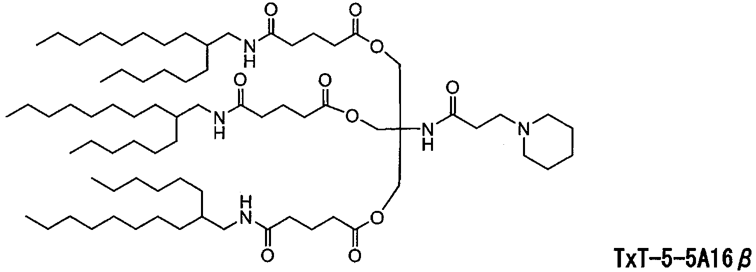

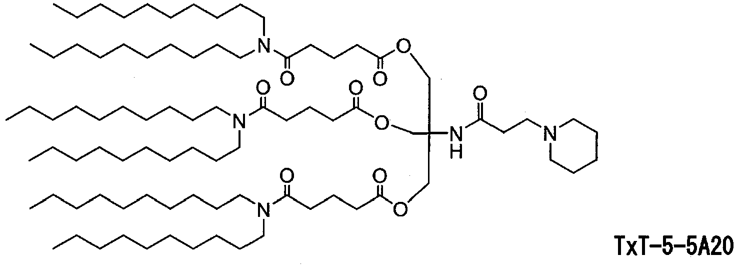

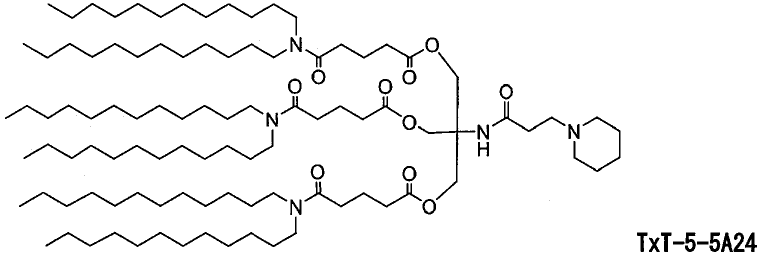

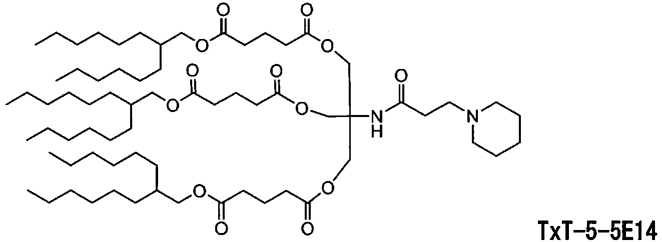

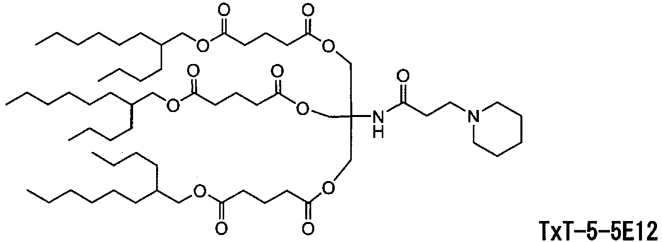

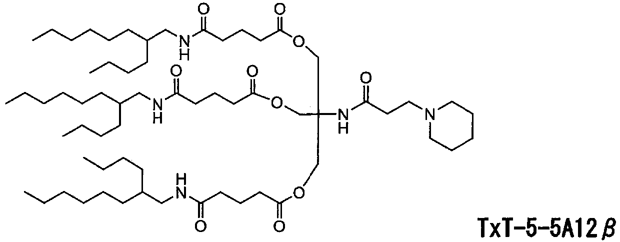

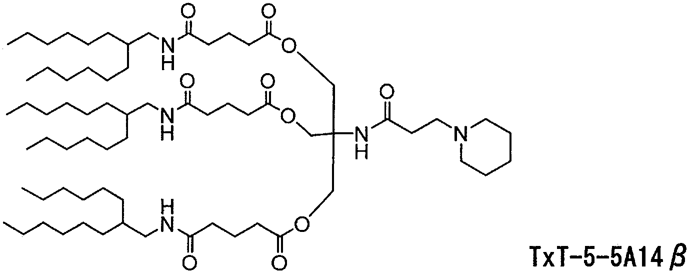

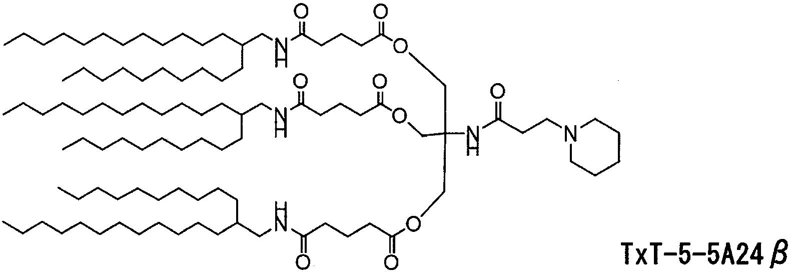

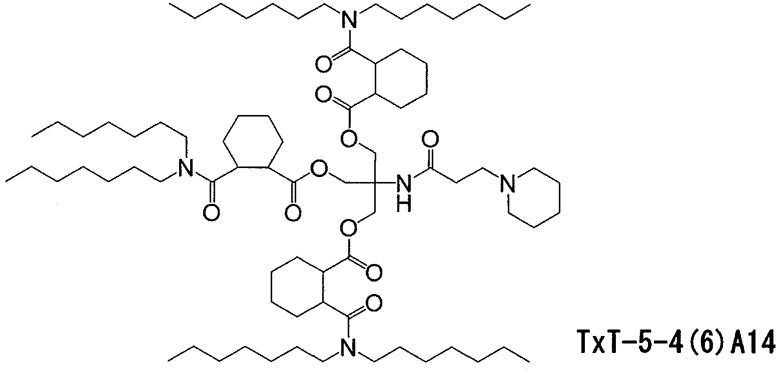

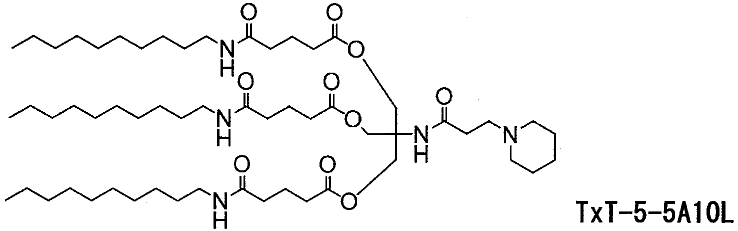

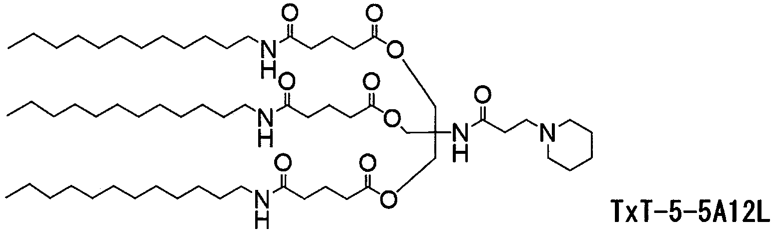

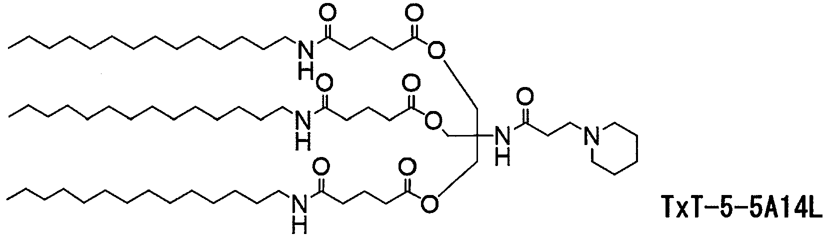

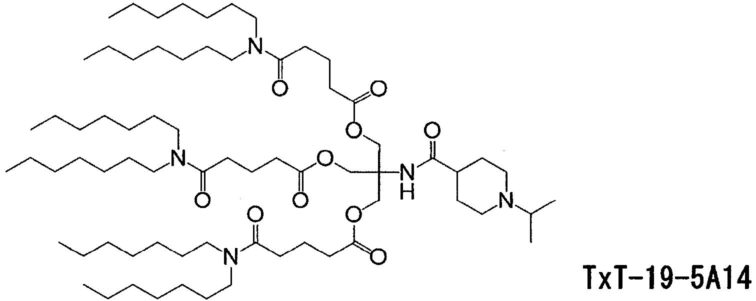

8. A method for producing a pH-sensitive cationic lipid, the method comprising condensing a compound represented by the following general formula (A), a compound represented by the following general formula (B), and tris(hydroxymethyl)aminomethane to produce a pH-sensitive cationic lipid comprising a compound represented by the following general formula (I): 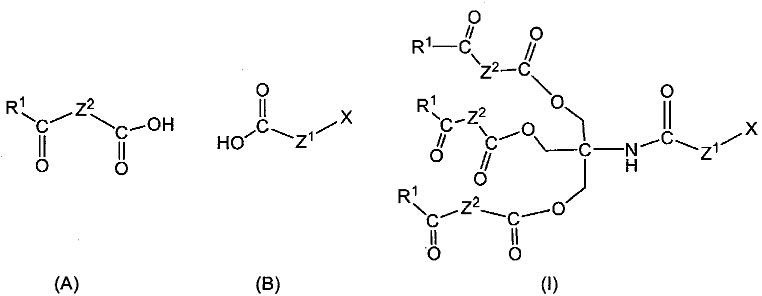
wherein R¹ is a group represented by any of the general formulae (T-1) to (T-3): 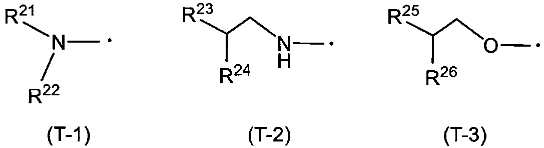
wherein R²¹ is a hydrocarbon group having 1 to 22 carbon atoms; R²² is a hydrocarbon group having 1 to 22 carbon atoms; R²³ is a hydrocarbon group having 1 to 22 carbon atoms; R²⁴ is a hydrogen atom or a hydrocarbon group having 1 to 22 carbon atoms; R²⁵ is a hydrocarbon group having 1 to 22 carbon atoms; and R²⁶ is a hydrogen atom or a hydrocarbon group having 1 to 22 carbon atoms, provided that the total number of carbon atoms in R²¹ and R²² is equal to or greater than 8, the total number of carbon atoms in R²³ and R²⁴ is equal to or greater than 6, and the total number of carbon atoms in R²⁵ and R²⁶ is equal to or greater than 6; and a black circle represents a bonding arm; and
Z² is a linear alkylene group having 4 to 6 carbon atoms or a cycloalkylene group; Z¹ is an alkylene group having 1 to 6 carbon atoms which may be substituted with one hydroxyalkyl group having 1 to 3 carbon atoms, or a single bond; X is -N(R²)(R³) or a 5-to 7-membered nonaromatic heterocyclic group, wherein the group is bonded to Z¹ via a carbon atom, and the ring may be substituted to have one or two hydrocarbon groups having 1 to 4 carbon atoms or amide groups thereon; and R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group,
in the formula (I), three R¹ groups in one molecule may be the same as or different from each other; and in the formula (I), three Z² in one molecule may be the same as or different from each other.

9. A method for producing a pH-sensitive cationic lipid, the method comprising:
condensing a compound represented by the following general formula (A-0) and a compound represented by the following general formula (D) to produce a compound represented by the following general formula (A'): 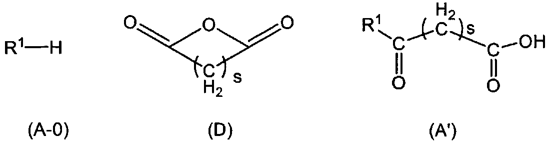
wherein R¹ is a group represented by any of the general formulae (T-1) to (T-3): 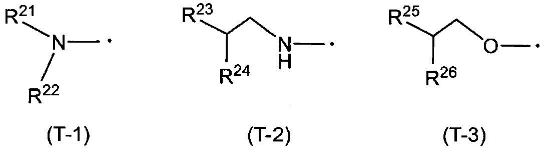
wherein R²¹ is a hydrocarbon group having 1 to 22 carbon atoms; R²² is a hydrocarbon group having 1 to 22 carbon atoms; R²³ is a hydrocarbon group having 1 to 22 carbon atoms; R²⁴ is a hydrogen atom or a hydrocarbon group having 1 to 22 carbon atoms; R²⁵ is a hydrocarbon group having 1 to 22 carbon atoms; and R²⁶ is a hydrogen atom or a hydrocarbon group having 1 to 22 carbon atoms, provided that the total number of carbon atoms in R²¹ and R²² is equal to or greater than 8, the total number of carbon atoms in R²³ and R²⁴ is equal to or greater than 6, and the total number of carbon atoms in R²⁵ and R²⁶ is equal to or greater than 6; and a black circle represents a bonding arm; and
"s" is 2, 3, or 4; and
condensing the compound represented by the general formula (A'), a compound represented by the following general formula (B), and tris(hydroxymethyl)aminomethane to produce a pH-sensitive cationic lipid comprising a compound represented by the following general formula (1'): 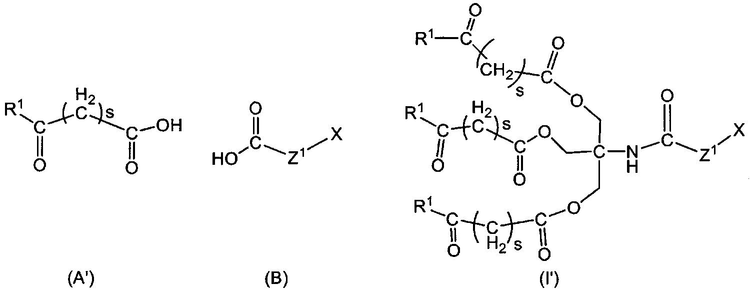
wherein R¹ and "s" are defined in the same manner as described above;
in formula (I'), three R¹ groups in one molecule may be the same as or different from each other;
in formula (I'), three "s" in one molecule may be the same as or different from each other; and
Z¹ is an alkylene group having 1 to 6 carbon atoms which may be substituted with one hydroxyalkyl group having 1 to 3 carbon atoms, or a single bond; X is - N(R²)(R³) or a 5- to 7-membered nonaromatic heterocyclic group, wherein the group is bonded to Z¹ via a carbon atom, and the ring may be substituted to have one or two hydrocarbon groups having 1 to 4 carbon atoms or amide groups thereon; and R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group.

10. A method for producing a pH-sensitive cationic lipid, the method comprising:
adding N-[tris(hydroxymethyl)methyl]acrylamide to a compound represented by the following general formula (C): 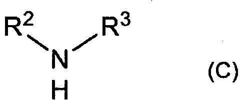
wherein R² and R³ are each independently a hydrogen atom or a hydrocarbon group having 1 to 4 carbon atoms in which one or two hydrogen atoms may be substituted with a phenyl group, a pyrrolyl group, a pyrimidyl group, or an indolyl group, or R² and R³ may be bonded to each other to form a 5- to 7-membered nonaromatic heterocycle in which a hydrogen atom of the ring may be substituted with a hydrocarbon group having 1 to 4 carbon atoms, an amide group, or a nitrogen-containing cyclic group, and one carbon atom of the ring may be a carbon atom constituting other nitrogen-containing cyclic group; and
then condensing the resulting compound with a compound represented by the following general formula (A) or (A'): 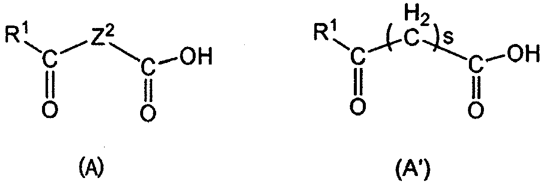
wherein R¹ is a group represented by any of the general formulae (T-1) to (T-3): 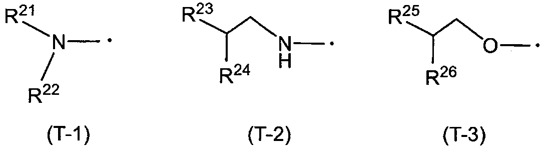
wherein R²¹ is a hydrocarbon group having 1 to 22 carbon atoms; R²² is a hydrocarbon group having 1 to 22 carbon atoms; R²³ is a hydrocarbon group having 1 to 22 carbon atoms; R²⁴ is a hydrogen atom or a hydrocarbon group having 1 to 22 carbon atoms; R²⁵ is a hydrocarbon group having 1 to 22 carbon atoms; and R²⁶ is a hydrogen atom or a hydrocarbon group having 1 to 22 carbon atoms, provided that the total number of carbon atoms in R²¹ and R²² is equal to or greater than 8, the total number of carbon atoms in R²³ and R²⁴ is equal to or greater than 6, and the total number of carbon atoms in R²⁵ and R²⁶ is equal to or greater than 6; and a black circle represents a bonding arm;
in formula (A), Z² is a linear alkylene group having 4 to 6 carbon atoms or a cycloalkylene group; and
in formula (A'), "s" is 2, 3, or 4,
thereby producing a pH-sensitive cationic lipid comprising a compound represented by the following general formula (I-a) or (I'-a): 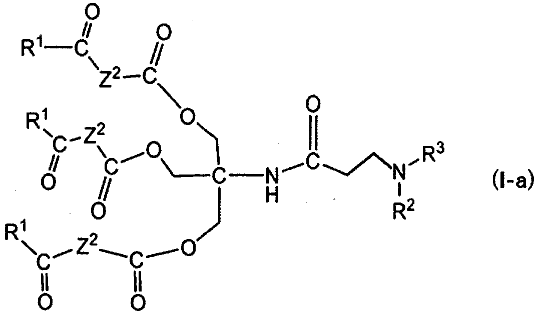 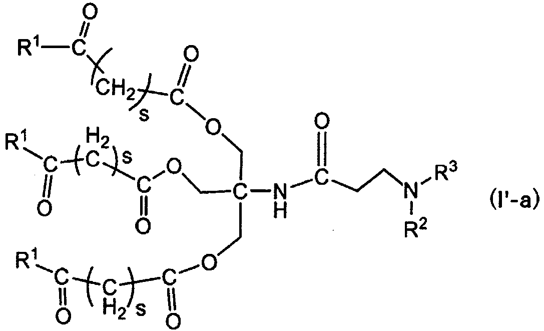
wherein R¹, R², and R³ are defined in the same manner as described above; three R¹ groups in one molecule may be the same as or different from each other;
in formula (I-a), Z² is defined in the same manner as described above; and
in formula (I'-a), "s" is defined in the same manner as described above.

11. A lipid nanoparticle comprising the pH-sensitive cationic lipid according to any one of claims 1 to 7.

12. The lipid nanoparticle according to claim 11, further comprising a sterol and a polyalkylene glycol-modified lipid.

13. The lipid nanoparticle according to claim 11, further comprising phosphatidylcholine.

14. The lipid nanoparticle according to claim 11, containing a nucleic acid.

15. The lipid nanoparticle according to claim 14, wherein the nucleic acid is siRNA, mRNA, gRNA, or plasmid DNA.

16. A pharmaceutical composition comprising the lipid nanoparticle containing the pH-sensitive cationic lipid according to any one of claims 1 to 7 as an active ingredient.

17. A pharmaceutical composition comprising, as an active ingredient, the lipid nanoparticle containing the pH-sensitive cationic lipid according to any one of claims 1 to 7 and a nucleic acid.

18. The pharmaceutical composition according to claim 16, which is used for gene therapy.

19. A method for expressing a foreign gene, comprising administering to a subject animal the lipid nanoparticle according to claim 14, which encapsulates a foreign gene of interest to be expressed in a target cell, and expressing the foreign gene in the target cell of the subject animal.
